# EUROPEAN PATENT APPLICATION

(11) **EP 2 548 876 A1**
(43) Date of publication of application: **23.01.2013**
(21) Application number: 11382245.6
(22) Date of filing: 18.07.2011
(51) Int. Cl.: C07D 471/04, C07D 487/04, A61K 31/437, A61P 11/00

(54) **New CRTh2 antagonists**

(71) Applicant: Almirall, S.A., 08022 Barcelona (ES)
(72) Inventor: Vidal Juan, Bernat, 08980 Sant Feliú de Llobregat (ES); Alonso Diez, Juan Antonio, 08980 Sant Feliú de Llobregat (ES); Buil Albero, María Antonia, 08980 Sant Feliú de Llobregat (ES); Eastwood, Paul Robert, 08980 Sant Feliú de Llobregat (ES); Esteve Trias, Cristina, 08980 Sant Feliú de Llobregat (ES); Lozoya Toribio, María Estrella, 08980 Sant Feliú de Llobregat (ES); Roberts, Richard Spurring, 08980 Sant Feliú de Llobregat (ES); Vidal Gispert, Laura, 08980 Sant Feliú de Llobregat (ES)
(74) Representative: Elzaburu Marquez, Alberto

(57) **Abstract**

The present invention relates to compounds of formula (I), to the process for preparing such compounds and to their use in the treatment of a pathological condition or disease susceptible to amelioration by CRTh2 antagonist activity.

## Description

### FIELD OF THE INVENTION

The present invention relates to novel compounds having CRTh2 activity. This invention also relates to pharmaceutical compositions containing them, processes for their preparation and their use in respiratory therapies.

### BACKGROUND OF THE INVENTION

Prostaglandin D2 (PGD2) is a prostaglandin derived from the metabolism of arachidonic acid by cyclooxygenases and downstream PGD2 synthases. In the immune system, PGD2 is mainly produced by mast cells (Lewis et al., J. Immunol., 1982, 129, 1627-1631) but also although at lower levels, by macrophages and Th2 lymphocytes (Urade; J. Immunol, 1989, 143, 2982-2989. Tanaka; J. Immunol, 2000, 164, 2277-2280) in response to local tissue damage and allergic inflammation in diseases such as asthma (Murray et al., N. Engl. J. Med., 1986, 315, 800-804; Liu et al; Am. Rev. Respir. Dis., 1990, 142, 126-132; Wenzel et al., J. Allergy Clin. Immunol., 1991, 87, 540-548), rhinitis (Naclerio et al., Am. Rev. Respir. Dis., 1983, 128, 597-602; Brown et al., Arch. Otolarynol. Head Neck Surg., 1987, 113, 179-183; Lebel et al; J. Allergy Clin. Immunol., 1988, 82, 869-877) and atopic dermatitis among others. Exogenous PGD2 applied to bronchial airways elucidates many characteristics of asthmatic response suggesting that this prostaglandin plays an important pro-inflammatory role in allergic diseases (Hardy; N. Engl. J. Med., 1980, 311, 209-213. Sampson; Thorax, 1997, 52, 513-518). PGD2 binds to three different receptors, the thromboxane-type prostanoid receptor (TP) (Coleman; Br. J. Pharmacol., 1989, 96, 688-692. Francis; Br. J. Pharmacol., 1991, 104, 596-602), the PGD2 receptor (DP also known as DP1) (Boie et al; J. Biol. Chem., 1995, 270, 18910-18916) and the chemoattractant receptor-homologous molecule expressed on Th2 (CRTh2 also known as DP2) (Nagata et al; J. Immunol., 1999, 162, 1278-1289; Powell; Prostaglandins Leukot. Essent. Fatty Acids, 2003, 69, 179-185). CRTh2 is a Gi-coupling GPCR signalling through reduction of intracellular cAMP and calcium mobilization and it is involved in the chemotaxis of Th2 lymphocytes, eosinophils and basophils (Hirai, J. Exp. Med. 2001, 193, 255-261. Shichijo; J. Pharmacol. Exp. Ther., 2003, 307, 518-525). CRTh2 also inhibits the apoptosis of Th2 lymphocytes (Xue; J. Immunol., 2009, 182, 7580-7586) and stimulates the production of IL4, IL5, and IL13 (Xue; J. Immunol., 2005, 175, 6531-6536) which are cytokines involved in important biological responses such as eosinophil recruitment and survival, mucus secretion, airway hyperresponsiveness and immunoglobulin E production among others. Therefore, molecules that antagonize the mentioned pro-inflammatory PGD2 effects mediated by CRTh2 on key cell types associated with allergic inflammation like basophils, eosinophils and Th2 lymphocytes would have a potential benefit in related pathologies.

In view of these physiological effects, CRTh2 inhibitors of varied chemical structures have been recently disclosed for the treatment or prevention of chronic and acute inflammatory diseases and other pathological conditions, diseases and disorders known to be susceptible to amelioration by inhibition of CRTh2. Several structural families of antagonists are observed.

In general antagonists can be grouped into one of the following families: indole acetic acids, phenylacetic acids, phenoxyacetic acids and tetrahydroquinolines derivatives.

Examples of indole acetic acids derivatives are those described in GB2407318, WO2003101981, WO2005019171, WO2006036994, WO2007022501, WO2008012511, WO2009063215, US2010063103.

Structurally similar analogues of the indole group have also been reported. Compounds disclosed induce structural variations such as: azaindoles (for example: WO2007068418), indolizines (for example: WO2009044147), tetrahydoindazoles (for example: WO2010006944), tetrahydroindoles (for example: WO2010039982), benzimidazoles (for example: WO2006034418), oxindoles (for example: WO2009061676) and thienopyrroles (for example: WO2009102462).

Additionally, tricyclic variations of some of the above structures have been disposed. For example: WO2010008864, WO2010031183.

For examples of phenylacetic acids and Structurally similar analogues, see: WO2004058164, WO2007039736, WO2008024746, WO2009061730 and WO2010003120.

Structurally similar analogues of the phenylacetic group have also been reported. Compounds disclosed include structural variations such as: naphthalene acetic acids (for example: WO2010055006), pyrimidine acetic acids (for example: WO2010027448), pyrrole acetic acids (for example: WO2007144127), thiophene acetic acids (for example: WO2007062797) and other heterocycle-acetic acids (for example: WO2010057118).

For examples of phenoxyacetic acids see: WO2004089884 WO2005018529, WO2006005909, WO2007036743 WO2008119917, WO2009004379 and WO2010018109.

For examples of tetrahydroquinolines see: WO2004032848 WO2006091674.

Ramatroban, {(3R)-3-[(4-fluorophenyl)sulphonylamino]-1,2,3,4-tetrahydro-9H-carbazole-9-propanoic acid}, a thromboxane A2 receptor antagonist is also known to be a CRTh2 antagonist (Sugimoto et al; J. Pharmacol. Exp. Ther., 2003, 305, 347-352). A few compounds having the capacity to selectively inhibit CRTh2 are in active development. Examples of these compounds are ODC-91 01, AZD-1981, ACT-129968, AP-761, AM211, AM461, ADC-3680, ARRY-502, MK-7246 and RG-7185.

In addition, compounds having the capacity to selectively inhibit both DP1 and CRTh2 have been reported. An example of this type of compound is AMG-853.

These observations suggest that CRTh2 antagonists may have valuable properties for the treatment of diseases mediated by PGD2.

### SUMMARY OF THE INVENTION

The invention provides a compound of formula (I), or a Pharmaceutical acceptable salt, N-oxide, solvate, stereiosomer or deuterated derivative thereof: 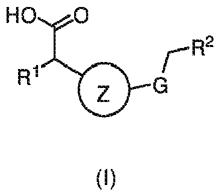 wherein:
- Z represents a 9-membered bicyclic heteroaryl group comprising 2 nitrogen atoms and optionally comprising further heteroatoms selected from N, S and O; wherein said heteroaryl group is optionally substituted by one to four substituents selected from the group consisting of a halogen atom, a linear or branched C₁₋₄ alkyl group, a C₃₋₇ cycloalkyl group, a linear or branched C₁₋₄ haloalkyl group, a -CN group, a -OH group, a -OR^{a} group, a -CO₂H group, a -CO₂R^{a} group, a -SR^{a} group, and a -SO₂R^{a} group;
- R¹ is selected from the group consisting of a hydrogen atom and a linear or branched C₁₋₄ alkyl group;
- G is selected from the group consisting of a phenyl group and a 5 to 6-membered N-containing heteroaryl group, wherein the phenyl and heteroaryl groups are optionally substituted by one or more substituents selected from the group consisting of a halogen atom, a cyano group, a nitro group, a -CHF₂ group, a -CF₃ group, a linear or branched 6₁₋₄ alkyl group, a C₃₋₇ cycloalkyl group, a -OH group, a -OR^{a} group, a -SR^{a} group and a -SO₂R^{a} group;
- R² is selected from the group consisting of a -N(R³)COR⁴ group, a -N(R³)SO₂R⁴ group and a heteroaryl group selected from the group consisting of a triazolyl group, an isoxazolyl group, a pyrazolyl group and an oxazolyl group, wherein the heteroaryl group is optionally substituted by one or more substituents selected from the group consisting of a halogen atom, a -CF₃ group, a linear or branched C₁₋₄ alkyl group, a C₃₋₇ cycloalkyl group, a benzyl group, a phenyl, group, a hydroxy group and a carboxy group;
- R³ is selected from the group consisting of a linear or branched 6₁₋₄ alkyl group; a C₃₋₇ cycloalkyl group, a linear or branched C₁₋₄ haloalkyl group and a benzyl group;
- R⁴ is selected from the group consisting of a linear or branched 6₁₋₄ alkyl group, a -(CH₂)ₙ-C₃₋₇ cycloalkyl group, a -CF₃ group, a linear or branched C₁₋₄ alkoxy group, a -C₁₋₄ alkyl-C₁₋₄ alkoxy group, a -(CH₂)ₙR^{b}R^{c} group, a 4- to 6-membered heterocyclyl group containing at least one heteroatom selected from N, S and O, a -(CH₂)ₙ-phenyl group, a -(CH₂)ₙ-(5- to 6-membered heteroaryl) group containing at least one heteroatom selected from N, S and O, wherein the phenyl, heteroaryl and heterocyclyl groups are optionally substituted by one or more substituents selected from the group consisting of a halogen atom, a linear or branched C₁₋₄ alkyl group, an acyl group and a linear or branched C₁₋₄ alkoxy group;
- R^{a} is selected from the group consisting of a linear or branched C₁₋₄ alkyl group, a linear or branched C₁₋₄ haloakyl group and a C₃₋₇ cycloalkyl group;
- R^{b} and R^{c} are independently selected from the group consisting of a hydrogen atom, a linear or branched C₁₋₄ alkyl group, a C₃₋₇ cycloalkyl group, a phenyl, group, a 5- to 6-membered heteroaryl containing at least one heteroatom selected from N, S and O, a -C₁₋₄ alkyl-phenyl group and -C₁₋₄alkyl-5- to 6-membered heteroaryl group; or R^{b} and R^{c} together with the nitrogen atom to which they are attached form a 3- to 7-membered N-containing heterocycyl group which is optionally substituted by one or more substitutents selected by the group consisting of a halogen atom, a linear or branched 6₁₋₄ alkyl group and a linear or branched C₁₋₄ alkoxy group; and
- n is an integer selected from 0 to 2.

The invention provides synthetic processes and intermediates described herein, which are useful for preparing compounds of the invention.

The invention also provides a pharmaceutical composition comprising a compound of the invention and a pharmaceutically-acceptable carrier. The invention further provides combinations comprising a compound of the invention and one or more other therapeutic agents and pharmaceutical compositions comprising such combinations.

The invention also provides a compound of the invention for use in the treatment of the human or animal body by therapy.

The invention also provides a compound of the invention for use in the treatment of a disease or condition susceptible to amelioration by CRTh2 antagonists, in a mammal, in particular wherein the pathological condition or disease is selected from asthma, chronic obstructive pulmonary disease (COPD), rhinitis, emphysema, allergic airway syndrome, allergic rhinobronchitis, psoriasis, dermatitis, allergic conjunctivitis, atopic dermatitis, pulmonary hypertension, interstitial lung fibrosis, allergy, psoriasis, adult respiratory distress syndrome (ARDS), myocardial infarction, aneurysm, stroke, cancer, wound healing, endotoxic shock, pain, inflammatory conditions, eosinophilic esophagitis, eosinophil-associated gastrointestinal disorders (EGID), idiopathic otitis, airway constriction, mucus secretion, nasal congestion, increased microvascular permeability and recruitment of eosinophil, urticaria, sinusitis, angioedema, anaphylaxia, chronic cough, Churg Strauss syndrome, arthritis, irritable bowel disease and inflammatory bowel disease (such as Crohn's disease and ulcerative colitis).

The invention also provides the use of a compound of the invention in the manufacture of a formulation or medicament for treating a disease or condition susceptible to amelioration by CRTh2 antagonists, in particular wherein the condition or disease is as described above.

The invention also provides a method of treating a disease or condition as described above; comprising such method administering to the mammal, a therapeutically effective amount of a compound of the invention. The invention further provides a method of treatment comprising administering a therapeutically effective amount of a combination of a compound of the invention together with one or more other therapeutic agents.

### DETAILED DESCRIPTION OF THE INVENTION

When describing the compounds, compositions, combinations and methods of the invention, the following terms have the following meanings, unless otherwise indicated.

As used herein the term C₁₋₄ alkyl embraces optionally substituted, linear or branched radicals having 1 to 4 carbon atoms. Examples include methyl, ethyl, n-propyl, i-propyl, n-butyl, sec-butyl or t-butyl. Optional substituents of a C₁₋₄ alkyl group include a halogen atom and C₁₋₄ alkoxy group.

As used herein, the term C₁₋₄ alkoxy (or alkyloxy) embraces optionally substituted, linear or branched oxy-containing radicals each having alkyl portions of 1 to 4 carbon atoms. Examples of C₁₋₄ alkoxy radicals include methoxy, ethoxy, n-propoxy, i-propoxy, n-butoxy, sec-butoxy and t-butoxy. Optional substituents of a C₁₋₄ alkoxy group induce a halogen atom, a C₁₋₄ alkyl group and a C₁₋₄ alkoxy group.

When it is mentioned that alkyl or alkoxy radicals may be optionally substituted it is meant to include linear or branched alkyl or alkoxy radicals as defined above, which may be unsubstituted or substituted in any position by one or more substituents, for example by 1, 2 or 3 substituents.

A said optionally substituted alkyl group is typically unsubstituted or substituted with 1, 2 or 3 substituents which may be the same or different. Typically, substituents on an alkyl group are themselves unsubstituted.

As used herein, the term C₁-C₄ haloalkyl group is a linear or branched alkyl group, which is substituted by one or more, preferably 1, 2 or 3 halogen atoms. Examples include CCl₃, CF₃ and CHF₂.

As used herein, the term C₃₋₇ cycloalkyl group embraces saturated carbocyclic radicals monocyclic or polycyclic ring having from 3 to 7 carbon atoms. Examples include cyclopropyl, cyclobutyl, cyclopentyl, cyclohexyl and cycloheptyl.

As used herein, the term C₆₋₁₀ aryl radical embraces typically a C₆-C₁₀ monocyclic or polycyclic aryl radical such as phenyl and naphthyl.

As used herein, the term 5- to 6- membered heteroaryl radical embraces typically a 5-to 6- membered ring system containing at least one heteroatom selected from O, S and N. Examples include pyridinyl, pyrazinyl, pyrimidinyl, pyridazinyl, furyl, oxadiazolyl, oxazolyl, isoxazolyl, imidazolyl, thiazolyl, thiadiazolyl, isothiazolyl, thienyl, pyrrolyl, pyridinyl, triazolyl, imidazolidinyl, and pyrazolyl.

As used herein, the term 5- to 6- membered N-containing heteroaryl radical embraces typically a 5- to 6- membered ring system containing at least one N atom and optionally further containing one or more heteroatoms selected from O, S and N. Examples include pyridinyl, pyrazinyl, pyrimidinyl, pyridazinyl, oxadiazolyl, oxazolyl, isoxazolyl, imidazalyl, thiazolyl, thiadiazolyl, isothiazolyl, pyrrolyl, pyridinyl, triazolyl, imidazolidinyl, and pyrazolyl.

As used herein, the term 9-membered bicyclic heteroaryl group embraces typically a 9-membered bicyclic ring system containing 2 nitrogen atoms and optionally further containing one or more, for example one or two, additional heteroatoms selected from N, S and O. Preferably said 9-membered bicycylic ring system is a 6-membered ring fused to a 5-membered ring. Examples include a 4-azaindolyl group, a 5-azaindolyl group, a 7-azaindolyl group, a pyrrolo[1,2-a]pyrimidinyl group, an imidazo[1,5-a]pyridyl group, a pyrrolo[1,2-a]pyrazinyl group, a pyrrolo[1,2-c]pyrimidinyl group and a pyrrolo[1,2-b]pyridazinyl group.

As used herein, the term 4 to 6-membered heterocyclyl radical embraces typically a non-aromatic, saturated or unsaturated C₄₋₆ carbocyclic ring system in which at least one carbon is replaced by a heteroatom selected from N, O and S. Exampels include pyrrolinyl, piperazinyl, morpholinyl, thiomorpholinyl, pyrazolinyl and azetidinyl.

As used herein, the term 3 to 7-membered N-containing heterocyclyl radical embraces typically a non-aromatic, saturated or unsaturated C₃-C₇ carbocyclic ring system in which at least one carbon is replaced by a N atom and optionally additional carbon atoms are replaced by a heteroatom selected from N, O and S. Examples of 3 to 7-membered N-containing heterocyclic radicals include piperidinyl, pyrrolidinyl, pyrrolinyl, piperazinyl, morpholinyl, thiomorpholinyl, pyrazolinyl and azetidinyl.

As used herein, the term halogen atom embraces chlorine, fluorine, bromine or iodine atoms typically a fluorine, chlorine or bromine atom. The term halo when used as a prefix has the same meaning.

As used herein, the term acyl embraces -C(O)C₁₋₄ alkyl group and a -C(O)C₃₋₇ cycloalkyl group.

The term "therapeutically effective amount" refers to an amount sufficient to effect treatment when administered to a patient in need of treatment.

The term "treatment" as used herein refers to the treatment of a disease or medical condition in a human patient which includes:
(a) preventing the disease or medical condition from occurring, i.e., prophylactic treatment of a patient;
(b) ameliorating the disease or medical condition, i.e., causing regression of the disease or medical condition in a patient;
(c) suppressing the disease or medical condition, i.e., slowing the development of the disease or medical condition in a patient; or
(d) alleviating the symptoms of the disease or medical condition in a patient.

The phrase "disease or condition associated with CRTh2 activity" includes all disease states and/or conditions that are acknowledged now, or that are found in the future, to be associated with CRTh2 activity. Such disease states include, but are not limited to, asthma, chronic obstructive pulmonary disease (COPD), rhinitis, emphysema, allergic airway syndrome, allergic rhinobronchitis, psoriasis, dermatitis, allergic conjunctivitis, atopic dermatitis, pulmonary hypertension, interstitial lung fibrosis, allergy, psoriasis, adult respiratory distress syndrome (ARDS), myocardial infarction, aneurysm, stroke, cancer, wound healing, endotoxic shock, pain, inflammatory conditions, eosinophilic esophagitis, eosinophil-associated gastrointestinal disorders (EGID), idiopathic otitis, airway constriction, mucus secretion, nasal congestion, increased microvascular permeability and recruitment of eosinophils, urticaria, sinusitis, angioedema, anaphylaxia, chronic cough, Churg Strauss syndrome, arthritis, irritable bowel disease and inflammatory bowel disease (such as Crohn's disease and ulcerative colitis). Preferably, such disease states are asthma or chronic obstructive pulmonary disease (COPD), rhinitis and atopic dermatitis.

The term "pharmaceutically-acceptable salt" refers to a salt prepared from a base or acid which is acceptable for administration to a patient, such as a mammal. Such salts can be derived from pharmaceutically-acceptable inorganic or organic bases and from pharmaceutically-acceptable inorganic or organic acids.

Salts derived from pharmaceutically-acceptable acids include acetic, benzenesulfonic, benzoic, camphosulfonic, citric, ethanesulfonic, formic, fumaric, gluconic, glutamic, hydrobromic, hydrochloric, hydrofluoric, lactic, maleic, malic, mandelic, methanesulfonic, mucic, nitric, pantothenic, phosphoric, succinic, sulfuric, tartaric, p-toluenesulfonic, xinafoic (1-hydroxy-2-naphthoic acid), napadisilic (1,5-naphthalenedisulfonic acid), triphenyl acetic and the like. Particularly preferred are salts derived from formic, fumaric, hydrobromic, hydrochloric, hydrofluoric, acetic, sulfuric, methanesulfonic, xinafoic, tartaric, maleic, succinic and napadisilic acids.

Salts derived from pharmaceutically-acceptable inorganic bases include aluminum, calcium, copper, ferric, ferrous, lithium, magnesium, manganic, manganous, potassium, sodium, zinc and the like. Particularly preferred are calcium, magnesium, potassium and sodium salts.

Salts derived from pharmaceutical-acceptable organic bases include salts of ammonia, primary, secondary and tertiary amines, inducing substituted amines, cyclic amines, naturally-occurring amines and the like, such as ammonia, arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

Other preferred salts according to the invention are quaternary ammonium compounds wherein an equivalent of an anion (X⁻) is associated with the positive charge of the N atom. X⁻ may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and p-toluenesulphonate. X- is preferably an anion selected from chloride, bromide, iodide, sulphate, nitrate, acetate, maleate, oxalate, succinate or trifluoroacetate. More preferably X⁻ is chloride, bromide, trifluoroacetate or methanesulphonate.

As used herein, an N-oxide is formed from the tertiary basic amines or imines present in the molecule, using a convenient oxidising agent.

As used herein, the term solvate means a compound which further includes a stoichiometric or non-stoichiometric amount of solvent such as water, acetone, methanol, methanol, dichloromethane, 2-propanol, or the like, bound by non-covalent intermolecular forces. When the solvent is water, the term hydrate is used instead of solvate.

The invention also includes isotopically-labeled compounds of the invention, wherein one or more atoms is replaced by an atom having the same atomic number, but an atomic mass or mass number different from the atomic mass or mass number usually found in nature. Examples of isotopes suitable for infusion in the compounds of the invention include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I, nitrogen, such as ¹³N and ¹⁵N, oxygen, such as ¹⁵O,¹⁷O and ¹⁸O, phosphorus, such as ³²P , and sulfur, such as ³⁵S. Certain isotopically-labeled compounds of the invention, for example, those incorporating a radioactive isotope, are useful in drug and/or substrate tissue distribution studies. The radioactive isotopes tritium, ³H, and carbon-14, ¹⁴C, are particularly useful for this purpose in view of their ease of incorporation and ready means of detection. Substitution with heavier isotopes such as deuterium, ²H, may afford certain therapeutic advantages resulting from greater metabolic stability, for example, increased in vivo half-life or reduced dosage requirements, and hence may be preferred in some circumstances. Substitution with positron emitting isotopes, such as ¹¹C, ¹⁸F, ¹⁵O and ¹³N, can be useful in Positron Emission Topography (PET) studies for examining substrate receptor occupancy. Isotopically-labeled compounds of the invention can generally be prepared by conventional techniques known to those skilled in the art or by processes analogous to those described herein, using an appropriate isotopically-labeled reagent in place of the non-labeled reagent otherwise employed.

Preferred isotopically-labeled compounds include deuterated derivatives of the compounds of the invention. As used herein, the term deuterated derivative embraces compounds of the invention where in a particular position at least one hydrogen atom is replaced by deuterium. Deuterium (D or ²H) is a stable isotope of hydrogen which is present at a natural abundance of 0.015 molar %.

Hydrogen deuterium exchange (deuterium incorporation) is a chemical reaction in which a covalently bonded hydrogen atom is replaced by a deuterium atom. Said exchange (incorporation) reaction can be total or partial.

Typically, a deuterated derivative of a compound of the invention has an isotopic enrichment factor (ratio between the isotopic abundance and the natural abundance of that isotope, i.e. the percentage of incorporation of deuterium at a given position in a molecule in the place of hydrogen) for each deuterium present at a site designated as a potential site of deuteration on the compound of at least 3500 (52.5% deuterium incorporation).

In a preferred embodiment, the isotopic enrichment factor is at least 5000 (75% deuterium). In a more preferred embodiment, the isotopic enrichment factor is at least 6333.3 (95% deuterium incorporation). In a most preferred embodiment, the isotopic enrichment factor is at least 6633.3 (99.5% deuterium incorporation). It is understood that the isotopic enrichment factor of each deuterium present at a site designated as a site of deuteration is independent from the other deuteration sites.

The isotopic enrichment factor can be determined using conventional analytical methods known too en ordinary skilled in the art, including mass spectrometry (MS) and nuclear magnetic resonance (NMR).

The term "amino-protecting group" refers to a protecting group suitable for preventing undesired reactions at amino nitrogen. Representative amino-protecting groups include, but are not limited to, formyl; acyl groups, for example alkanoyl groups such as acetyl, benzoyl, pivaloyl or trifluoroacetyl; alkoxycarbonyl groups such as tert-butoxycarbonyl (Boc); arylmethoxycarbonyl groups such as benzyloxycarbonyl (Cbz) and 9-fluorenylmethoxycarbonyl (Fmoc); arylmethyl groups such as benzyl (Bn), trityl (Tr), and 1,1-di-(4'-methoxyphenyl)methyl and the like.

The term "hydroxy-protecting group" refers to a protecting group suitable for preventing undesired reactions at a hydroxy group. Representative hydroxy-protecting groups induce, but are not limited to, alkyl groups, such as methyl, ethyl, and tert-butyl; acyl groups, for example alkanoyl groups, such as acetyl, pivaloyl or trifluoroacetyl; arylcarbonyl groups, such as benzoyl; alkoxycarbonyl groups such as tert-butoxycarbonyl (Boc); arylmethoxycarbonyl groups such as benzyloxycarbonyl (Cbz) and 9-fluorenylmethoxycarbonyl (Fmoc); arylmethyl groups, such as benzyl (Bn), p-methoxybenzyl (PMB), 9-fluorenylmethyl (Fm), and diphenylmethyl (benzhydryl, DPM); silyl groups, such as trimethylsilyl (TMS) and tert-butyldimethylsilyl (TBS); and the like.

The compounds of the invention may contain one or more chiral centers. Accordingly, the invention includes racemic mixtures, enantiomers, and mixtures enriched in one or more stereoisomer. The scope of the invention as described and claimed encompasses the racemic forms of the compounds as well as the individual enantiomers, diastereomers, and stereoisomer-enriched mixtures.

Typically, Z is selected from the group consisting of a 4-azaindolyl group, a 5-azaindolyl group, a 7-azaindolyl group, a pyrrolo[1,2-a]pyrimidinyl group, an imidazo[1,5-a]pyridyl group, a pyrrolo[1,2-a]pyrazinyl group, a pyrrolo[1,2-c]pyrimidinyl group and a pyrrolo[1,2-b]pyridazinyl group, wherein said groups are optionally substituted by one to four substitutents selected from the group consisting of a halogen atom, a linear or branched C₁₋₄ alkyl group, a C₃₋₇ cycloalkyl group, a linear or branched C₁₋₄ haloalkyl group, a -CN group, a -OH group, a -OR^{a} group, a "CO₂H group, a - CO₂R^{a} group, a -SR^{a} group, and a -SO₂R^{a} group.

In a preferred embodiment, Z represents a group of formula A, B, C, D, E, F, G or H: 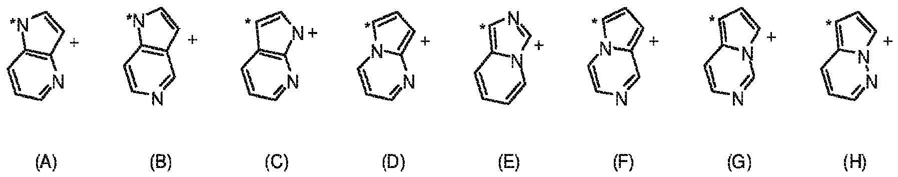 wherein the asterisk symbol indicates the bond that is linked to the -CH-R¹ moiety of Formula (I) and the plus symbol indicates the bond that is linked to the G group of Formula (I), and wherein said groups of formula A to H are optionally substituted by one to four substitutents selected from the group consisting of a fluorine atom, a chlorine atom, a C₁₋₄ alkyl group, a methoxy group, a -CN group, a cyclopropyl group, a -CF₃ group and a -CHF₂ group.

In a more preferred embodiment, Z is a group of formula A, C or D, which is optionally substituted by one or two substituents selected from the group consisting of a methyl group, an ethyl group, a cyclopropyl group, a -CF₃ group a -CHF₂ group and a methoxy group.

Typically, R¹ is selected from the group consisting of a hydrogen atom and methyl group.

Typically, G is selected from the group consisting of a phenyl group, a pyridyl group, a pyrimidinyl group, a pyridazinyl group and a pyrazolyl group, wherein said ring groups are optionally substituted by one or two substituents selected from the group consisting of a halogen atom, a methyl group, a -SO₂Me group, a -CHF₂ group and a -CF₃ group.

Typically, the Z moiety and the -CH₂R₂ moiety are connected to the ring that G represents at positions which are ortho to each other.

In a preferred embodiment, G represents a phenyl group optionally substituted by one substitutent selected from the group consisting of a fluorine atom and a -CF₃ group.

Typically, R² is selected from the group consisting of a -N(R³)COR⁴ group and a heteroaryl group selected from the group consisting of a triazolyl group and an isoxazolyl group, wherein the heteroaryl group is optionally substituted by one or more substituents selected from the group consisting of a methyl group and a cyclopropyl group.

Typically, R³ is selected from the group consisting of a linear or branched C₁₋₄ alkyl group and a benzyl group, preferably an ethyl group and a benzyl group.

Typically, R⁴ is selected from the group consisting of a linear or branched C₁₋₄ alkyl group, a -(CH₂)ₙ-C₃₋₇ cycloalkyl group, a linear or branched C₁₋₄ alkoxy group, a -C₁₋₂ alkyl-C₁₋₂ alkoxy group, a benzyl group, a -(CH₂)ₙNR^{b}R^{c} group, a 4- to 6-membered heterocylyl group containing at least one heteroatom selected from N, S and O and a - (CH₂)ₙ-(5- to 6-membered N-containing heteroaryl) group, wherein the heterocyclyl and heteroaryl groups are optionally substituted by one or more substituents selected from the group consisting of a halogen atom, an acyl group and a methyl group.

Typically, R^{a} is a linear or branched C₁₋₄ alkyl group.

Typically R^{b} and R^{c} are independently selected from the group consisting of a hydrogen atom, a linear or branched C₁₋₄ alkyl group, a C₃₋₇ cycloalkyl group, a phenyl group and a -C₁₋₄ alkyl-phenyl group. Preferably R^{b} and R^{c} are independently selected from the group consisting of a hydrogen atom, a linear or branched C₁₋₄ alkyl group, a phenyl and a benzyl group.

Typically, n is 0 or 1.

In a preferred embodiment of the present invention:
- Z represents a group of formula A, C or D, which is optionally substituted by one or two substituents selected from the group consisting of a methyl group, an ethyl group, a cyclopropyl group, a -CF₃ group, a -CHF₂ group and a methoxy group;
- R¹ is selected from the group consisting of a hydrogen atom and methyl group;
- G is selected from the group consisting of a phenyl group, a pyridyl group, a pyrimidinyl group, a pyridazinyl group and a pyrazolyl group, wherein said ring groups are optionally substituted by one or two substituents selected from the group consisting of a halogen atom, a methyl group, a -CHF² group and a -CF₃ group; preferably G represents a phenyl group optionally substituted by one substitutent selected from the group consisting of a fluorine atom and a -CF₃ group;
- R² is selected from the group consisting of a -N(R³)COR⁴ group and a heteroaryl group selected from the group consisting of a triazolyl group and an isoxazolyl group, wherein the heteroaryl group is optionally substituted by one or more substituents selected from the group consisting of a methyl group and a cyclopropyl group;
- R³ is selected from the group consisting of an ethyl group and a benzyl group;
- R⁴ is selected from the group consisting of a linear or branched C₁₋₄ alkyl group, a -(CH₂)ₙ-C₃₋₇ cycloalkyl group, a linear or branched C₁₋₄ alkoxy group, a -(CH₂)ₙNR^{b}R^{c} group and a -(CH₂)ₙ-(5- to 6-membered N-containing heteroaryl) group, which is optionally substituted by one or more substituents selected from the group consisting of a halogen atom and a methyl group, wherein R^{b} and R^{c} are preferably independently selected from a hydrogen atom, a linear or branched C₁₋₄ alkyl group, a phenyl, and a benzyl group; and
- n is 0 or 1.

In another preferred embodiment of the present invention:
- Z represents a group of formula A, B, C, D, E, F or G, which is optionally substituted by one or two substituents selected from the group consisting of a methyl group, an ethyl group, a cyclopropyl group, a -CF₃ group, a -CHF₂ group and a methoxy group;
- R¹ is selected from the group consisting of a hydrogen atom and methyl group;
- G represents a phenyl group optionally substituted by one substitutent selected from the group consisting of a fluorine atom and a -CF₃ group;
- R² is a -N(R³)COR⁴ groups;
- R³ is selected from the group consisting of a linear or branched C₁₋₄ alkyl group, preferably an ethyl group, and a benzyl group; and
- R⁴ is selected from the group consisting of a C₃₋₇ cycloalkyl group, preferably a cyclopropyl group, and a -(CH₂)ₙNR^{b}R^{c} group, wherein R^{b} represents a hydrogen atom and R^{c} represents a benzyl moiety.

Preferably the compound of the invention is other than any of compounds 1 to 27 set out below.
1. {3-[2-{[(Cyclopropylcarbonyl)(ethyl)amino]methyl}-4-(trifluoromethyl)phenyl]-1H-indazol-1-yl}acetic acid;
2. {4-Chloro-3-[2-{[(cydopropylcarbonyl)(ethyl)amino]methyl}-4-(trifluoromethyl)phenyl]-1H-indazol-1-yl}acetic acid;
3. {3-[2-{[(Cyclopropylcarbonyl)(ethyl)amino]methyl}-4-(trifluoromethyl)phenyl]-5-fluoro-1 H-indazol-1-yl}acetic acid;
4. [4-Chloro-3-(2-{[(cyclopropylcarbonyl)(ethyl)amino]methyl}-4-fluorophenyl)-1H-indazol-1-yl]acetic acid;
5. {5-Chloro-3-[2-{[(cyclopropylcarbonyl)(ethyl)amino]methyl}-4-(trifluoromethyl)phenyl]-1H-indazol-1-yl}acetic acid;
6. {3-[2-{[(Cyclopropylcarbonyl)(ethyl)amino]methyl}-4-(trifluoromethyl)phenyl]-6-fluoro-1H-indazol-1-yl}acetic acid;
7. {3-[2-{[(Cyclopropylcarbonyl)(ethyl)amino]methyl}-4-(trifluoromethyl)phenyl]-4-methoxy-1H-indazol-1-yl}acetic acid;
8. [3-(2-{[(Cyclopropylcarbonyl)(ethyl)amino]methyl}-4-fluorophenyl)-5-(trifluoromethyl)-1H-indazol-1-yl]acetic acid;
9. 2-{4-Chloro-3-[2-{[(cyclopropylcarbonyl)(ethyl)amino]methyl}-4-(trifluoromethyl)phenyl]-1H-indazol-1-yl}propanoic acid;
10. [3-(2-{[(Cyclopropylcarbonyl)(ethyl)amino]methyl}-4-fluorophenyl)-4-(trifluoromethyl)-1H-indazol-1-yl]acetic acid;
11. [5-Chloro-3-(2-{[(cyclopropylcarbonyl)(ethyl)amino]methyl}-4-fluorophenyl)-1H-indazol-1-yl]acetic acid;
12.{4-Cyano-3-[2-{[(cyclopropylcarbonyl)(ethyl)amino]methyl}-4-(trifluoromethyl)phenyl]-1H-indazol-1-yl}acetic acid;
13.2-{5-Chloro-3-[2-{[(cyclopropylcarbonyl)(ethyl)amino]methyl}-4-(trifluoromethyl)phenyl]-1H-indazol-1-yl}propanoic acid;
14.{3-[2-{[(Cyclopropylcarbonyl)(ethyl)amino]methyl}-4-(trifluoromethyl)phenyl]-4-fluoro-1H-indazol-1-yl}acetic acid;
15.{3-[2-{[(Cyclopropylcarbonyl)(ethyl)amino]methyl}-4-(trifiuoromethyl)phenyl]-5-methyl-1H-indazol-1-yl}acetic acid;
16. {3-[2-{[(Cyclopropylcarbonyl)(ethyl)amino]methyl}-4-(trifluoromethyl)phenyl]-4-methyl-1H-indazol-1-yl}acetic acid;
17. {4-Chloro-3-[2-{[(cyclopropylcarbonyl)(ethyl)amino]methyl}-4-(trifluoromethyl)phenyl]-5-fluoro-1H-indazol-1-yl}acetic acid;
18. [3-(2-{[(tert-Butoxycarbonyl)(ethyl)amino]methyl}-4-fluorophenyl)-5-chloro-1H-indazol-1-yl]acetic acid;
19. [5-Chloro-3-(2-{[(cyclobutylcarbonyl)(ethyl)amino]methyl}-4-fluorophenyl)-1H-indazol-1-yl]acetic acid;
20. [5-Chloro-3-(2-{[ethyl(isobutyryl)amino]methyl}-4-fluorophenyl)-1H-indazol-1-yl]acetic acid;
21. [5-Chloro-3-(2-{[ethyl(methylsulfonyl)amino]methyl}-4-fluorophenyl)-1H-indazol-1-yl]acetic acid;
22. [3-(2-{[Acetyl(ethyl)amino]methyl}-4-fluorophenyl)-5-chloro-1H-indazol-1-yl]acetic acid;
23. {3-[2-{[[(Benzylamino)carbonyl](ethyl)amino]methyl}-4-(trifluoromethyl)phenyl]-4-chloro-1H-indazol-1-yl}acetic acid;
24. [5-Chloro-3-(2-{[(cyclopropylcarbonyl)(ethyl)amino]methyl}phenyl)-1H-indazol-1-yl]acetic acid;
25. [5-Chloro-3-(2-{[(cyclopentylcarbonyl)(ethyl)amino]methyl}-4-fluorophenyl)-1H-indazol-1-yl]acetic acid;
26. [5-Chloro-3-(2-{[(cyclopropylacetyl)(ethyl)amino]methyl}-4-fluorophenyl)-1H-indazol-1-yl]acetic acid;
27. {5-Chloro-3-[2-{[(cyclopropylcarbonyl)(ethyl)amino]methyl}-4-(trifluoromethyl)phenyl]-4-fluoro-1H-indazol-1-yl}acetic acid.

Preferably, Z does not represent a group of formula J or K: 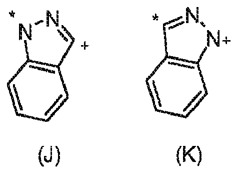 wherein the * and ⁺ are as defined above and said group is unsubstituted or substituted by one to four substituents selected from the group consisting of a halogen atom, a linear or branched C₁₋₄ alkyl group, a C₃₋₇ cycloalkyl group, a linear or branched 6₁₋₄ haloalkyl group, a -CN group, a -OH group, a -OR^{a} group, a -CO₂H group, a -CO₂R^{a} group, a -SR^{a} group, and a -SO₂R^{a} group.

Particular individual compounds of the invention include:
3-[2-{[(Cyclopropylearbonyl)(ethyl)amino]methyl}-4-(trifluoromethyl)phenyl]-1H-pyrrolo[3,2-b]pyridin-1-yl}acetic acid;
[3-(2-{[(Cyclopropylcarbonyl)(ethyl)amino]methyl}-4-fluorophenyl)-1H-pyrrolo[3,2-b]pyridin-1-yl]acetic acid;
{3-[2-{[(Cyclopropylcarbonyl)(ethyl)amino]methyl}-4-(trifluoromethyl)phenyl]-5-methyl-1H-pyrrolo[3,2-b]pyridin-1-yl}acetic acid;
2-{3-[2-{[(Cyclopropylcarbonyl)(ethyl)amino]methyl}-4-(trifluoromethyl)phenyl]-1H-pyrrolo[3,2-b]pyridin-1-yl}propanoicacid;
{5-Cyclopropyl-3-[2-{[(cyclopropylcarbonyl)(ethyl)amino]methyl}-4-(trifluoromethyl)phenyl]-1H-pyrrolo[3,2-b]pyridin-1-yl}acetic acid;
2-{3-[2-{[(Cyclopropylcarbonyl)(ethyl)amino]methyl}-4-(trifluoromethyl)phenyl]-5-methyl-1H-pyrrolo[3,2-b]pyridin-1-yl}propanoic acid;
[3-(2-{[(Cyclopropylcarbonyl)(ethyl)amino]methyl}-4-fluorophenyl)-5-(trifluoromethyl)-1H-pyrrolo[3,2-b]pyridin-1-yl]acetic acid;
{3-[2-{[[(Benzylamino)carbonyl](ethyl)amino]methyl}-4-(trifluoromethyl)phenyl]-5-methyl-1H-pyrrolo[3,2-b]pyridin-1-yl}acetic acid;
{3-[2-{[Benzyl(cyclopropylcarbonyl)amino]methyl}-4-(trifluoromethyl)phenyl]-5-methyl-1H-pyrrolo[3,2-b]pyridin-1-yl}acetic acid;
{3-[2-{[(Cyclopropylcarbonyl)(ethyl)amino]methyl}-4-(trifluoromethyl)phenyl]-1H-pyrrolo[3,2-c]pyridin-1-yl}acetic acid;
{3-[2-{[(Cyclopropylcarbonyl)(ethyl)amino]methyl}-4-(trifluoromethyl)phenyl]-2,4-dimethyl-1H-pyrrolo[3,2-c]pyridin-1-yl}acetic acid;
{1-[2-{[(Cyclopropylcarbonyl)(ethyl)amino]methyl}-4-(trifluoromethyl)phenyl]-1H-pyrrolo[2,3-b]pyridin-3-yl}acetic acid;
[1-(2-{[(Cyclopropylcarbonyl)(ethyl)amino]methyl}-4-fluorophenyl)-1H-pyrrolo[2,3-b]pyridin-3-yl]acetic acid;
{1-[2-{[(Cyclopropylcarbonyl)(ethyl)amino]methyl}-4-(trifluoromethyl)phenyl]-6-methyl-1H-pyrrolo[2,3-b]pyridin-3-yl}acetic acid;
{6-Cyclopropyl-1-[2-{[(cyclopropylcarbonyl)(ethyl)amino]methyl}-4-(trifluoromethyl)phenyl]-1H-pyrrolo[2,3-b]pyridin-3-yl}acetic acid;
[1-(2-{[(Cyclopropylcarbonyl)(ethyl)amino]methyl}-4-fluorophenyl)-6-methyl-1H-pyrrolo[2,3-b]pyridin-3-yl]acetic acid;
[1-(2-{[(Cyclopropylcarbonyl)(ethyl)amino]methyl}phenyl)-6-methyl-1H-pyrrolo[2,3-b]pyridin-3-yl]acetic acid;
{1-[2-{[(Cyclopropylcarbonyl)(ethyl)amino]methyl}-4-(trifluoromethyl)phenyl]-6-methoxy-1H-pyrrolo[2,3-b]pyridin-3-yl}acetic acid;
2-(1-(2-((N-Ethylcyclopropanecarboxamido)methyl)-4-(trifluoromethyl)phenyl)-6-(trifluoromethyl)-1H-pyrrolo[2,3-b]pyridin-3-yl)acetic acid;
{8-[2-{[(Cyclopropylcarbonyl)(ethyl)amino]methyl}-4-(trifluoromethyl)phenyl]pyrrolo[1,2-a]pyrimidin-6-yl}acetic acid;
[8-(2-{[(Cyclopropylcarbonyl)(ethyl)amino]methyl}-4-fluorophenyl)pyrrolo[1,2-a]pyrimidin-6-yl]acetic acid;
[8-[2-{[(Cyclopropylcarbonyl)(ethyl)amino]methyl}-4-(trifluoromethyl)phenyl]-2-(trifluoromethyl)pyrrolo[1,2-a]pyrimidin-6-yl]acetic acid;
{8-[2-{[(Cyclopropylcarbonyl)(ethyl)amino]methyl}-4-(trifluoromethyl)phenyl]-2-methylpyrrolo[1,2-a]pyrimidin-6-yl}acetic acid;
{3-[2-{[(Cyclopropylcarbonyl)(ethyl)amino]methyl}-4-(trifluoromethyl)phenyl]imidazo[1,5-a]pyridin-1-yl}acetic acid;
{8-[2-{[(Cyclopropylcarbonyl)(ethyl)amino]methyl}-4-(trifluoromethyl)phenyl]-1,7-dimethylpyrrolo[1,2-a]pyrazin-6-yl}acetic acid;
{7-[2-{[(Cyclopropylcarbonyl)(ethyl)amino]methyl}-4-(trifluoromethyl)phenyl]pyrrolo[1 ,2-a]pyrimidin-5-yl}acetic acid;
{7-[2-{[(Cyclopropylcarbonyl)(ethyl)amino]methyl}-4-(trifluoromethyl)phenyl]-1-methylpyrrolo[1,2-c]pyrimidin-5-yl}acetic acid;
or a pharmaceutically acceptable salt, N-oxide, solvate, stereiosomer or deuterated derivative thereof.

Of particular interest are the compounds:
{3-[2-{[(Cyclopropylcarbonyl)(ethyl)amino]methyl}-4-(trifluoromethyl)phenyl]-1H-pyrrolo[3,2-b]pyridin-1-yl}acetic acid;
[3-(2-{[(Cyclopropylcarbonyl)(ethyl)amino]methyl}-4-fluorophenyl)-1H-pyrrolo[3,2-b]pyridin-1-yl]acetic acid;
{3-[2-{[(Cyclopropylcarbonyl)(ethyl)amino]methyl}-4-(trifluoromethyl)phenyl]-5-methyl-1H-pyrrolo[3,2-b]pyridin-1-yl}acetic acid;
2-{3-[2-{[(Cyclopropylcarbonyl)(ethyl)amino]methyl}-4-(trifluoromethyl)phenyl]-5-methyl-1H-pyrrolo[3,2-b]pyridin-1-yl}propanoic acid;
[3-(2-{[(Cyclopropylcarbonyl)(ethyl)amino]methyl}-4-fluorophenyl)-5-(trifluoromethyl)-1H-pyrrolo[3,2-b]pyridin-1-yl]acetic acid;
{3-[2-{[[(Benzylamino)carbonyl](ethyl)amino]methyl}-4-(trifluoromethyl)phenyl]-5-methyl-1H-pyrrolo[3,2-b]pyridin-1-yl}acetic acid;
{3-[2-{[Benzyl(cyclopropylcarbonyl)amino]methyl}-4-(trifluoromethyl)phenyl]-5-methyl-1H-pyrrolo[3,2-b]pyridin-1-yl}acetic acid;
{1-[2-{[(Cyclopropylcarbonyl)(ethyl)amino]methyl}-4-(trifluoromethyl)phenyl]-1H-pyrrolo[2,3-b]pyridin-3-yl}acetic acid;
[1-(2-{[(Cyclopropylcarbonyl)(ethyl)amino]methyl}-4-fluorophenyl)-1H-pyrrolo[2,3-b]pyridin-3-yl]acetic acid;
{1-[2-{[(Cyclopropylcarbonyl)(ethyl)amino]methyl}-4-(trifluoromethyl)phenyl]-6-methyl-1H-pyrrolo[2,3-b]pyridin-3-yl}acetic acid;
[1-(2-{[(Cyclopropylcarbonyl)(ethyl)amino]methyl}-4-fluorophenyl)-6-methyl-1H-pyrrolo[2,3-b]pyridin-3-yl]acetic acid;
[1-(2-{[(Cyclopropylcarbonyl)(ethyl)amino]methyl}phenyl)-6-methyl-1H-pyrrolo[2,3-b]pyridin-3-yl]acetic acid;
2-(1-(2-((N-Ethylcyclopropanecarboxamido)methyl)-4-(trifluoromethyl)phenyl)-6-(trifluoromethyl)-1H-pyrrolo[2,3-b]pyridin-3-yl)acetic acid;
{8-[2-{[(Cyclopropylcarbonyl)(ethyl)amino]methyl}-4-(trifluoromethyl)phenyl]pyrrolo[1,2-a]pyrimidin-6-yl}acetic acid;
[8-(2-{[(Cyclopropylcarbonyl)(ethyl)amino]methyl}-4-fluorophenyl)pyrrolo[1 ,2-a]pyrimidin-6-yl]acetic acid;
[8-[2-{[(Cyclopropylcarbonyl)(ethyl)amino]methyl}-4-(trifluoromethyl)phenyl]-2-(trifluoromethyl)pyrrolo[1,2-a]pyrimidin-6-yl]acetic acid; or
{8-[2-{[(Cyclopropylcarbonyl)(ethyl)amino]methyl}-4-(trifluoromethyl)phenyl]-2-methylpyrrolo[1,2-a]pyrimidin-6-yl}acetic acid.

### GENERAL SYNTHETIC PROCEDURES

The compounds of the invention can be prepared using the methods and procedures described herein, or using similar methods and procedures. It will be appreciated that where typical or preferred process conditions (i.e., reaction temperatures, times, mole ratios of reactants, solvents, pressures, etc.) are given; other process conditions can also be used unless otherwise stated. Optimum reaction conditions may vary with the particular reactants or solvent used, but such conditions can be determined by one skilled in the art by routine optimization procedures.

Additionally, as will be apparent to those skilled in the art, conventional protecting groups may be necessary to prevent certain functional groups from undergoing undesired reactions. The choice of a suitable protecting group for a particular functional group, as well as suitable conditions for protection and de-protection, are well known in the art. For example, numerous protecting groups, and their introduction and removal are described in T. W. Greene and G. M. Wuts, Protecting Groups in Organic Synthesis, Third Edition, Wiley, New York, 1999, and references cited therein. Processes for preparing compounds of the invention are provided as further embodiments of the invention and are illustrated by the procedures below.

R¹, R², R³, R⁴, R^{a}, R^{b}, R^{c}, G and Z are defined according to claim 1. R⁸, R⁹, R¹⁰, R¹¹ and R¹² represent a hydrogen atom, a halogen atom, a linear or branched C₁₋₄ alkyl group, a 6₃₋₇ cycloalkyl group, a linear or branched C₁₋₄ haloalkyl group, a -CN group, a -OH group, a -OR^{a} group, a -CO₂H group, a -CO₂R^{a} group, a - SR^{a} group, or a -SO₂R^{a} group.

Specific synthetic processes not covered by Schemes 1 - 15 are described in detail in the Experimental section.

In the case wherein R² is a -N(R³)COR⁴ group or a -N(R³)SO₂R⁴ group, intermediates of the general formula (VIII) may be prepared following the synthetic scheme depicted in Scheme 1.

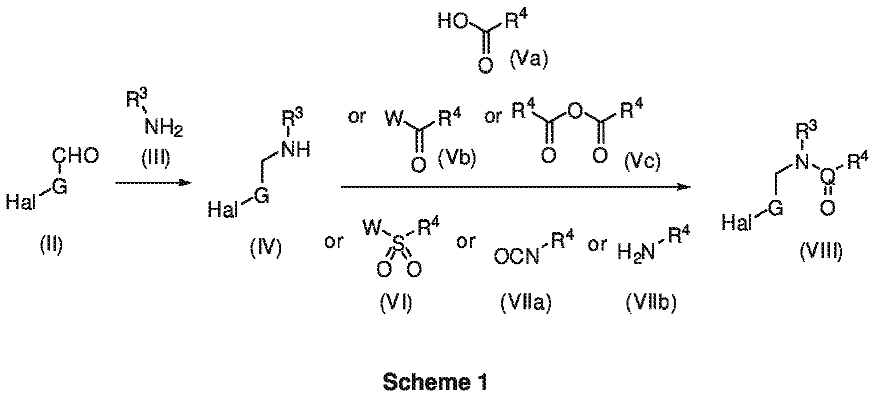

Compounds of general formula (IV) wherein Hal represents a halogen atom such as chlorine, bromine or iodine may be prepared by mixing compounds of formula (II) and an amine of formula (III) in the presence of a reducing agent such as sodium cyanoborohydride in the presence of an acid such as acetic acid in a solvent such as methanol, tetrahydrofuran or mixtures thereof at a temperature from 0 ºC to the boiling point of the solvent.

Compounds of general formula (VIII) wherein Q represents a carbon or a S=O group may be prepared from compounds of Formula (IV) and an appropriate acylating or sulfonylating agent such as:
- a carboxylic acid of formula (Va) in the presence of a peptide coupling reagent such as dicyclohexylcarbodiimide,
- an acyl halide of formula (Vb) wherein W represents a halogen atom such as fluorine, chlorine or bromine,
- an anhydride such of formula (Vc),
- a sulfonyl halide of formula (VI) wherein W is as hereinbefore defined,
- an isocyanate of formula (VIIa) or
- an amine of formula (VIIb) in the presence of a phosgene equivalent such as phosgene, triphosgene or carbonyl diimidazole.

The reaction is carried out by mixing a compound of formula (IV) and a compound of formula (Va) - (VIIb) according to standard literature methods known to those skilled in the art of formation of amide, carbamates, ureas and sulfonamides to yield the compound of formula (VIII).

In the particular case wherein R² is a substituted isoxazolyl group or a substituted pyrazolyl group, intermediates of the general formula (XII) and (XIV) wherein R⁵ and R⁶ each independently represent a group selected from a -CF₃ group, a linear or branched C₁₋₄ alkyl group, a C₃₋₇ cycloalkyl group, a benzyl group, a phenyl group, a hydroxy group or a carboxy group and R⁷ represents a -CF₃ group, a linear or branched C₁₋₄ alkyl group, a C₃₋₇ cycloalkyl group, a benzyl group or a phenyl group, may be prepared following the synthetic scheme depicted in Scheme 2.

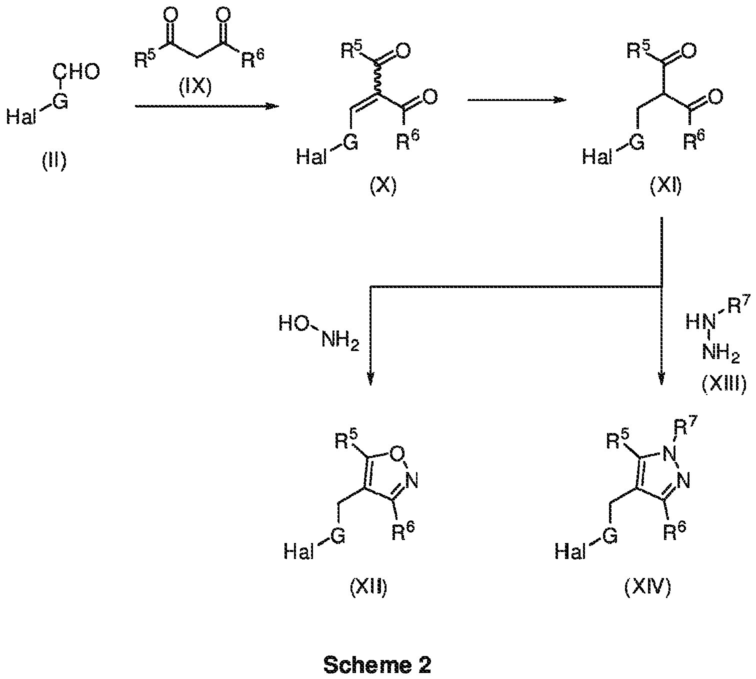

Compounds of general formula (X) may be prepared by mixing compounds of formula (II) and a dicarbonyl of formula (IX) in the presence of a base such as piperidine and acid such as acetic acid and in the presence or absence of a dessicant such as molecular sieves in a solvent such as ethanol at a temperature from ambient to the boiling point of the solvent to yield the compound of formula (X).

Compounds of general formula (XI) may be prepared by mixing a compound of formula (X) in the presence of a reducing agent such as zinc metal in the presence of an acid such as acetic acid at a temperature from 0 ºC to the boiling point of the solvent.

Compounds of general formula (XII) may be prepared by mixing a compound of formula (XI) with hydroxylamine hydrochloride in a solvent such as ethanol at a temperature from 0 ºC to the boiling point of the solvent.

Compounds of general formula (XIV) may be prepared by mixing a compound of formula (XI) with a hydrazine hydrochloride of formula (XIII) in the presence of an acid such as acetic acid at a temperature from 0 ºC to the boiling point of the solvent.

In the particular case wherein R² is a substituted triazolyl, intermediates of the general formula (XIX) wherein R⁵ and R⁶ are as hereinbefore defined may be prepared following the synthetic scheme depicted in Scheme 3.

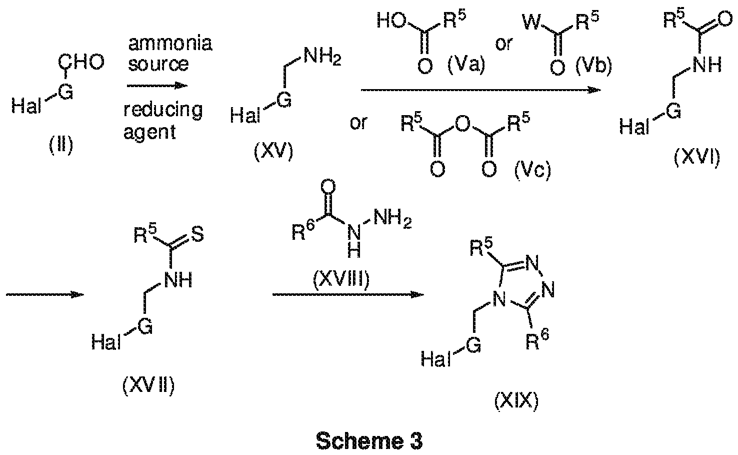

Compounds of general formula (XV) may be prepared from compounds of formula (II) and an ammonia source in the presence of a reducing agent.

In one example, the reaction is carried out by mixing a compound of formula (II) and an ammonia source such as ammonium acetate in the presence of a reducing agent such as sodium cyanoborohydride in the presence of an acid such as acetic acid in a solvent such as methanol, tetrahydrofuran or mixtures thereof at a temperature from 0 ºC to the boiling point of the solvent to yield the compound of formula (XV).

In another example, the reaction is carried out by mixing a compound of formula (II) and hydroxylamine in a solvent such as methanol, followed by the subsequent addition of a reducing agent such as zinc metal in the presence of an acid such as acetic acid at a temperature from 0 ºC to the boiling point of the solvent to yield the compound of formula (XV).

Compounds of general formula (XVI) may be prepared from compounds of formula (XV) and appropriate acylating agent such as:
- a carboxylic acid of formula (Va) wherein R⁵ is as hereinbefore defined in the presence of a peptide coupling reagent such as dicyclohexylcarbodiimide,
- an acyl halide of formula (Vb) wherein W represents a halogen atom such as fluorine, chloride or bromine and wherein R⁵ is as hereinbefore defined or
- an anhydride such of formula (Vc) wherein R⁵ is as hereinbefore defined.

The reaction is carried out by mixing a compound of formula (XVI) and compound of formula (Va) - (Vc) according to standard literature methods known to those skilled in the art of formation of amides to yield the compound of formula (XVI).

Compounds of general formula (XVII) may be prepared by mixing a compound of formula (XVI) and a thionating agent such as phosphorus pentasulfide in the presence of a base such as diisopropylethylamine in a solvent such as tetrahydrofuran at a temperature from 0°C to the boiling point of the solvent to yield the compound of formula (XVII).

Compounds of general formula (XIX) may be prepared by mixing a compound of formula (XVII) and an acyl hydrazide of formula (XVIII) in the presence of a Lewis acid such as mercuric(II) acetate in a solvent such as acetonitrile at a temperature from 0ºC to the boiling point of the solvent to yield the compound of formula (XIX).

Intermediates of the general formula (XXI) and (XXIII) may be prepared following the synthetic scheme depicted in Scheme 4.

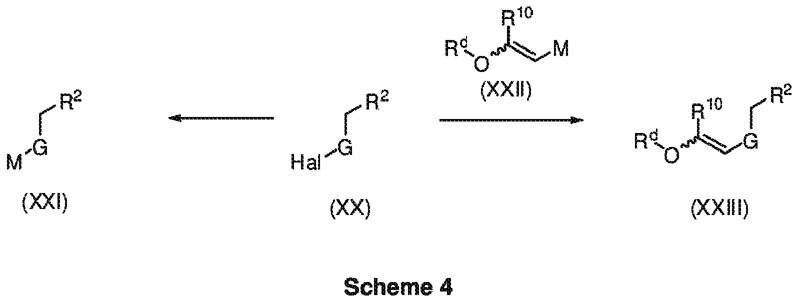

Compounds of general formula (XXI) wherein M represents lithium or salts or complexes of magnesium, zinc, copper, tin or boron may be prepared by treating a compound of formula (XX) according to standard literature methods known to those skilled in the art of transmetalation. For example, in one instance the reaction can be carried out by treating a compound of formula (XX) with 4,4,4',4',5,5,5',5'-octamethyl-2,2'-bis(1 ,3,2-dioxaborolane) in the presence of a suitable transition metal catalyst such as [1,1'-bis(diphenylphosphino)-ferrocene]dichloropalladium(II) in the presence of a base such as potassium acetate in a solvent such as dioxane under an inert atmosphere such as argon at a temperature from 50 ºC to the boiling point of the solvent to yield the compound of formula (XXI).

Compounds of general formula (XXIII) wherein R^{d} represents an alkyl group such as methyl, ethyl or propyl may be prepared from compounds of formula (XX) with an organometallic reagent of formula (XXII) wherein M represents lithium or salts or complexes of magnesium, zinc, copper, tin or boron. The reaction is carried out by treating a compound of formula (XX) according to standard literature methods known to those skilled in the art of carbon-carbon cross-coupling reactions. For example, in one instance the reaction can be carried out by treating a compound of formula (XX) with a vinyl stanane of formula (XXII) wherein M is tributyl tin in the presence of a suitable transition metal catalyst such as tetrakis(triphenylphosphine)palladium in a solvent such as toluene under an inert atmosphere such as argon at a temperature from ambient to the boiling point of the solvent to yield the compound of formula (XXIII).

In the particular case wherein Z is a 4-azaindolyl group, compounds of the general formula (Ia) may be prepared following the synthetic scheme depicted in Scheme 5.

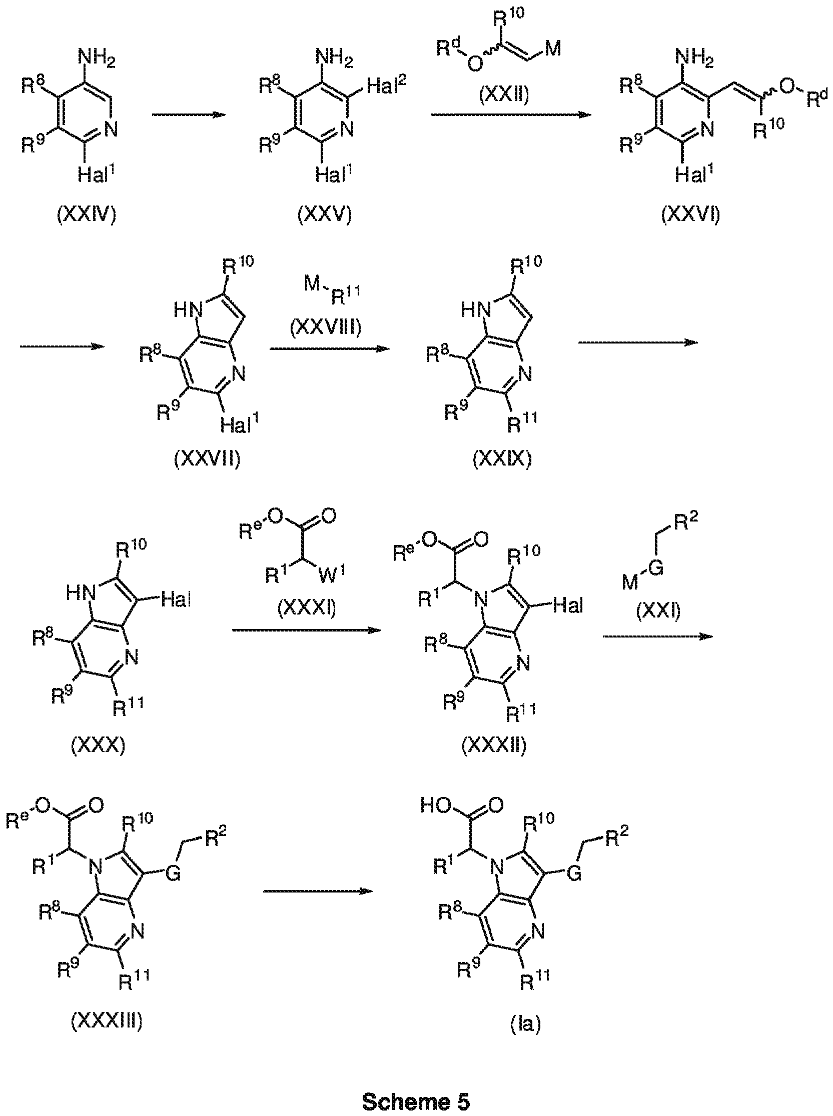

Compounds of formula (XXIV) are prepared according to standard literature methods and specific examples are described in the experimental section.

Compounds of general formula (XXV) wherein Hal¹ represents a halogen atom such as chlorine or bromine and Hal² represents a halogen atom such as bromine or iodine may be prepared from compounds of formula (XXIV) by halogenation.

The reaction is carried out by mixing a compound of formula (XXIV) and a halogenating reagent such as N-iodosuccinimide, in a solvent such as dimethylformamide at a temperature from 0 ºC to the boiling point of the solvent to yield the compound of formula (XXV).

Compounds of general formula (XXVI) wherein R^{d} represents an alkyl group such as methyl, ethyl or propyl may be prepared from compounds of formula (XXV) and an organometallic reagent of formula (XXII) wherein M represents lithium or salts or complexes of magnesium, zinc, copper, tin or boron.

The reaction is carried out by treating a compound of formula (XXV) according to standard literature methods known to those skilled in the art of carbon-carbon cross-coupling reactions. For example, in one instance the reaction can be carried out by treating a compound of formula (XXV) with a borane of formula (XXII) such as tris((E)-2-ethoxyvinyl)borane in the presence of a suitable transition metal catalyst such as tetrakis(triphenylphosphine)palladium and a base such as sodium hydroxide in a solvent such as tetrahydrofuran under an inert atmosphere such as argon at a temperature from ambient to the boiling point of the solvent to yield the compound of formula (XXVI).

Compounds of general formula (XXVII) may be prepared by cyclisation, treating a compound of formula (XXVI) with an acid such as hydrochloric acid in a solvent such as water or methanol or mixtures thereof at a temperature from ambient to the boiling point of the solvent.

Compounds of general formula (XXIX) may be prepared from compounds of formula (XXVII) and an organometallic reagent of formula (XXVIII) wherein M represents lithium or salts or complexes of magnesium, zinc, copper, tin or boron.

The reaction is carried out by treating a compound of formula (XXVII) according to standard literature methods known to those skilled in the art of carbon-carbon cross-coupling reactions. For example, in one instance the reaction can be carried out by treating a compound of formula (XXVII) with a compound of formula (XXVIII) where M is boronic acid, boronate ester or boroxine in the presence of a suitable transition metal catalyst such as [1,1'-bis(diphenylphosphino)ferrocene]dichloropalladium(II) or palladium(II) acetate, in the presence or absence of suitable ligands such as tricyclohexylphosphine, and with a base such as potassium carbonate or potassium phosphate in a solvent such as dimethyoxyethane, toluene, water or mixtures thereof under an inert atmosphere such as argon at a temperature from ambient to 150 ºC to yield the compound of formula (XXIX).

Compounds of general formula (XXX) wherein Hal represents a halogen atom such as chlorine, bromine or iodine may be prepared by mixing a compound of formula (XXIX) and a halogenating reagent such as copper(II) bromide, in a solvent such as acetonitrile at a temperature from 0 ºC to the boiling point of the solvent.

Compounds of general formula (XXXII) wherein R^{e} represents an alkyl group such as methyl, ethyl, propyl, tert-butyl or benzyl may be prepared from compounds of formula (XXX) and an alkylating agent of formula (XXXI) wherein W¹ represents a halogen atom such as chlorine, bromine or iodine or a pseudohalogen such as paratoluenesulphonate.

The reaction is carried out by mixing a compound of formula (XXX) with an alkylating agent of formula (XXXI) in the presence of a base such as sodium hydride or caesium carbonate in a solvent such as dimethylformamide at a temperature from 0 ºC to the boiling point of the solvent to yield the compound of formula (XXXII).

Compounds of general formula (XXXIII) may be prepared from compounds of formula (XXXII) with an organometallic reagent of formula (XXI) wherein M represents lithium or salts or complexes of magnesium, zinc, copper, tin or boron.

The reaction is carried out by treating a compound of formula (XXXII) according to standard literature methods known to those skilled in the art of carbon-carbon cross-coupling reactions. For example, in one instance the reaction can be carried out by treating a compound of formula (XXXII) with a boronate ester of formula (XXI) wherein M is 4,4,5,5-tetramethyl-13,2-dioxaborolan-2-yl in the presence of a transition metal catalyst such as [1,1'-bis(diphenylphosphino)ferrocene]dichloropalladium(II) in the presence of a base such as caesium carbonate in a solvent such as dioxane at a temperature from ambient to the boiling point of the solvent under an inert atmosphere such as argon to yield the compound of formula (XXXIII).

Compounds of general formula (Ia) may be prepared from compounds of formula (XXXIII) by ester hydrolysis.

The reaction may be carried out by treating a compound of formula (XXXIII) with an acid such as trifluoroacetic acid or hydrochloric acid and in a solvent such as chloroform, dichloromethane or dioxane at a temperature from 0 ºC to the boiling point of the solvent to yield the compound of formula (Ia). Alternatively, the reaction may be carried out by treating a compound of formula (XXXIII) with a base such as lithium hydroxide in a solvent such as water, tetrahydrofuran or mixtures thereof, at a temperature from 0 ºC to the boiling point of the solvent to yield the compound of formula (Ia).

Intermediates of formula (XXIX) may also be prepared following the synthetic scheme depicted in Scheme 6.

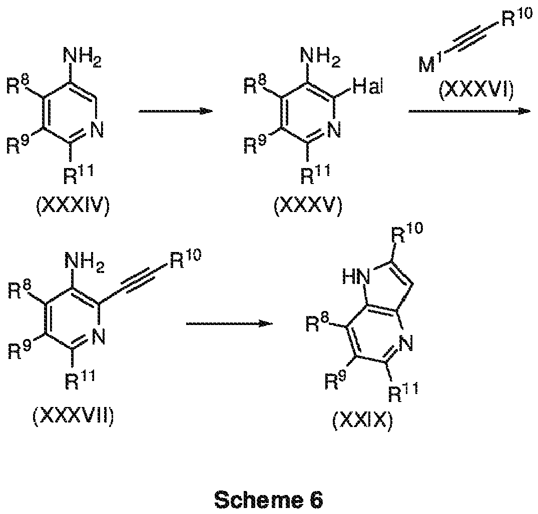

Compounds of formula (XXXIV) are prepared according to standard literature methods and specific examples are described in the experimental section.

Compounds of general formula (XXXV) wherein Hal represents a halogen atom such as chlorine, bromine or iodine may be prepared from compounds of formula (XXXIV) by halogenation. The reaction is carried out by mixing a compound of formula (XXXIV) and a halogenating reagent such as N-bromosuccinimide, in a solvent such as acetonitrile at a temperature from -15 ºC to the boiling point of the solvent to yield the compound of formula (XXXV).

Compounds of general formula (XXXVII) may be prepared from compounds of formula (XXXV) and an alkyne of formula (XXXVI) wherein M¹ represents hydrogen, lithium, magnesium salts such as magnesium chloride or magnesium bromide, zinc salts such as zinc chloride or zinc bromide, or trialkyl tin groups such as trimethyl tin or tributyl tin. The reaction is carried out by treating a compound of formula (XXXV) according to standard literature methods known to those skilled in the art of carbon-carbon cross-coupling reactions. For example, in one instance the reaction can be carried out by treating a compound of formula (XXXV) with an alkyne of formula (XXXVI) wherein M¹ is hydrogen with a one or more transition metal catalysts such as copper(I) iodide or bis(triphenylphosphine)-palladium(II) dichloride or mixtures thereof in the presence of a base such as triethylamine in a solvent such as tetrahydrofuran at a temperature from 0 ºC to the boiling point of the solvent under an inert atmosphere such as argon to yield the compound of formula (XXXVII).

Compounds of general formula (XXIX) may be prepared from compounds of formula (XXXVII) by cyclisation. The reaction is carried out by treating a compound of formula (XXXVII) with a base such as potassium tert-butoxide in a solvent such as N-methylpyrrolidone at a temperature from ambient to the boiling point of the solvent to yield the compound of formula (XXIX).

In the particular case where Z is a 5-azaindolyl group, compounds of the general formula (Ib) may be prepared following the synthetic scheme depicted in Scheme 7.

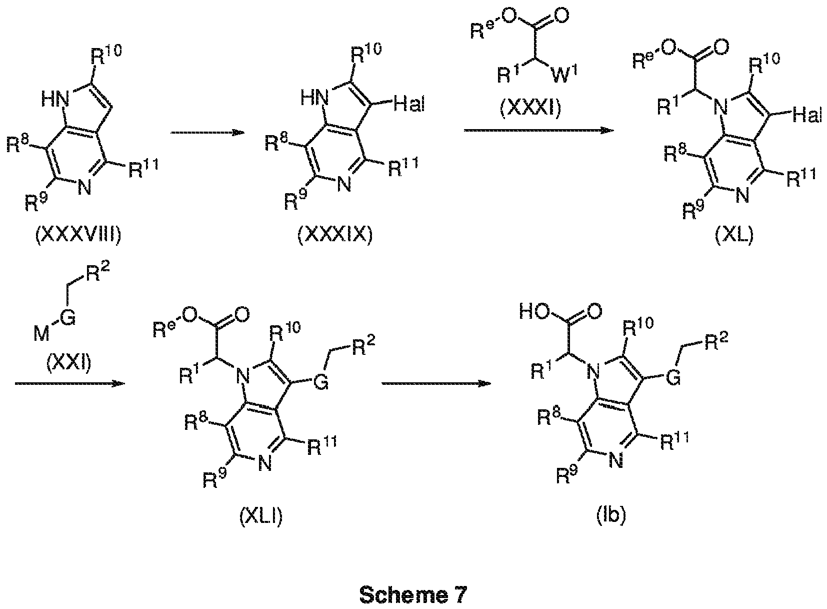

Compounds of formula (XXXVIII) are prepared according to standard literature methods and specific examples are described in the experimental section.

Compounds of general formula (XXXIX) wherein Hal represents a halogen atom such as chlorine, bromine or iodine may be prepared from compounds of formula (XXXVIII) by halogenation. The reaction is carried out by mixing a compound of formula (XXXVIII) and a halogenating reagent such as copper(II) bromide, in a solvent such as acetonitrile at a temperature from 0 ºC to the boiling point of the solvent to yield the compound of formula (XXXIX).

Compounds of general formula (XL) may be prepared from compounds of formula (XXXIX) and an alkylating agent of formula (XXXI) wherein W¹ represents a halogen atom such as chlorine, bromine or iodine or a pseudohalogen such as paratoluenesulphonate.

The reaction is carried out by mixing a compound of formula (XXXIX) with an alkylating agent of formula (XXXI) in the presence of a base such as sodium hydride or caesium carbonate in a solvent such as dimethylformamide at a temperature from 0 ºC to the boiling point of the solvent to yield the compound of formula (XL).

Compounds of general formula (XLI) may be prepared from compounds of formula (XL) with an organometallic reagent of formula (XXI) wherein M represents lithium or salts or complexes of magnesium, zinc, copper, tin or boron. The reaction is carried out by treating a compound of formula (XL) according to standard literature methods known to those skilled in the art of carbon-carbon cross-coupling reactions. For example, in one instance the reaction can be carried out by treating a compound of formula (XL) with a boronate ester of formula (XXI) wherein M is 4,4,5,5-tetramethyl-1,3,2-dioxaborolan-2-yl in the presence of a transition metal catalyst such as [1,1'-bis(diphenylphosphino)ferrocene]dichloropalladium(II) in the presence of a base such as caesium carbonate in a solvent such as dioxane at a temperature from ambient to the boiling point of the solvent under an inert atmosphere such as argon to yield the compound of formula (XLI).

Compounds of general formula (Ib) may be prepared from compounds of formula (XLI) by ester hydrolysis. The reaction may be carried out by treating a compound of formula (XLI) with an acid such as trifluoroacetic acid or hydrochloric acid and in a solvent such as chloroform, dichloromethane or dioxane at a temperature from 0 ºC to the boiling point of the solvent to yield the compound of formula (Ib). Alternatively, the reaction may be carried out by treating a compound of formula (XLI) with a base such as lithium hydroxide in a solvent such as water, tetrahydrofuran or mixtures thereof, at a temperature from 0 ºC to the boiling point of the solvent to yield the compound of formula (Ib).

Intermediates of formula (XLI) may also be prepared following the synthetic scheme depicted in Scheme 8.

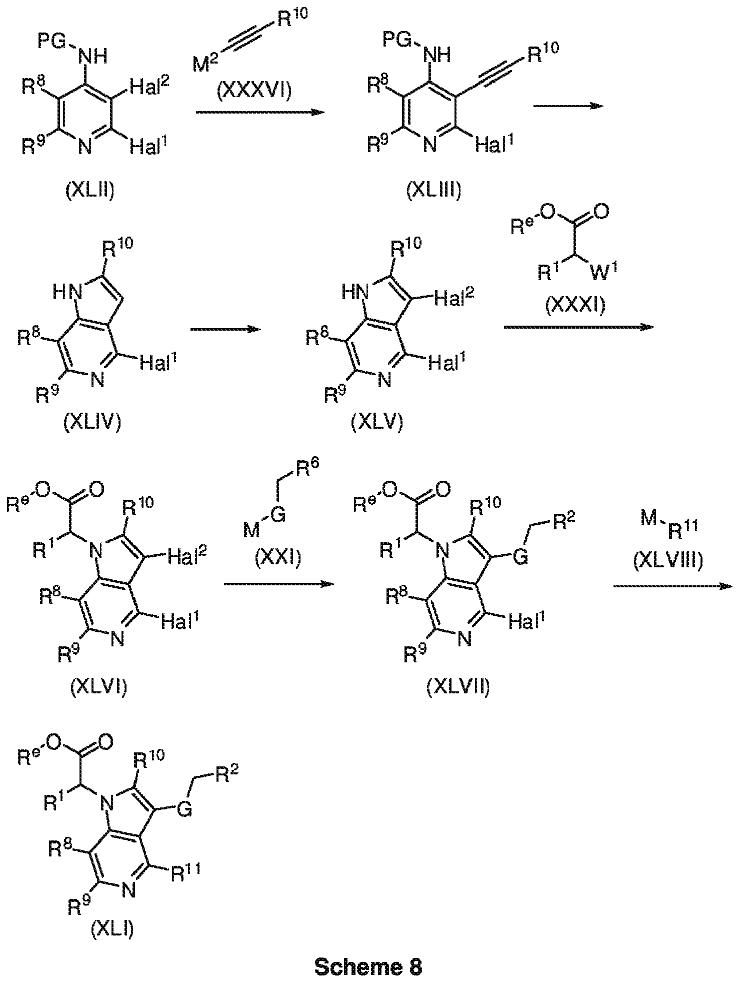

Compounds of formula (XLII) are prepared according to standard literature methods and specific examples are described in the experimental section.

Compounds of general formula (XLIII) may be prepared from compounds of formula (XLII) wherein Hal¹ represents a halogen atom such as chlorine or bromine, Hal² represents a halogen atom such as bromine or iodine and PG represents an amine protecting group such as ethoxycarbonyl, benzyloxycarbonyl, tert-butoxycarbonyl, para-toluenesulfonate or benzenesulfonate.

The reaction is carried out by treating a compound of formula (XLII) with an alkyne of formula (XXXVI) wherein M² represents hydrogen, lithium or salts or complexes of magnesium, zinc, tin or boron, according to standard literature methods known to those skilled in the art of carbon-carbon cross-coupling reactions. For example, in one instance the reaction can be carried out by treating a compound of formula (XLII) with an alkyne of formula (XXXVI) wherein M is hydrogen with one or more transition metal catalysts such as copper(I) iodide or bis(triphenylphosphine)-palladium(II) dichloride or mixtures thereof in the presence of a base such as triethylamine in a solvent such as tetrahydrofuran at a temperature from 0 ºC to the boiling point of the solvent under an inert atmosphere such as argon to yield the compound of formula (XLIII).

Compounds of general formula (XLIV) may be prepared from compounds of formula (XLIII) by cyclisation. The reaction is carried out by treating a compound of formula (XLIII) with a metal catalyst such as copper(I) iodide in a solvent such as dimethylformamide at a temperature from ambient to the boiling point of the solvent to yield the compound of formula (XLIV).

Compounds of general formula (XLV) may be prepared by mixing a compound of formula (XLIV) and a halogenating reagent such as N-iodosuccinimide, in a solvent such as dimethylformamide at a temperature from 0 ºC to the boiling point of the solvent to yield the compound of formula (XLV).

Compounds of general formula (XLVI) may be prepared from compounds of formula (XLV) and an alkylating agent of formula (XXXI). The reaction is carried out by mixing a compound of formula (XLV) with an alkylating agent of formula (XXXI) in the presence of a base such as sodium hydride or caesium carbonate in a solvent such as dimethylformamide at a temperature from 0 ºC to the boiling point of the solvent to yield the compound of formula (XLVI).

Compounds of general formula (XLVII) may be prepared from compounds of formula (XLVI) wherein with an organometallic reagent of formula (XXI) wherein M represents lithium or salts or complexes of magnesium, zinc, copper, tin or boron. The reaction is carried out by treating a compound of formula (XLVI) according to standard literature methods known to those skilled in the art of carbon-carbon cross-coupling reactions. For example, in one instance the reaction can be carried out by treating a compound of formula (XLVI) with a boronate ester of formula (XXI) wherein M is 4,4,5,5-tetramethyl-1,3,2-dioxaborolan-2-yl in the presence of a transition metal catalyst such as bis(dibenzylideneacetone)palladium in the presence of a ligand such as 2-di(cyclohexyl)phosphino-2',6'-dimethoxybiphenyl (S-Phos) a base such as potassium phosphate in a solvent such as dimethylformamide at a temperature from ambient to the boiling point of the solvent under an inert atmosphere such as argon to yield the compound of formula (XLVII).

Compounds of general formula (XLI) may be prepared from compounds of formula (XLVII) with an organometallic reagent of formula (XLVIII) wherein M represents lithium or salts or complexes of magnesium, zinc, copper, tin or boron. The reaction is carried out by treating a compound of formula (XLVII) according to standard literature methods known to those skilled in the art of carbon-carbon cross-coupling reactions. For example, in one instance the reaction can be carried out by treating a compound of formula (XLVII) with an organometallic reagent of formula (XLVIII) wherein M is boronic acid in the presence of a transition metal catalyst such as [1,1'-bis(diphenylphosphino)ferrocene]dichloropalladium(II) in the presence of a base such as caesium carbonate in a solvent such as dimethoxyethane at a temperature from ambient to the boiling point of the solvent under an inert atmosphere such as argon to yield the compound of formula (XLI).

In the particular case where Z is a 7-azaindolyl group, compounds of the general formula (Ic) may be prepared following the synthetic scheme depicted in Scheme 9. 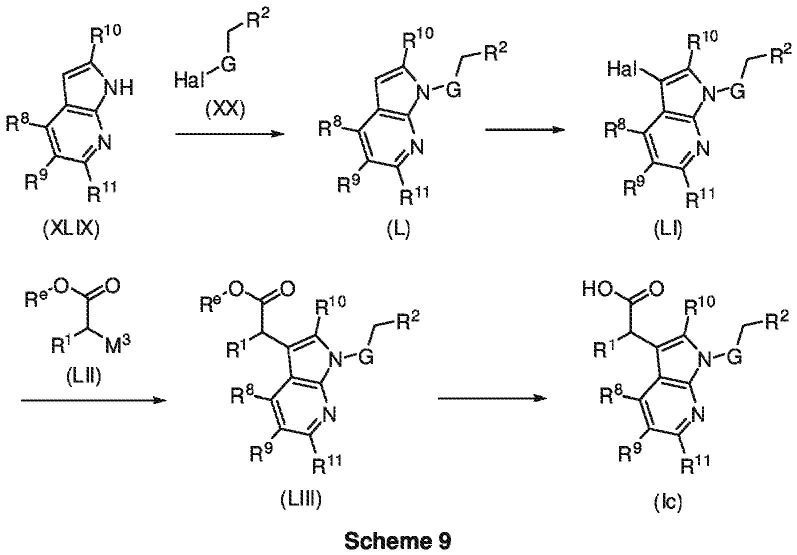 Compounds of formula (XLIX) are prepared according to standard literature methods and specific examples are described in the experimental section.

Compounds of general formula (L) may be prepared from compounds of formula (XLIX) and compounds of formula (XX) wherein Hal represents a halogen atom such as chlorine, bromine or iodine. The reaction is carried out by treating a compound of formula (XLIX) with a compound of formula (XX) in the presence of a transition metal catalyst such as copper(I) iodide or copper(I) oxide, a suitable ligand such as trans-N,N'-dimethylcyclohexane-1,2-diamine, N,N'-dimethylethylenediamine or 2-hydroxybenzaldehyde oxime and a base such as potassium phosphate or caesium carbonate in a solvent such as dioxane or acetonitrile at a temperature from ambient to 150 ºC under an inert atmosphere such as argon to yield the compound of formula (L).

Compounds of general formula (LI) may be prepared from compounds of formula (L) by halogenation. The reaction is carried out by mixing a compound of formula (L) and a halogenating reagent such as N-bromosuccinimide, in a solvent such as ethyl acetate at a temperature from -15 ºC to the boiling point of the solvent to yield the compound of formula (LI).

Compounds of general formula (LIII) may be prepared from compounds of formula (LI) and an organometallic reagent of formula (LII) wherein M³ represents lithium or salts or complexes of magnesium or zinc. The reaction is carried out by treating a compound of formula (LI) according to standard literature methods known to those skilled in the art of carbon-carbon cross-coupling reactions. For example, in one instance the reaction can be carried out by treating a compound of formula (LI) with an organometallic reagent of formula (LII) wherein M³ is zinc chloride in the presence of a transition metal catalyst such as bis(dibenzylidene-acetone)palladium and a ligand such as pentaphenylferrocenyl di-tert-butylphosphine (Q-Phos) in a solvent such as tetrahydrofuran at a temperature from ambient to 150 ºC under an inert atmosphere such as argon to yield the compound of formula (LIII).

Compounds of general formula (Ic) may be prepared from compounds of formula (LIII) by ester hydrolysis. The reaction may be carried out by treating a compound of formula (LIII) with an acid such as trifluoroacetic acid or hydrochloric acid and in a solvent such as chloroform, dichloromethane or dioxane at a temperature from 0 ºC to the boiling point of the solvent to yield the compound of formula (Ic). Alternatively, the reaction may be carried out by treating a compound of formula (LIII) with a base such as lithium hydroxide in a solvent such as water, tetrahydrofuran or mixtures thereof, at a temperature from 0 ºC to the boiling point of the solvent to yield the compound of formula (Ic).

Intermediates of formula (XLIX) may also be prepared following the synthetic scheme depicted in Scheme 10.

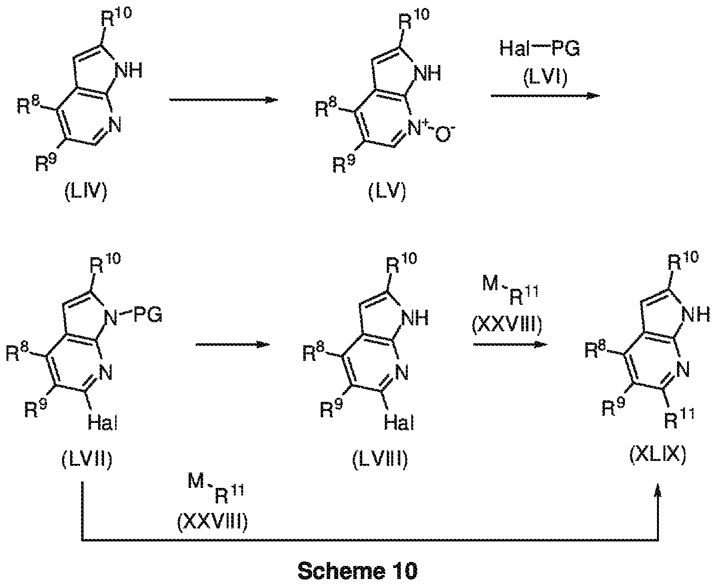

Compounds of formula (LIV) are prepared according to standard literature methods and specific examples are described in the experimental section.

Compounds of general formula (LV) may be prepared from compounds of formula (LIV) by oxidation. The reaction is carried out by mixing a compound of formula (LIV) and an oxidising agent such as meta-chloroperbenzoic acid in a solvent such as dichloromethane at a temperature from -15 ºC to the boiling point of the solvent to yield the compound of formula (LV).

Compounds of general formula (LVII) wherein Hal represents a halogen atom such as chlorine, bromine or iodine and PG represents an amine protecting group such as methoxycarbonyl, ethoxycarbonyl, benzyloxycarbonyl or tert-butoxycarbonyl may be prepared from compounds of formula (LV) and a reagent of formula (LVI). The reaction can be carried out by mixing a compound of formula (LV) with a reagent of formula (LVI) in the presence of a base such as 1,1,1,3,3,3-hexametildisilazane in a solvent such as tetrahydrofuran at a temperature from 0 ºC to the boiling point of the solvent under an inert atmosphere such as nitrogen to yield the compound of formula (LVII).

Compounds of general formula (LVIII) may be prepared from compounds of formula (LVII) by hydrolysis. The reaction can be carried out by mixing a compound of formula (LVII) with a base such as sodium hydroxide in a solvent such as methanol or water or mixtures thereof at a temperature from 0 ºC to the boiling point of the solvent to yield the compound of formula (LVIII).

Compounds of general formula (XLIX) may be prepared from compounds of formula (LVIII) with an organometallic reagent of formula (XXVIII) wherein M represents lithium or salts or complexes of magnesium, zinc, copper, tin or boron. The reaction is carried out by treating a compound of formula (LVIII) according to standard literature methods known to those skilled in the art of carbon-carbon cross-coupling reactions. For example, in one instance the reaction can be carried out by treating a compound of formula (LVIII) with an organometallic reagent of formula (XXVIII) wherein M is a boronic acid or boronic anhydride in the presence of a transition metal catalyst such as [1,1'-bis(diphenylphosphino)ferrocene]dichloro-palladium(II) or palladium(II) acetate, in the presence or absence of a suitable ligand such as tricyclohexylphosphine, in the presence of a base such as potassium phosphate caesium carbonate in a solvent such as dimethoxyethane, toluene, water or mixtures thereof at a temperature from ambient to 150 ºC under an inert atmosphere such as argon to yield the compound of formula (XLIX).

Compounds of general formula (XLIX) may also be prepared from compounds of formula (LVII) with an organometallic reagent of formula (XXVIII) wherein M represents lithium or salts or complexes of magnesium, zinc, copper, tin or boron. The reaction is carried out by treating a compound of formula (LVII) according to standard literature methods known to those skilled in the art of carbon-carbon cross-coupling reactions. in one instance the reaction can be carried out by treating a compound of formula (LVII) with an organometallic reagent of formula (XXVIII) wherein M is zinc in the presence of a transition metal catalyst such as [1,1'-bis(diphenylphosphino)ferrocene]-dichloropalladium(II), in the presence or absence of a suitable ligand such as tricyclohexylphosphine in a solvent such as dioxane, hexane or mixtures thereof at a temperature from ambient to 150 ºC under an inert atmosphere such as argon to yield the compound of formula (XLIX).

In the particular case where Z is a pyrrolo[1,2-a]pyrimidinyl group, compounds of the general formula (Id) may be prepared following the synthetic scheme depicted in Scheme 11.

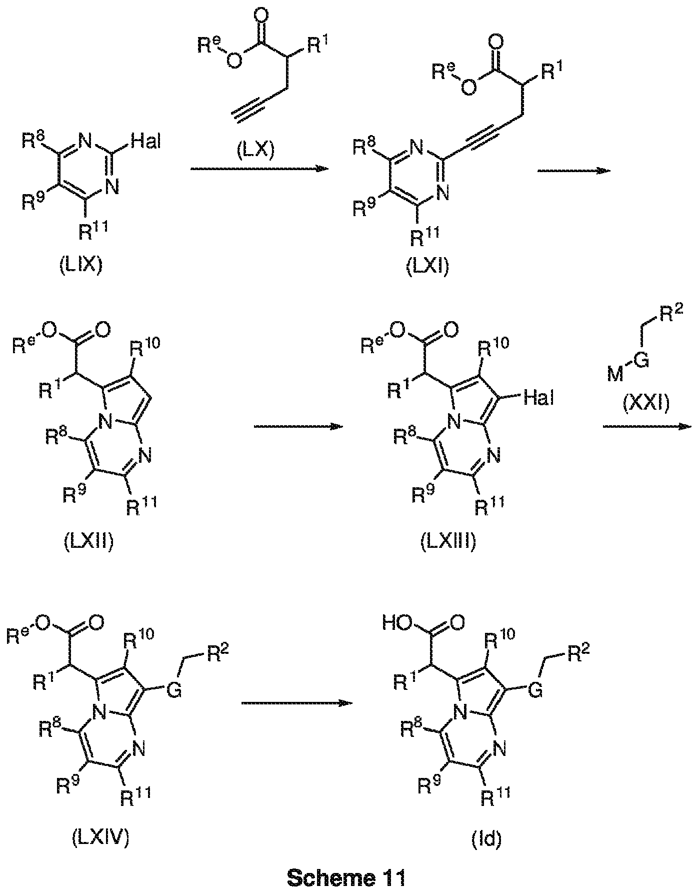

Compounds of formula (LIX) are prepared according to standard literature methods and specific examples are described in the experimental section.

Compounds of general formula (LXI) may be prepared from compounds of formula (LIX) and an alkyne of formula (LX) wherein R^{a} is as hereinbefore defined. The reaction is carried out by treating a compound of formula (LIX) with an alkyne of formula (LX) with a transition metal catalysts such as copper(I) iodide or bis(triphenylphosphine)palladium(II) dichloride or mixtures thereof in the presence of a base such as triethylamine in a solvent such as tetrahydrofuran at a temperature from 0 ºC to the boiling point of the solvent under an inert atmosphere such as argon to yield the compound of formula (LXI).

Compounds of general formula (LXII) may be prepared by treating a compound of formula (LXI) with a metal catalyst such as copper(I) iodide or copper(I) bromide in the presence of a base such as triethylamine in a solvent such as dimethylacetamide at a temperature from ambient to the boiling point of the solvent to yield the compound of formula (LXII).

Compounds of general formula (LXIII) may be by mixing a compound of formula (LXII) and a halogenating reagent such as N-bromosuccinimide, in a solvent such as dimethylformamide or dichloromethane at a temperature from -15 ºC to 50 ºC to yield the compound of formula (LXIII).

Compounds of general formula (LXIV) may be prepared from compounds of formula (LXIII) with an organometallic reagent of formula (XXI) wherein M represents lithium or salts or complexes of magnesium, zinc, copper, tin or boron. The reaction is carried out by treating a compound of formula (LXIII) according to standard literature methods known to those skilled in the art of carbon-carbon cross-coupling reactions. For example, in one instance the reaction can be carried out by treating a compound of formula (LXIII) with a boronate ester of formula (XXI) wherein M is 4,4,5,5-tetramethyl-1,3,2-dioxaborolan-2-yl in the presence of a transition metal catalyst such as [1,1'-bis(diphenylphosphino)ferrocene]dichloropalladium(II) in the presence of a base such as caesium carbonate in a solvent such as dioxane at a temperature from ambient to the boiling point of the solvent under an inert atmosphere such as argon to yield the compound of formula (LXIV).

Compounds of general formula (Ic) may be prepared from compounds of formula (LXIV) by ester hydrolysis. The reaction may be carried out by treating a compound of formula (LXIV) with an acid such as trifluoroacetic acid or hydrochloric acid and in a solvent such as chloroform, dichloromethane or dioxane at a temperature from 0 ºC to the boiling point of the solvent to yield the compound of formula (Id). Alternatively, the reaction may be carried out by treating a compound of formula (LXIV) with a base such as lithium hydroxide in a solvent such as water, tetrahydrofuran or mixtures thereof, at a temperature from 0 ºC to the boiling point of the solvent to yield the compound of formula (Id).

In the particular case where Z is an imidazo[1,5-a]pyridyl group, compounds of the general formula (Ie) may be prepared following the synthetic scheme depicted in Scheme 12.

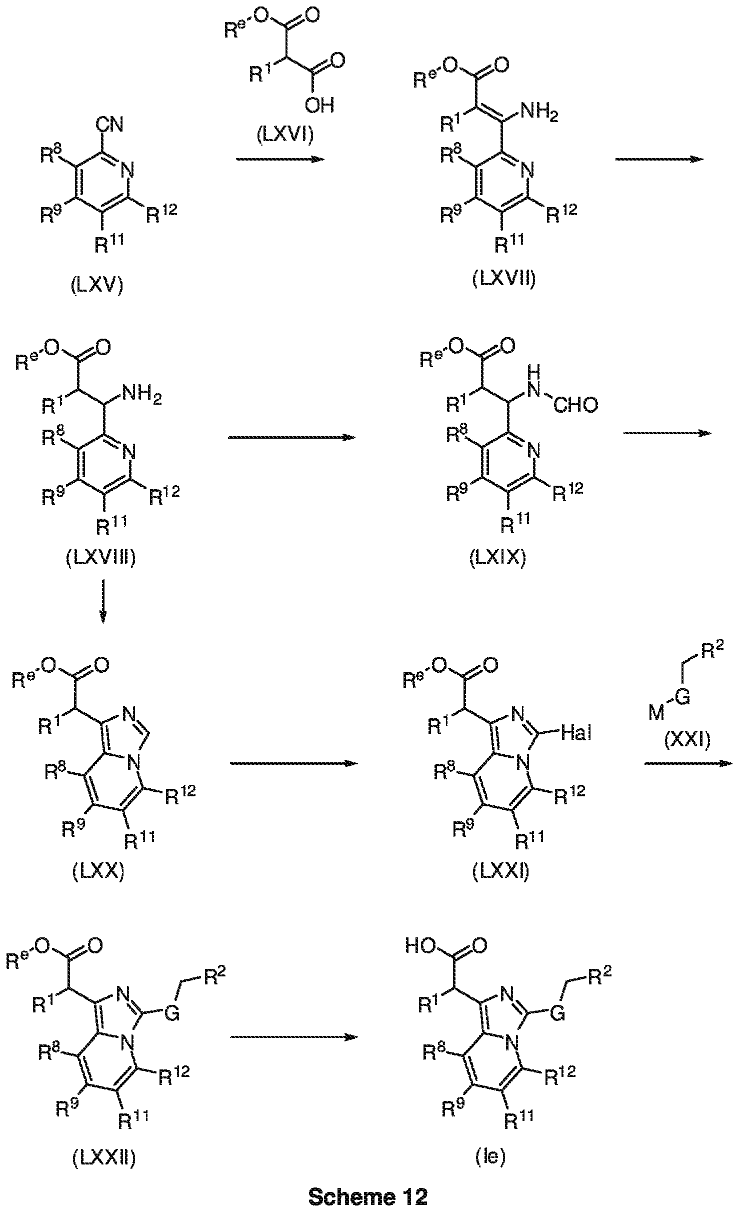

Compounds of formula (LXV) are prepared according to standard literature methods and specific examples are described in the experimental section.

Compounds of general formula (LXVII) may be prepared by treating a compound of formula (LXV) with a compound of Formula (LXVI) in the presence of a Lewis acid such as zinc chloride and a base such as diisopropylethylamine in a solvent such as dichloroethane at a temperature from ambient to the boiling point of the solvent.

Compounds of general formula (LXVIII) may be prepared from a compound of formula (LXVII) by reduction, treating a compound of formula (LXVII) under reducing conditions such as in the presence of a transition metal catalyst such as palladium hydroxide under an atmosphere of hydrogen in a solvent such as ethanol or acetic acid or mixtures thereof at a temperature from ambient to the boiling point of the solvent.

Compounds of general formula (LXIX) may be prepared by treating a compound of formula (LXVIII) with a formylating agent such as formic acid at a temperature from ambient to the boiling point of the solvent to yield the compound of formula (LXIX).

Compounds of general formula (LXX) may be prepared by treating a compound of formula (LXIX) with a dehydrating agent such as phosphoryl chloride in a solvent such as toluene at a temperature from ambient to the boiling point of the solvent.

Compounds of general formula (LXX) may also be prepared from a compound of formula (LXVIII) and a formylating agent. The reaction may be carried out by treating a compound of formula (LXVIII) with a formulating agent suchas as formic acid in the presence of a dehydrating agent such as propylphosphonic anhydride in a solvent such as n-butyl acetate at a temperature from ambient to the boiling point of the solvent to yield the compound of formula (LXX).

Compounds of general formula (LXXI) may be prepared from compounds of formula (LXX) by halogenation. The reaction is carried out by mixing a compound of formula (LXX) and a halogenating reagent such as N-bromosuccinimide, in a solvent such as dimethylformamide at a temperature from 0 ºC to 50 ºC to yield the compound of formula (LXXI).

Compounds of general formula (LXXII) may be prepared from compounds of formula (LXXI) with an organometallic reagent of formula (XXI) wherein M represents lithium or salts or complexes of magnesium, zinc, copper, tin or boron. The reaction is carried out by treating a compound of formula (LXXII) according to standard literature methods known to those skilled in the art of carbon-carbon cross-coupling reactions. For example, in one instance the reaction can be carried out by treating a compound of formula (LXXI) with a boronate ester of formula (XXI) wherein M is 4,4,5,5-tetramethyl-1,3,2-dioxaborolan-2-yl in the presence of a transition metal catalyst such as [1,1'-bis(diphenylphosphino)ferrocene]dichloropalladium(II) in the presence of a base such as caesium carbonate in a solvent such as dioxane at a temperature from ambient to the boiling point of the solvent under an inert atmosphere such as argon to yield the compound of formula (LXXII).

Compounds of general formula (Ie) may be prepared from compounds of formula (LXXII) by ester hydrolysis. The reaction may be carried out by treating a compound of formula (LXXII) with an acid such as trifluoroacetic acid or hydrochloric acid and in a solvent such as chloroform, dichloromethane or dioxane at a temperature from 0 ºC to the boiling point of the solvent to yield the compound of formula (Ie). Alternatively, the reaction may be carried out by treating a compound of formula (LXXII) with a base such as lithium hydroxide in a solvent such as water, tetrahydrofuran or mixtures thereof, at a temperature from 0 ºC to the boiling point of the solvent to yield the compound of formula (Ie).

In the particular case where Z is pyrrolo[1,2-a]pyrazinyl group, compounds of the general formula (If) may be prepared following the synthetic scheme depicted in Scheme 13.

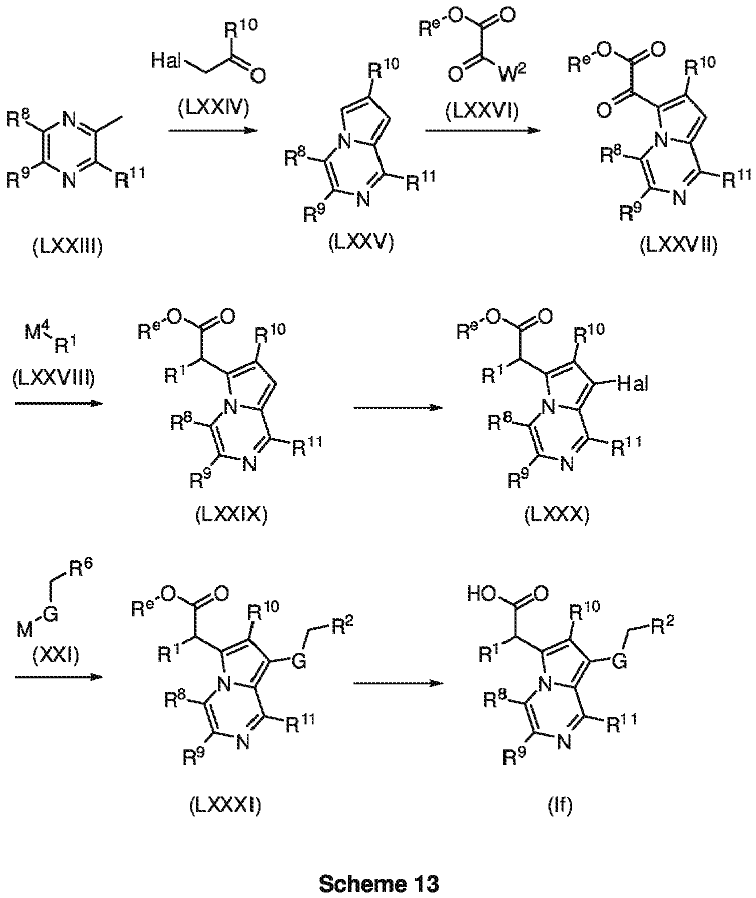

Compounds of formula (LXXIII) are prepared according to standard literature methods and specific examples are described in the experimental section.

Compounds of general formula (LXXV) may be prepared by treating a compound of formula (LXXIII) with a compound of formula (LXXIV) in a solvent such as acetonitrile at a temperature from ambient to the boiling point of the solvent.

Compounds of general formula (LXXVII) may be prepared from a compound of formula (LXXV) and a compound of formula (LXXVI) wherein W² represents a halogen atom such as chlorine, bromine or iodine. The reaction may be carried out by treating a compound of formula (LXXV) with a compound of formula (LXXVI) in the presence of a Lewis acid such as aluminium trichloride in a solvent such as chlorobenzene at a temperature from 0 ºC to the boiling point of the solvent to yield the compound of formula (LXXVII)

Compounds of general formula (LXXIX) may be prepared from a compound of formula (LXXVII) and a compound of formula (LXXVIII) wherein M⁴ represents lithium or salts or complexes of magnesium, zinc, or silicon under reducing conditions. For example, in one instance the reaction can be carried out by treating a compound of formula (LXXVII) under reducing conditions such as trimethylsilane in trifluoroacetic acid at a temperature from ambient to the boiling point of the solvent under an inert atmosphere such as argon to yield the compound of formula (LXXIX).

Compounds of general formula (LXXX) may be prepared by mixing a compound of formula (LXXIX) and a halogenating reagent such as N-bromosuccinimide, in a solvent such as dimethylformamide at a temperature from 0 ºC to 50 ºC to yield the compound of formula (LXXX).

Compounds of general formula (LXXXI) may be prepared from compounds of formula (LXXX) with an organometallic reagent of formula (XXI) wherein M represents lithium or salts or complexes of magnesium, zinc, copper, tin or boron. The reaction is carried out by treating a compound of formula (LXXX) according to standard literature methods known to those skilled in the art of carbon-carbon cross-coupling reactions. For example, in one instance the reaction can be carried out by treating a compound of formula (LXXX) with a boronate ester of formula (XXI) wherein M is 4,4,5,5-tetramethyl-1,3,2-dioxaborolan-2-yl in the presence of a transition metal catalyst such as [1,1'-bis(diphenylphosphino)ferrocene]dichloropalladium(II) in the presence of a base such as caesium carbonate in a solvent such as dioxane at a temperature from ambient to the boiling point of the solvent under an inert atmosphere such as argon to yield the compound of formula (LXXXI).

Compounds of general formula (If) may be prepared from compounds of formula (LXXXI) by ester hydrolysis. The reaction may be carried out by treating a compound of formula (LXXXI) with an acid such as trifluoroacetic acid or hydrochloric acid and in a solvent such as chloroform, dichloromethane or dioxane at a temperature from 0 ºC to the boiling point of the solvent to yield the compound of formula (If). Alternatively, the reaction may be carried out by treating a compound of formula (LXXXI) with a base such as lithium hydroxide in a solvent such as water, tetrahydrofuran or mixtures thereof, at a temperature from 0 ºC to the boiling point of the solvent to yield the compound of formula (If).

In the particular case where Z is a pyrrolo[1,2-c]pyrimidinyl group, compounds of the general formula (Ig) may be prepared following the synthetic scheme depicted in Scheme 14.

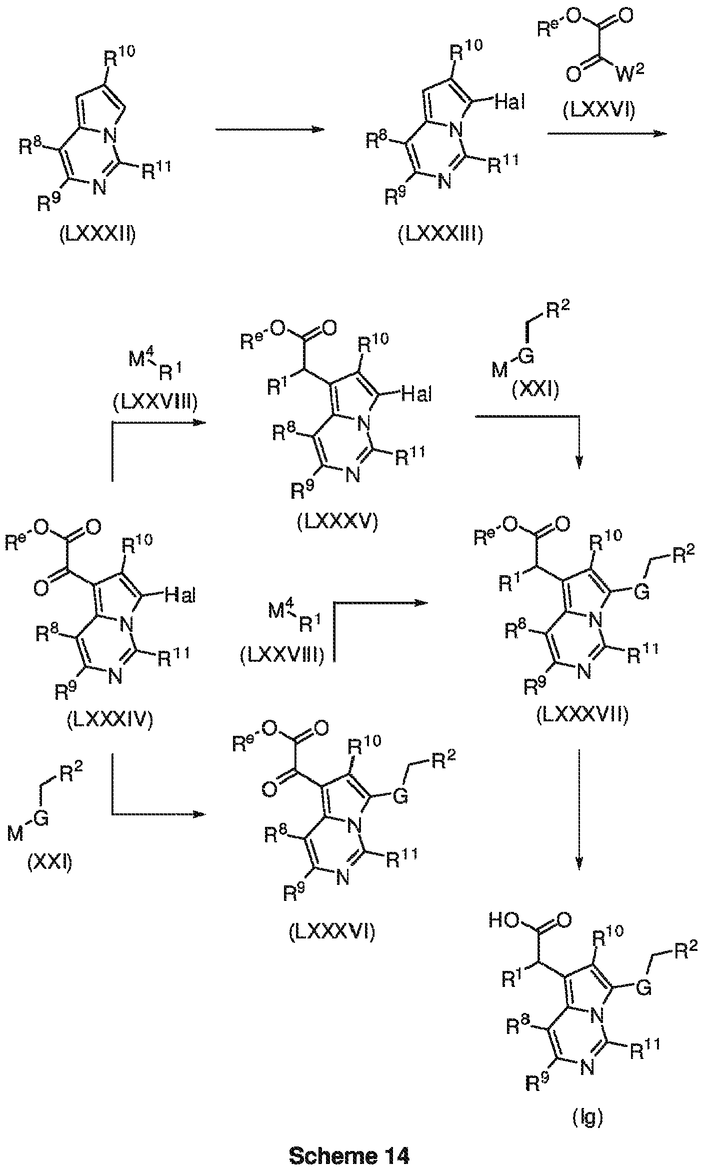

Compounds of formula (LXXXII) are prepared according to standard literature methods and specific examples are described in the experimental section.

Compounds of general formula (LXXXIII) may be prepared by mixing a compound of formula (LXXXII) and a halogenating reagent such as N-bromosuccinimide, in a solvent such as dichloromethane at a temperature from 0 ºC to 50 ºC to yield the compound of formula (LXXXIII).

Compounds of general formula (LXXXIV) may be prepared from a compound of formula (LXXXIII) and a compound of formula (LXXVI) wherein W² represents a halogen atom such as chlorine, bromine or iodine. The reaction may be carried out by treating a compound of formula (LXXXIII) with a compound of formula (LXXVI) in the presence of a Lewis acid such as aluminium trichloride in a solvent such as chlorobenzene at a temperature from 0 ºC to the boiling point of the solvent to yield the compound of formula (LXXXIV).

Compounds of general formula (LXXXV) may be prepared from a compound of formula (LXXXIV) and a compound of formula (LXXVIII) wherein M⁴ represents lithium or salts or complexes of magnesium, zinc, or silicon under reducing conditions.

For example, in one instance the reaction can be carried out by treating a compound of formula (LXXXIV) under reducing conditions such as trimethylsilane in trifluoroacetic acid at a temperature from ambient to the boiling point of the solvent under an inert atmosphere such as argon to yield the compound of formula (LXXVIII).

Compounds of general formula (LXXXVI) may be prepared from compounds of formula (LXXXIV) with an organometallic reagent of formula (XXI) wherein M represents lithium or salts or complexes of magnesium, zinc, copper, tin or boron. The reaction is carried out by treating a compound of formula (LXXXIV) according to standard literature methods known to those skilled in the art of carbon-carbon cross-coupling reactions. For example, in one instance the reaction can be carried out by treating a compound of formula (LXXXIV) with a boronate ester of formula (XXI) wherein M is 4,4,5,5-tetramethyl-1,3,2-dioxaborolan-2-yl in the presence of a transition metal catalyst such as bis(dibenzylideneacetone)palladium in the presence of a ligand such as 2-dicyclohexylphosphino-2',6'-dimethoxybiphenyl (S-Phos) a base such as potassium phosphate in a solvent such as dimethylformamide at a temperature from ambient to the boiling point of the solvent under an inert atmosphere such as argon to yield the compound of formula (LXXXVI).

Compounds of general formula (LXXXVII) may be prepared from compounds of formula (LXXXV) with an organometallic reagent of formula (XXI) wherein M represents lithium or salts or complexes of magnesium, zinc, copper, tin or boron. The reaction is carried out by treating a compound of formula (LXXXV) according to standard literature methods known to those skilled in the art of carbon-carbon cross-coupling reactions. For example, in one instance the reaction can be carried out by treating a compound of formula (LXXXV) with a boronate ester of formula (XXI) wherein M is 4,4,5,5-tetramethyl-1,3,2-dioxaborolan-2-yl in the presence of a transition metal catalyst such as [1,1'-bis(diphenylphosphino)ferrocene]dichloropalladium(II) in the presence of a base such as caesium carbonate in a solvent such as dioxane at a temperature from ambient to the boiling point of the solvent under an inert atmosphere such as argon to yield the compound of formula (LXXXVII).

Compounds of general formula (LXXXVII) may also be prepared from a compound of formula (LXXXVI) and a compound of formula (LXXVIII) wherein M⁴ represents lithium or salts or complexes of magnesium, zinc, or silicon under reducing conditions.

For example, in one instance the reaction can be carried out by treating a compound of formula (LXXXVI) under reducing conditions such as trimethylsilane in trifluoroacetic acid at a temperature from ambient to the boiling point of the solvent under an inert atmosphere such as argon to yield the compound of formula (LXXXVII).

Compounds of general formula (Ig) may be prepared from compounds of formula (LXXXVII) by ester hydrolysis. The reaction may be carried out by treating a compound of formula (LXXXVII) with an acid such as trifluoroacetic acid or hydrochloric acid and in a solvent such as chloroform, dichloromethane or dioxane at a temperature from 0°C to the boiling point of the solvent to yield the compound of formula (Ig). Alternatively, the reaction may be carried out by treating a compound of formula (LXXXVII) with a base such as lithium hydroxide in a solvent such as water, tetrahydrofuran or mixtures thereof, at a temperature from 0 ºC to the boiling point of the solvent to yield the compound of formula (Ig).

In the particular case wherein R² represents a -N(R³)COR⁴ group or a -N(R³)SO₂R⁴ group, compounds of the general formula (XC) may be prepared following the synthetic scheme depicted in Scheme 15.

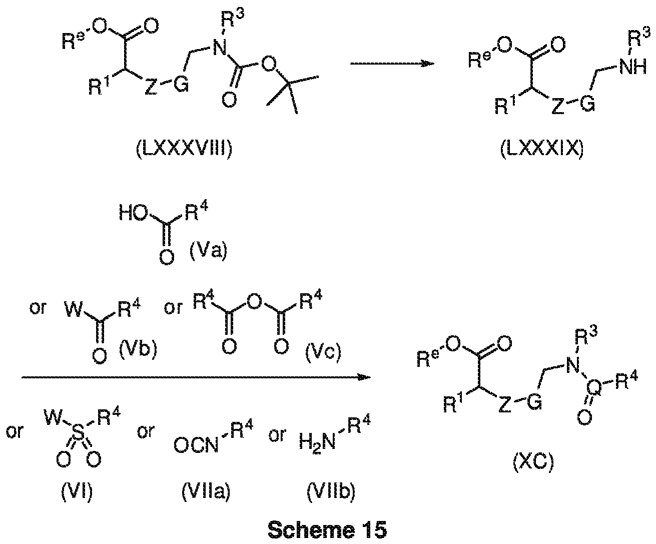

Compounds of general formula (LXXXIX) may be prepared from compounds of formula (LXXXVIII) by amine deprotection. The reaction is carried out by treating a compound of formula (LXXXVIII) with an acid such as trifluoroacetic acid in a solvent such as dichloromethane at a temperature from 0 ºC to the boiling point of the solvent to yield the compound of formula (LXXXIX).

Compounds of general formula (XC) wherein Q represents a carbon or a S=O group may be prepared from compounds of formula (LXXXIX) and an appropriate acylating or sulfonylating agent such as:
- a carboxylic acid of formula (Va) in the presence of a peptide coupling reagent such as dicyclohexylcarbodiimide.
- an acyl halide of formula (Vb) wherein W represents a halogen atom such as fluorine, chloride or bromine,
- an anhydride such of formula (Vc),
- a sulfonyl halide of formula (VI) wherein W is hereinbefore defined,
- an isocyanate of formula (VIIa) or
- an amine of formula (VIIb) in the presence of a phosgene equivalent such as phosgene, triphosgene or carbonyl diimidazole.

The reaction is carried out by mixing a compound of formula (LXXXIX) and a compound of formula (Va) - (VIIb) according to standard literature methods known to those skilled in the art of formation of amide, carbamates, ureas and sulfonamides to yield the compound of formula (XC).

### PREPARATION EXAMPLES

The syntheses of the compounds of the invention are illustrated by the following Examples (1 to 27) including Preparation Examples (1 to 118) which do not limit the scope of the invention in any way.

### General

Reagents, starting materials, and solvents were purchased from commercial suppliers and used as received. Concentration or evaporation refer to evaporation under vacuum using a Büchi rotatory evaporator.

Reaction products were purified, when necessary, by flash chromatography on silica gel (40-63 µm) with the solvent system indicated. Purifications in reverse phase were made in a Biotage Isolera® automated purification system equipped with a C18 column and using a gradient, unless otherwise stated, of water-acetonitrile/MeOH (1:1) (0.1% v/v ammonium formate both phases) from 0% to 100% acetonitrile/MeOH (1:1 in 40 column volumes. The conditions "formic acid buffer" refer to the use of 0.1% v/v formic acid in both phases. The appropriate fractions were collected and the solvents evaporated under reduced pressure and/or liofilized.

Purifications in reverse phase were also made in a Biotage SP1® automated purification system equipped with a C18 column and using a gradient of, unless otherwise stated, water-acetonitrile/MeOH (1:1) (0.1% v/v ammonium formate both phases) from 0% to 100% acetonitrile/MeOH (1:1) in 80 column volumes. The conditions "formic acid buffer" refer to the use of 0.1% v/v formic acid in both phases. The appropriate fractions were collected and freeze dried.

Gas chromatography was performed using a Thermo Trace Ultra gas chromatograph, coupled to a DSQ mass detector. Injections were performed on a split/splitless injector and a HP-1MS was the capillary column. Mass spectra were obtained by electron impact ionisation at 70eV.

Preparative HPLC-MS were performed on a Waters instrument equipped with a 2767 injector/collector, a 2525 binary gradient pump, a 2996 PDA detector, a 515 pump as a make-up pump and a ZQ4000 Mass spectrometer detector.

The chromatographic separations were obtained using a Waters 2795 system equipped with a Symmetry C18 (2.1 x 50 mm, 3.5 µM) column for methods A, B and C and a Symmetry C18 (2.1 x 100 mm, 3.5 µM) for method D. The mobile phases were (B): formic acid (0.4 ml), ammonia (0.1 ml), methanol (500 ml) and acetonitrile (500 ml) and (A): formic acid (0.5 ml), ammonia (0.125 ml) and water (1000 ml) (A), the gradients are specified in the following table for each method used.

| Method | Run time | 0% B | 0 to 95% B | 95% B |
|---|---|---|---|---|
| A | 5 min | 0.2 min | 3 min | 0.8 min |
| B | 9 min | 0.5 min | 6.5 min | 1 min |
| C | 15 min | 0 min | 10.5 min | 1.5 min |
| D | 30 min | 0 min | 20 min | 4 min |

The flow rate was 0.8 ml/min for method A and 0.4 ml/min for method B, C and D. The injection volume was 5 microliter. A Waters 2996 diode array was used as a UV detector. Chromatograms were processed at 210 nM or 254 nM. Mass spectra of the chromatograms were acquired using positive and negative electrospray ionization in a Micromass ZMD or in a Waters ZQ detectors coupled to the HPLC.

The UPLC chromatographic separations were obtained using a Waters Acquity UPLC system coupled to a SQD mass spectrometer detector. The system was equipped with an ACQUITY UPLC BEH C-18 (2.1x50mm, 1.7 mm) column. The mobile phase was formic acid (0.4 ml), ammonia (0.1 ml), methanol (500 ml) and acetonitrile (500 ml) (B) and formic acid (0.5 ml), ammonia (0.125 ml) and water (1000 ml) (A). A gradient between 0 to 95% of B was used. The run time was 3 or 5 minutes. The injection volume was 0.5 microliter. Chromatograms were processed at 210 nM or 254 nM. Mass spectra of the chromatograms were acquired using positive and negative electrospray ionization.

¹H Nuclear Magnetic Resonance Spectra were recorded on a Varian Gemini-2000 spectrometer operating at a frequency of 300MHz for the ¹H spectra or in a Varian Mercury plus operating at a frequency of 400MHz for the ¹H spectra. Samples were dissolved in the specified deuterated solvent. Tetramethylsilane was used as references. The ¹H NMR spectrum for the following compounds was not recorded: Preparations 10, 23, 29, 30, 31, 38, 47, 48, 53, 67, 70, 71, 97, 108, 109, 110, 115 and 117.

Mass Spectra (*mlz*) were recorded on a Micromass ZMD or in a Waters ZQ mass spectrometer using ESI ionization. The mass spectrum for the following compounds was not recorded: Preparations 41 and 81

Due to the presence of rotameric amides in the ¹H NMR spectra of many of the Preparations and Examples, in many cases only representative NMR peaks are quoted.

### PREPARATIONS

### PREPARATION 1

### Methyl 2-{[(4-mehylphenyl)sulfonyl]oxy}propanoate

Methyl-2-hydroxypropanoate (1.0 g, 9.6 mmol) was dissolved in 12 ml acetonitrile. The mixture was cooled in an ice-bath and triethylamine (1.3 ml, 9.6 mmol) and trimethylamine hydrochloride (100 mg, 1.05 mmol) were added. A solution of *p-*toluensulfonic acid (1.83 g, 9.60 mmol) in 4 ml acetonitrile was added dropwise. The mixture was stirred at 0 ºC for 1 h forming a precipitate. The solid was removed by filtration, washed with acetonitrile and the combined filtrate was evaporated under reduced pressure. The residue was dissolved in ether, washed sequentially with water, saturated sodium bicarbonate solution and brine, dried over sodium sulphate, filtered and evaporated under reduced pressure to give 2.0 g of the crude title compound as a colourless oil. Used as such without further purification. Purity 60%.

¹H NMR (300 MHz, CHLOROFORM-d) δ ppm 7.82 (d, *J*=8.24 Hz, 2 H), 7.35 (d, *J*=7.97 Hz, 2 H), 4.95 (q, *J*=6.96 Hz, 1 H), 3.67 (s, 3 H), 2.45 (s, 3 H), 1.51 (d, *J*=7.14 Hz, 3 H).

UPLC/MS (3 min) retention time 1.56 min.

LRMS: m/z 259 (M+1).

### PREPARATION 2

**N-[2-Bromo-5-(trifluoromethyl)benzyl]ethanamme**

2-Bromo-5-(trifluoromethyl)benzaldehyde (20.0 g, 79.0 mmol) was dissolved in 300 ml methanol. Ethylamine (2.0 M solution in tetrahydrofuran, 60 ml, 120 mmol) was added followed by the portion-wise addition of sodium cyanoborohydride (7.50 g, 120 mmol). Acetic acid (8.0 ml, 140 mmol) was added drop-wise and the reaction mixture was stirred overnight at room temperature. The organics were evaporated under reduced pressure and the mixture was partitioned between dichloromethane and water. The aqueous phase was extracted twice with dichloromethane and the combined organics were washed with 4% w/v sodium bicarbonate solution, dried over sodium sulphate, filtered and evaporated under reduced pressure to give 15.0 g (53.2 mmol, 67%) of the title compound as an oil. Purity 100%.

¹H NMR (300 MHz, CHLOROFORM-*d*) δ ppm 7.70 (1 H, s), 7.66 (1 H, d, *J*=8.2 Hz), 7.37 (1 H, dd, *J*=8.2, 1.4 Hz), 3.91 (2 H, s), 2.71 (2 H, q, *J*=7.1 Hz), 1.68 (1 H, br. s.), 1.17 (3 H, t, *J*=7.1 Hz).

UPLC/MS (3 min) retention time 0.95 min.

LRMS: m/z 282, 284 (M+1).

### PREPARATION 3

### N-[2-Bromo-5-(trifluoromethyl)benzyl]-N-ethylcyclopropanecarboxamide

The title compound of Preparation 2 (6.0 g, 18.1 mmol) was dissolved in 50 ml dichloromethane and Triethylamine (3.0 ml, 21.5 mmol). Cyclopropanecarbonyl chloride (2.30 ml, 25.3 mmol) was added drop-wise over 15 min and the mixture was stirred at room temperature for 2 h. The mixture was diluted with dichloromethane and was washed sequentially with 4% w/v sodium bicarbonate solution, water and brine. The organics were dried over sodium sulphate, filtered and evaporated under reduced pressure.The residue was purified using the Isolera Purification System (ethyl acetate-hexane gradient, 0:100 rising to 30:70) to give 5.50 g (15.7 mmol, 87%) of the title compound as a colourless oil. Purity 100%.

¹H NMR (300 MHz, CHLOROFORM-d) 60:40 mixture of two rotamers. Major rotamer δ ppm 7.57 - 7.80 (1 H, m), 7.30 - 7.54 (2 H, m), 4.71 (2 H, s), 3.42 - 3.63 (2 H, m), 1.76 - 1.96 (1 H, m), 1.28 (3 H, t, *J*=7.1 Hz), 0.98 - 1.11 (2 H, m), 0.79 - 0.92 (2 H, m).

Minor rotamer δ ppm 7.57 - 7.80 (1 H, m), 7.30 - 7.54 (2 H, m), 4.74 (2 H, s), 3.42 - 3.63 (2 H, m), 1.36 - 1.48 (1 H, m), 1.16 (3 H, t, *J*=7.0 Hz), 0.98 - 1.11 (2 H, m), 0.63 - 0.76 (2 H, m).

UPLC/MS (3 min) retention time 1.90 min.

LRMS: m/z 350, 352 (M+1).

### PREPARATION 4

### N-Ethyl-N-[2-(4,4,5,5-tetramethyl-1,3,2-dioxaborolan-2-yl)-5-(trifluoromethyl)benzyl]cyclopropanecarboxamide

The title compound of Preparation 3 (3.76 g, 10.7 mmol) and 4,4,4',4',5,5,5',5'-octamethyl-2,2'-bis(1,3,2-dioxaborolane) (2.54 g, 14.0 mmol) were dissolved in 15 ml dioxane in a Schlenk vessel. Potassium acetate (3.16 g, 32.2 mmol) was added and the reaction mixture was submitted to three vacuum-argon cycles. [1,1'-bis(diphenylphosphino)ferrocene]dichloropalladium(II), complex with dichloromethane (0.47 g, 0.54 mmol) was added and the mixture was further submitted to three vacuum-argon cycles. The reaction vessel was sealed and the mixture was stirred at 100 ºC for 3 h. The mixture was cooled to room temperature, diluted with ethyl acetate and filtered through Celite. The filtrate was evaporated under reduced pressure and the residue purified using the Isolera Purification System (ether-hexane gradient, 0:100 rising to 100:0) to give 3.20 g (8.06 mmol, 75%) of the title compound as a colourless oil. Purity 90%.

Partial ¹H NMR (300 MHz, CHLOROFORM-d) 55:45 mixture of two rotamers.

Major rotamer δ ppm 4.99 (s, 2 H).

Minor rotamer δ ppm 5.02 (s, 2 H).

UPLC/MS (3 min) retention time 2.19 min.

LRMS: m/z 398 (M+1).

### PREPARATION 5

### N-(2-Bromo-5-fluorobenzyl)ethanamine

2-Bromo-5-fluorobenzaldehyde (4.88 g, 24.0 mmol) was treated with ethylamine (2M in tetrahydrofuran, 18 ml, 36 mmol), sodium cyanoborohydride (2.27 g, 36.1 mmol) and acetic acid (2.06 ml, 36.0 mmol) according to the method of Preparation 2 to give 4.00 g (17.2 mmol, 72%) of the title compound as a colourless oil. Purity 100%.

¹H NMR (300 MHz, CHLOROFORM-*d*) δ ppm 7.49 (dd, *J*=8.65, 5.36 Hz, 1 H), 7.19 (dd, *J*=9.34, 3.02 Hz, 1 H), 6.86 (td, *J*=8.24, 3.02 Hz, 1 H), 3.85 (s, 2 H), 2.70 (q, *J*=7.14 Hz, 2 H), 1.17 (t, *J*=7.14 Hz, 3 H).

UPLC/MS (3 min) retention time 0.69 min.

LRMS: m/z 232, 234 (M+1).

### PREPARATION 6

### N-(2-Bromo-5-fluorobenzyl)-N-ethylcyclopropanecarboxamide

The title compound of Preparation 5 (3.90 g, 16.8 mmol) was treated with triethylamine (2.8 ml, 20 mmol) and cyclopropanecarbonyl chloride (2.20 ml, 24.2 mmol) according to the method of Preparation 3. The resulting residue was purified using the Isolera Purification System (ether-hexane gradient, 0:100 rising to 30:70) to give 2.80 g (9.33 mmol, 55%) of the title compound as a colourless oil. Purity 90%.

Partial ¹H NMR (300 MHz, CHLOROFORM-d) 65:35 mixture of two rotamers.

Major rotamer δ ppm 4.66 (s, 2 H).

Minor rotamer δ ppm 4.67 (s, 2 H).

UPLC/MS (3 min) retention time 1.78 min.

LRMS: m/z 300, 302 (M+1).

### PREPARATION 7

### N-Ethyl-N-[5-fluoro-2-(4,4,5,5-tetramethyl-1,3,2-dioxaborolan-2-yl)benzyl]cyclopropanecarboxamide

The title compound of Preparation 6 (1.50 g, 5.00 mmol) was treated with 4,4,4',4',5,5,5',5'-octamethyl-2,2'-bi(1 3,2-dioxaborolane) (1.65 g, 6.50 mmol), potassium acetate (1.47 g, 15.0 mmol) and [1,1'-bis(diphenylphosphino)ferrocene]-dichloropalladium(II), complex with dichloromethane (0.20 g, 0.24 mmol) according to the method of Preparation 4. The resulting residue was purified using the Isolera Purification System (ether-hexane gradient, 0:100 rising to 0:100) to give 1.2 g (3.46 mmol, 69%) of the title compound as a colourless oil.

¹H NMR (300 MHz, CHLOROFORM-d) δ ppm 8.06 - 8.48 (m, 1 H), 7.58 - 7.78 (m, 1 H), 7.33 - 7.51 (m, 1 H), 5.26 - 5.56 (m, 2 H), 3.69 - 4.07 (m, 2 H), 2.38 - 2.59 (m, 1 H), 1.72 - 1.90 (m, 12 H), 1.36 - 1.74 (m, 5 H), 1.01 - 1.36 (m, 2 H).

UPLC/MS (3 min) retention time 2.08 min.

LRMS: m/z 348 (M+1).

### PREPARATION 8

### N-Benzyl-[2-bromo-5-(trifluoromethyl)benzyl]-N-ethylurea

The title compound of Preparation 2 (0.5 g, 1.77 mmol) was dissolved in 7 ml dichloromethane and the mixture was cooled in an ice bath. Diisopropylethylamine (0.68 ml, 3.9 mmol) and phosgene (20% solution in toluene, 1.13 ml, 2.1 mmol) were added and the mixture was stirred for 2 h in the ice-bath. Benzylamine (0.29 ml, 2.66 mmol) was added and the mixture was stirred for 15 min. Triethylamine (0.35 ml, 2.5 mmol) was added and the mixture was stirred for 90 min at room temperature. The mixture was partitioned between dichloromethane and water. The aqueous phase was extracted twice with dichloromethane and the combined organics were washed with brine, dried over sodium sulphate, filtered and evaporated under reduced pressure. The residue was purified using the Isolera Purification System (ethyl acetate-hexane gradient, 0:100 rising to 50:50) to give 0.64 g (1.54 mmol, 87%) of the title compound. Purity 100%.

¹H NMR (400 MHz, CHLOROFORM-d) δ 7.67 (d, J = 8.1 Hz, 1H), 7.50 (s, 1H), 7.39 (d, J = 8.3 Hz, 1H), 7.36 - 7.30 (m, 2H), 7.28 (m, 3H), 4.73 (s, 1H), 4.60 (s, 2H), 4.48 (d, J = 5.4 Hz, 2H), 3.34 (q, J = 7.1 Hz, 2H), 1.19 (t, J = 7.1 Hz, 3H).

UPLC/MS (3 min) retention time 1.95 min.

LRMS: m/z 415,417 (M+1).

### PREPARATION 9

### N'-Benzyl-N-ethyl-N-[2-(4,4,5,5-tetramethyl-1,3,2-diexaborolan-2-yl)-5-(trifluoromethyl)benzyl]urea

The title compound of Preparation 8 (400 mg, 0.96 mmol) was treated with 4,4,4',4',5,5,5',5'-octamethyl-2,2'-bis(1,3,2-dioxaborolane) (490 mg, 1.9 mmol), potassium acetate (280 mg, 2.9 mmol) and [1,1'-bis(diphenylphosphino)ferrocene]-dichloropalladium(II), complex with dichloromethane (43 mg, 0.05 mmol) according to the method of Preparation 5. The resulting residue was purified using the Isolera Purification System (ether-hexane gradient, 0:100 rising to 100:0) to give 204 mg (0.44 mmol, 46%) of the title compound as a colourless oil. Purity 100%.

¹H NMR (400 MHz, CHLOROFORM-d) δ 7.91 (d, J = 7.6 Hz, 1H), 7.60 (s, 1H), 7.49 (d, J = 6.7 Hz, 1H), 7.33 - 7.26 (m, 3H), 7.24 - 7.15 (m, 2H), 4.81 (s, 2H), 4.45 (d, J = 5.6 Hz, 2H), 3.40 (d, J = 7.1 Hz, 2H), 1.27 (s, 4H), 1.24 (s, 8H), 1.18 (t, J = 7.1 Hz, 3H).

UPLC/MS (3 min) retention time 2.16 min.

LRMS: m/z 463 (M+1).

### PREPARATION 10

### N-(2-Bromobenzyl)ethanamine

2-Bromobenzaldehyde (2.00 g, 10.8 mmol) was treated with ethylamine (2M in tetrahydrofuran, 9.6 ml, 19.2 mmol), sodium cyanoborohydride (1.21 g, 19.2 mmol) and acetic acid (1.10 ml, 19.2 mmol) according to the method of Preparation 2 to give 2.13 g of the crude title compound. Used as such without further purification. Purity 51%.

UPLC/MS (3 min) retention time 0.72 min.

LRMS: m/z 214, 216 (M+1).

### PREPARATION 11

### N-(2-Bromobenzyl)-N-ethylcyclopropanecarboxamide

The crude title compound of Preparation 10 (2.13 g) was treated with triethylamine (2.08 ml, 14.9 mmol) and cyclopropanecarbonyl chloride (1.35 ml, 14.9 mmol) according to the method of Preparation 003. The resulting residue was purified using the Isolera Purification System (ether-hexane gradient, 0:100 rising to 60:40) to give 1.34 g (4.75 mmol, 44% over two steps) of the title compound as a colourless oil. Purity 97%.

¹H NMR (300 MHz, CHLOROFORM-*d*) δ ppm 1.77 - 1.90 (m, 1 H), 1.41 - 1.53 (m, 1 H), 1.25 (t, *J*=7.04 Hz, 3 H), 1.15 (t, *J*=7.04 Hz, 3 H), 0.96 1.11 (m, 4 H), 0.78 - 0.87 (m, 2 H), 0.64 - 0.74 (m, 2 H).

UPLC/MS (3 min) retention time 1.80 min.

LRMS: m/z 282, 284 (M+1).

### PREPARATION 12

### N-Ethyl-N-[2-(4,4,5,5-tetramethyl-1,3,2-dioxaborolan-2-yl)benzyl]cyclopropanecarboxamide

N-(2-(4,4,5,5-Tetramethyl-1,3,2-dioxaborolan-2-yl)benzyl)ethanamine (70 mg, 0.27 mmol) and triethylamine (44 µl, 0.32 mmol) were dissolved in 2 ml dichloromethane and the mixture was cooled in an ice-bath. A solution of cyclopropanecarbonyl chloride (29 µl, 0.32 mmol) dissolved in 1 ml dichloromethane was added drop-wise and the mixture was stirred at room temperature for 1 h. The mixture was washed with water and the organics were dried over sodium sulphate, filtered and evaporated under reduced pressure to give 82 mg (0.25 mmol, 91%) of the title compound as a colourless oil. Purity 100%.

Partial ¹H NMR (300 MHz, CHLOROFORM-d) 55:45 mixture of two roamers.

Major rotamer δ ppm 5.05 (s, 2 H), 1.19 (3 H, t, *J*=7.0 Hz).

Minor rotamer δ ppm 5.02 (s, 2 H), 1.27 (3 H, t, *J*=7.1 Hz).

UPLC/MS (3 min) retention time 2.05 min.

LRMS: m/z 330 (M+1).

### PREPARATION 13

### N-Benzyl-1-[2-bromo-5-(trifluoromethyl)phenyl]methanamine

2-Bromo-5-(trifluoromethyl)benzaldehyde (3.00 g, 11.9 mmol) was treated with benzylamine (1.94 ml, 17.8 mmol), sodium cyanoborohydride (1.12 g, 17.8 mmol) and acetic acid (1.02 ml, 17.8 mmol) according to the method of Preparation 2 to give 1.87 g (5.43 mmol, 46%) of the title compound as an oil. Purity 98%.

¹H NMR (300 MHz, CHLOROFORM-*d*) δ ppm 7.73 (d, *J*=1.76 Hz, 1 H), 7.66 (d, *J*=8.22 Hz, 1 H), 7.33 - 7.39 (m, 5 H), 7.26 - 7.32 (m, 1 H), 3.92 (s, 2 H), 3.83 (s, 2 H).

UPLC/MS (3 min) retention time 1.20 min.

LRMS: m/z 344, 346 (M+1).

### PREPARATION 14

### N-Benzyl-N-[2-bromo-5-(trifluoromethyl)benzyl]cyclopropanecarboxamide

The title compound of Preparation 13 (0.51 g, 1.48 mmol) was treated with triethylamine (0.24 ml, 1.72 mmol) and cyclopropanecarbonyl chloride (0.19 ml, 2.04 mmol) according to the method of Preparation 3 to give 0.37 g (0.90 mmol, 61%) of the title compound as light yellow oil. Purity 97%.

Partial ¹H NMR (300 MHz, CHLOROFORM-*d*) 57:43 mixture of two roamers.

Major rotamer δ ppm 7.70 (1 H, d, *J*=7.6 Hz), 4.67 (s, 2 H).

Minor rotamer δ ppm 7.64 (1 H, d, *J*=8.2 Hz), 4.70 (s, 2 H).

UPLC/MS (3 min) retention time 2.08 min.

LRMS: m/z 412, 414 (M+1).

### PREPARATION 15

### N-Benzy-N-[2-(4,4,5,5-tetramethyl-1,3,2-dioxaborolan-2-yl)-5-(trifluoromethyl)benzyl]cyclopropanecarboxamide

The title compound of Preparation 14 (0.37 g, 0.87 mmol) was treated with 4,4,4',4',5,5,5',5'-octamethyl-2,2'-bis(13,2-dioxaborolane) (0.30 g, 1.16 mmol), potassium acetate (0.26 g, 2.65 mmol) and [1,1'-bis(diphenylphosphino)ferrocene]-dichloropalladium(II), complex with dichloromethane (0.04 g, 0.05 mmol) according to the method of Preparation 4. The resulting residue was purified using the Isolera Purification System (ether-hexane gradient, 0:100 rising to 50:50) to give 165 mg of the crude title compound as a colourless oil. Purity 68%.

Partial ¹H NMR (300 MHz, CHLOROFORM-*d*) 50:50 mixture of two rotamers.

Rotamer 1 δ ppm 7.94 (d, J = 7.6 Hz, 1 H), 5.06 (s, 2 H).

Rotamer 2 δ ppm 7.85 (d, J = 7.6 Hz, 1 H), 5.00 (s, 2 H).

UPLC/MS (3 min) retention time 2.26 min.

LRMS: m/z 460 (M+1).

### PREPARATION 16

### Ethyl (3-bromo-1H-pyrrolo[3,2-b]pyridin-1-yl)acetate

Sodium hydride (60% dispersion in oil, 40 mg, 1.08 mmol) was suspended in 3 ml dimethylformamide under nitrogen atmosphere and was cooled in an ice-bath. 3-Bromo-1*H*-pyrrolo[3,2*b*]pyridine (200 mg, 1.02 mmol) was added anf the mixture was stirred for 45 min, warming to room temperature. Ethyl 2-bromoacetate (123 mg, 1.11 mmol) was slowly added and the mixture was stirred for stirred for 1.25 h. The mixture was partitioned between ethyl acetate and water. The aqueous phase was extracted twice with ethyl acetate and the combined organics were washed with brine, dried over anhydrous magnesium sulphate, filtered and evaporated under reduced pressure. The resulting residue was purified using the Isolera Purification System (ethyl acetate-hexane gradient, 0:100 rising to 80:20) to give 230 mg (0.81 mmol, 75%) of the title compound as a white solid. Purity 100%.

¹H NMR (300 MHz, CHLOROFORM-*d*) δ ppm 8.30 - 8.88 (m, 1 H), 7.54 - 7.66 (m, 1 H), 7.41 (d, *J*=2.7 Hz, 1 H), 7.14 - 7.26 (m, 1 H), 4.84 (s, 2 H), 4.24 (q, *J*=7.1 Hz, 2 H), 1.28 (t, *J*=7.1 Hz, 3 H).

UPLC/MS (3 min) retention time 0.98 min.

LRMS: m/z 283, 285 (M+1).

### PREPARATION 17

### 6-Chloro-2-iodopyridin-3-amine

6-Chloropyridin-3-amine (3.00 g, 23.3 mmol) was dissolved in 40 ml dimethylformamide. N-lodosuccinimide (5.25 g, 23.3 mmol) was added and the mixture was stirred at room temperature for 16 h. Further N-iodosuccinimide (1.79 g, 7.9 mmol) was added and stirring was continued for 4 h. The mixture was then partitioned between water and ether and the organic layer was washed with water, dried over magnesium sulphate, filtered and evaporated under reduced pressure. The residue was purified by flash chromatography (ethyl acetate-hexane, 20:80) to yield 4.03 g of the title compound (15.8 mmol, 68%) as a beige solid. Purity 95%.

¹H NMR (300 MHz, CHLOROFORM-*d*) δ ppm 7.06 (d, J = 8.3 Hz, 1H), 6.90 (d, J = 8.3 Hz, 1H), 4.14 (br s, 2H).

HPLC/MS (9 min) retention time 4.88 min.

LRMS: m/z 255 (M+1).

### PREPARATION 18

### 6-Chloro-2-[(E)-2-ethoxyvinyl]pyridin-3-amine

The title compound of Preparation 17 (3.01 g, 11.8 mmol) was dissolved in 45 ml tetrahydrofuran under an argon atmosphere. Powdered sodium hydroxide (1.42 g, 35.5 mmol), tetrakis(triphenylphosphine)palladium (0.41 g, 0.35 mmol) and tris[(E)-2-ethoxyvinyl]borane (0.15 M in tetrahydrofuran, 30.7 ml, 4.6 mmol) were added and the resulting mixture was stirred at 65 ºC for 24 h. The mixture was allowed to cool and was partitioned between water and ethyl acetate. The organic layer was washed with brine, dried over magnesium sulphate, filtered and evaporated under reduced pressure. The residue was purified by flash chromatography (ethyl acetate-hexane gradient, 15:85 rising to 25:75) to yield 0.87 g (4.39 mmol, g, 37%) of the title compound as a colourless oil. Purity 90%.

¹H NMR (300 MHz, CHLOROFORM-*d*) δ+ ppm 7.54 (d, J = 12.1 Hz, 1H), 6.88 (m, 2H), 5.77 (d, J = 12.1 Hz, 1H), 3.99 (q, J = 7.2 Hz, 2H), 3.56 (br s, 2H), 1.36 (t, J = 7.2 Hz, 3H).

HPLC/MS (9 min) retention time 5.32 min.

LRMS: m/z 199 (M+1).

### PREPARATION 19

### 5-Chloro-1H-pyrrolo[3,2-b]pyridine

The title compound of Preparation 18 (1.01 g, 5.1 mmol) was dissolved in 20 ml methanol. 1 ml Concentrated hydrochloric acid was added and the mixture was stirred at 75 ºC for 21 h. The mixture was evaporated under reduced pressure and the residue was partitioned between saturated potassium carbonate solution and ethyl acetate. The organic layer was dried over magnesium sulphate, filtered and evaporated under reduced pressure. The residue was purified by flash chromatography (ethyl acetate-hexane, 33:67) to yield 0.55 g (3.6 mmol, 71%) of the title compound as a pale yellow solid. Purity 95%.

¹H NMR (300 MHz, CHLOROFORM-*d* + DMSO-*d6*) δ ppm 9.85 (br. s, 1H), 7.67 (d, J = 8.5 Hz, 1H), 7.47 (m, 1H), 7.10 (d, J = 8.5 Hz,1H), 6.65 (m, 1H).

HPLC/MS (9 min) retention time 4.27 min.

LRMS: m/z 153 (M+1)

### PREPARATION 20

### 5-Methyl-1H-pyrrolo[3,2-b]pyridine

The title compound of Preparation 19 (200 mg, 1.31 mmol), trimethylboroxine (0.20 ml, 1.44 mmol) and potassium carbonate (540 mg, 3.93 mmol) were suspended in 5 ml 1,2-dimethoxyethane in a microwave reactor and the mixture was submitted to three vacuum-argon cycles. [1,1'-Bis(diphenylphosphino)ferrocene]dichloropalladium (II), complex with dichloromethane (53 mg, 0.07 mmol) was added and the resulting mixture was submitted to three further vacuum-argon cycles. The reaction was stirred at 120 ºC for 1h under microwave irradiation. The reaction was allowed to cool, diluted with ethyl acetate and water and filtered through Celite^{®} eluting with more ethyl acetate and water. The organic extract was washed with brine, dried over anhydrous magnesium sulphate, filtered and evaporated under reduced pressure. The resulting residue was purified using the Isolera Purification System (methanol-dichloromethane gradient, 0:100 rising to 5:95) to give 95 mg (0.72 mmol, 55%) of the title compound as an oil. Purity 96%.

¹H NMR (300 MHz, CHLOROFORM-*d*) δ ppm 8.44 (br. s., 1 H), 7.60 (d, *J*=8.24 Hz, 1 H), 7.41 (t, *J*=3.02 Hz, 1 H), 7.01 (d, *J*=8.24 Hz, 1 H), 6.69 (br. s., 1 H), 2.67 (s, 3 H).

UPLC/MS (3 min) retention time 0.45 min.

LRMS: m/z 133 (M+1).

### PREPARATION 21

### 3-Bromo-5-methyl-1H-pyrrolo[3,2-b]pyridine

The title compound of Preparation 20 (95 mg, 0.72 mmol) was dissolved in 7 ml acetonitrile. Copper(II) bromide (482 mg, 2.16 mmol) was added and the mixture was stirred at room temperature for 1 h. Ammonium hydroxide solution (7M in methanol , 5 ml) was added and the mixture was stirred at room temperature for 30 min. The mixture was partitioned between ethyl acetate and water. The organic phase was washed with brine, dried over anhydrous magnesium sulphate, filtered and evaporated under reduced pressure to give 147 mg (0.70 mmol, 97%) of the title compound as an oil. Purity 90%.

¹H NMR (300 MHz, CHLOROFORM-*d*) δ ppm 8.61 (br. s., 1 H), 7.59 (d, *J*=8.51 Hz, 1 H), 7.45 (d, *J*=2.75 Hz, 1 H), 7.06 (d, *J*=8.51 Hz, 1 H), 2.71 (s, 3H).

UPLC/MS (3 min) retention time 0.55 min.

LRMS: m/z 211, 213 (M+1).

### PREPARATION 22

### Ethyl (3-bromo-5-methyl-1H-pyrrolo[3,2-b]pyridin-1-yl)acetate

The title compound of Preparation 21 (125 mg, 0.59 mmol) was treated with sodium hydride (60% dispersion in oil, 25 mg, 0.65 mmol) and ethyl 2-bromoacetate (0.07 ml, 0.65 mmol) according to the method of Preparation 16. The resulting residue was purified using the Isolera Purification System (hexane-ethyl acetate gradient, 0:100 rising to 30:70) to give 100 mg (0.34 mmol, 57%) of the title compound as an oil. Purity 97%.

¹H NMR (300 MHz, CHLOROFORM-*d*) δ ppm 7.47 δ, *J*=8.51 Hz, 1 H), 7.32 (s, 1 H), 7.07 (d, *J*=8.51 Hz, 1 H), 4.79 (s, 2 H), 4.22 (q, *J*=7.05 Hz, 2 H), 2.71 (s, 3 H), 1.26 (t, *J*=7.14 Hz, 3 H).

UPLC/MS (3 min) retention time 0.88 min.

LRMS: m/z 297, 299 (M+1).

### PREPARATION 23

### Ethyl {3-[2-{[(cyclopropylcarbonyl)(ethyl)amino]methyl}-4-(trifluoromethyl)phenyl]-5-methyl-1H-pyrrolo[3,2-b]pyridin-1-yl}acetate

The title compound of Preparation 22 (100 mg, 0.34 mmol) and the title compound of Preparation 4 (147 mg, 0.37 mmol) were dissolved in 5 ml dioxane in a Schlenk vessel. Aqueous caesium carbonate solution (2M, 0.25 ml, 0.50 mmol) was added and the reaction mixture was submitted to three vacuum-argon cycles. [1,1'-Bis(diphenylphosphino)ferrocene]dichloropalladium(II) complex with dichloromethane (30 mg, 0.03 mmol) was added and the mixture was submitted to three further vacuum-argon cycles. The reaction vessel was sealed and the contents were stirred at 100 ºC overnight. The mixture was allowed to cool, was diluted with ethyl acetate and filtered through Celite^{®} eluting with more methyl acetate, and the filtrate was evaporated under reduced pressure. The resulting residue was purified using the Isolera Purification System (methanol-dichlorometane, 0:100 rising to 10:90) to give 110 mg of the crude title compound as an oil. Purity 40%.

UPLC/MS (3 min) retention time 1.66 min.

LRMS: m/z 488 (M+1).

### PREPARATION 24

### Methyl 2-(3-bromo-1H-pyrrolo[3,2-b]pyridin-1-yl)propanoate

3-Bromo-1*H*-pyrrolo[3,2*b*]pyridine (0.15 g, 0.76 mmol) was dissolved in 4 ml dimethylformamide. Caesium carbonate (0.49 g, 1.52 mmol) and crude title compound of Preparation 1 (0.29 g) were added and the mixture was stirred at room temperature for 2.5 h. The mixture was partitioned between ethyl acetate and water. The aqueous phase was extracted twice with ethyl acetate and the combined organics were washed with brine, dried over anhydrous magnesium sulphate, filtered and evaporated under reduced pressure. The residue was purified using the Isolera Purification System (ether-hexane gradient, 0:100 rising to 100: 0) to give 0.10 g (0.35 mmol, 46%) of the title compound as a colourless oil. Purity 90%.

¹H NMR (300 MHz, CHLOROFORM-*d*) δ ppm 8.56 (d, *J*=4.6 Hz, 1 H), 7.63 (d, *J*=8.5 Hz, 1 H), 7.55 (s, 1 H), 7.19 (dd, *J*=8.5, 4.6 Hz, 1 H), 5.11 (q, *J*=7.4 Hz, 1 H), 3.72 (s, 3 H), 1.84 (d, *J*=7.4 Hz, 3 H).

UPLC/MS (3 min) retention time 1.01 min.

LRMS: m/z 283, 285 (M+1).

### PREPARATION 25

### Methyl 2-{3-[2-{[(cyclopropylcarbonyl)(ethyl)amino]methyl}-4-(trifluoromethyl)phenyl]-1H-pyrrolo[3,2-b]pyridin-1-yl}propanoate

The title compound or Preparation 24 (50 mg, 0.18 mmol) and the title compound of Preparation 4 (70 mg, 0.18 mmol) were treated with aqueous caesium carbonate solution (2M, 0.17 ml, 0.35 mmol) and [1,1'-bis(diphenylphosphino)ferrocene]dichloro-palladium(II), complex with dichloromethane (14 mg, 0.02 mmol) according to the method of Preparation 23. The resulting residue was partially purified using the Isolera Purification System (methanol-dichloromethane gradient, 0:100 rising to 5:95) to give 42 mg of the crude title compound as a brown solid. Purity 80%.

Partial ¹H NMR (300 MHz, CHLOROFORM-d) 55:45 mixture of two rotamers.

Major rotamer δ ppm 4.90 (s, 2H), 3.77 (s, 2H).

Minor rotamer δ ppm 4.80 (s, 2 H), 3.79 (s, 2H).

UPLC/MS (3 min) retention time 1.72 min.

LRMS: m/z 474 (M+1).

### PREPARATION 26

### 5-Cyclopropyl-1H-pyrrolo[3,2-b]pyridine

The title compound of Preparation 19 (200 mg, 1.31 mmol) and cycloproylboronic acid (225 mg, 2.62 mmol) were dissolved in 4 ml toluene and 0.2 ml water in a microwave reactor. Potassium phosphate (835 mg, 3.93 mmol) and tricyclohexylphosphine (73.5 mg, 0.26 mmol) were added and the mixture was submitted to three vacuum-argon cycles. Palladium(II) acetate (29.4 mg, 0.13 mmol) was added and the mixture was further submitted to three vacuum-argon cycles. The reaction was stirred at 120 ºC for 2 h under microwave irradiation. The mixture was allowed to cool, diluted with ethyl acetate and filtered through Celite^{®} eluting with more ethyl acetate, and the filtrate was evaporated under reduced pressure. The resulting residue was purified using the Isolera Purification System (methanol-dichlorometane, 0:100 rising to 3:97) to give 101 mg (0.64 mmol, 49%) of the title compound as an oil. Purity 97%.

¹H NMR (300 MHz, CHLOROFORM-*d*) δ ppm 8.31 (br. s., 1 H), 7.58 (dd, *J*=8.38, 0.96 Hz, 1 H), 7.38 (d, *J*=1.92 Hz, 1 H), 6.94 (d, J=8.51 Hz, 1 H), 6.68 (d, *J*=3.30 Hz, 1 H), 2.14 - 2.25 (m, 1 H), 0.95 - 1.08 (m, 2 H), 0.47 - 0.71 (m, 2 H).

UPLC/MS (3 min) retention time 0.58 min.

LRMS: m/z 159 (M+1).

### PREPARATION 27

### 3-Bromo-5-cyclopropyl-1H-pyrrolo[3,2-b]pyridine

The title compound of Preparation 26 (101 mg, 0.64 mmol) was treated with copper(II) bromide (427 mg, 1.92 mmol) according to the method of Preparation 21 to give 115 mg (0.49 mmol, 76%) of the title compound as an oil. Purity 100%.

¹H NMR (300 MHz, CHLOROFORM-*d*) δ ppm 8.47 (br. s., 1 H), 7.55 (d, *J*=8.51 Hz, 1 H), 7.41 (d, *J*=2.47 Hz, 1 H), 6.91 (d, *J*=8.51 Hz, 1 H), 2.18 - 2.33 (m, 1 H), 0.96 - 1.07 (m, 4 H).

UPLC/MS (3 min) retention time 0.72 min.

LRMS: m/z 237, 239 (M+1).

### PREPARATION 28

### Ethyl (3-bromo-5-cyclopropyl-1H-pyrrolo[3,2-b]pyridin-1-yl)acetate

The title compound of Preparation 27 (110 mg, 0.46 mmol) was treated with sodium hydride (60% dispersion in oil, 20 mg, 0.51 mmol) and ethyl 2-bromoacetate (0.06 ml, 0.51 mmol) according to the method of Preparation 16. The resulting residue was purified using the Isolera Purification System (ethyl acetate-hexane gradient, 0:100 rising to 40:60) to give 98 mg (0.30 mmol, 66%) of the title compound as an oil. Purity 98%.

¹H NMR (300 MHz, CHLOROFORM-*d*) δ ppm 7.47 (d, *J*=9.61 Hz, 1 H), 7.32 (s, 1 H), 6.98 (d, *J*=8.51 Hz, 1 H), 4.81 (s, 2 H), 4.26 (q, *J*=7.14 Hz, 2 H), 2.19 - 2.37 (m, 1 H), 1.31 (t, *J*=7.14 Hz, 3 H), 1.02 - 1.14 (m, 4 H).

UPLC/MS (3 min) retention time 0.87 min.

LRMS: m/z 323 (M+1).

### PREPARATION 29

### Ethyl {5-cyclopropyl-3-[2-{[(cyclopropylcarbonyl)(ethyl)amino]methyl}-4-(trifluoromethyl)phenyl]-1H-pyrrolo[3,2-b]pyridin-1-yl}acetate

The title compound of Preparation 28 (98 mg, 0.30 mmol) was treated with the title compound of Preparation 4 (144 mg, 0.36 mmol), aqueous caesium carbonate solution (2 M, 0.30 ml, 0.6 mmol) and [1,1'-bis(diphenylphosphino)ferrocene]dichloro-palladium (II) complex with dichloromethane (27 mg, 0.03 mmol) according to the method of Preparation 23 followed by purification using the Isolera Purification System (methanol-dichloromethane gradient, 0:100 rising to 5:95) to give 100 mg (0.19 mmol, 65%) of the title compound as a white solid. Purity 96%.

UPLC/MS (3 min) retention time 1.96 min.

LRMS: m/z 514 (M+1).

### PREPARATION 30

### Methyl 2-(3-bromo-5-methyl-1H-pyrrolo[3,2-b]pyridin-1-yl)propanoate

The title compound of Preparation 21 (125 mg, 0.59 mmol) and the crude title compound of Preparation 1 (168 mg) were treated with sodium hydride (60% dispersion in oil, 0.026 g, 0.65 mmol) according to the method of Preparation 24. The resulting residue was partially purified using the Isolera Purification System (ether-hexane gradient, 0:100 rising to 100:0) to give 74 mg of the crude title compound as a colourless oil. Purity 76%.

UPLC/MS (3 min) retention time 0.86 min.

LRMS: m/z 297, 299 (M+1).

### PREPARATION 31

### Methyl 2-{3-[2-{[(cyclopropylcarbonyl)(ethyl)amino]methyl}-4-(trifluoromethyl)phenyl]-5-methyl-1H-pyrrolo[3,2-b]pyridin-1-yl}propanoate

The crude title compound of Preparation 30 (74 mg) and the title compound of Preparation 4 (98 mg, 0.25 mmol) were treated with aqueous caesium carbonate solution (2M, 0.25 mL, 0.50 mmol) and [1,1'-bis(diphenylphosphino)ferrocene]dichloro-palladium(II), complex with dichloromethane (20 mg, 0.02 mmol) according to the method of Preparation 23. The resulting residue was purified using the Isolera Purification System (methanol-dichloromethane gradient, 0:100 rising to 3:97) to give 53 mg (0.11 mmol, 18% over two steps) of the title compound as a brown solid. Purity 90%.

UPLC/MS (3 min) retention time 1.64 min.

LRMS: m/z 488 (M+1).

### PREPARATION 32

### 2-Bromo-6-(trifluoromethyl)pyridin-3-amine

6-(Trifluoromethyl)pyridine-3-amine (0.4 g, 2.51 mmol) was dissolved in 16 ml acetonitrile and the mixture was cooled in an ice bath. N-Bromosuccinimide (0.45 g, 2.54 mmol) was added and the reaction was stirred at 0 ºC for 30 min. The mixture was diluted with water and extracted three times with ethyl acetate. The combined organics were washed with brine, dried over anhydrous magnesium sulfate, filtered and evaporated under reduced pressure. The resulting residue was purified using the Isolera Purification System (ethyl acetate-hexane gradient, 0:100 rising to 20:80) to give 0.48 g (1.99 mmol, 80%) of the title compound as an oil. Purity 97%.

¹H NMR (300 MHz, CHLOROFORM-*d*) δ ppm 7.44 (d, *J*=8.24 Hz, 1 H), 7.05 (d, *J*=8.24 Hz, 1 H), 4.50 (br. s., 2 H).

UPLC/MS (3 min) retention time 1.43 min.

LRMS: m/z 241, 243 (M+1).

### PREPARATION 33

### 6-(Trifluoromethyl)-2-[(trimethylsilyl)ethynyl]pyridin-3-amine

The title compound of Preparation 32 (350 mg, 1.45 mmol) and ethynyltrimethylsilane (0.41 ml, 2.90 mmol) were dissolved in 15 ml tetrahydrofuran in a Schlenk vessel. 16 ml Triethylamine and copper(I) iodide (11 mg, 0.06 mmol) were added and the reaction mixture was submitted to three vacuum-argon cycles. Bis(triphenylphosphine)-palladium(II) dichloride (41 mg, 0.06 mmol) was added and the mixture was submitted to three further vacuum-argon cycles. The reaction vessel was sealed and the contents were stirred at 90 ºC for 3 h. The mixture was allowed to cool, diluted with ethyl acetate and filtered through Celite^{®} eluting with more ethyl acetate. The filtrate was washed with water, brine, dried over anhydrous magnesium sulfate, filtered and evaporated under reduced pressure. The resulting residue was purified using the Isolera Purification System (ethyl acetate-hexane gradient, 0:100 rising to 20:80) to give 295 mg (1.14 mmol, 79%). Purity 98%.

¹H NMR (300 MHz, CHLOROFORM-*d*) δ ppm 7.41 (d, *J*=8.51 Hz, 1 H), 7.07 (d, *J*=8.79 Hz, 1 H), 4.58 (br. s., 2 H), 0.30 (s, 9 H).

UPLC/MS (3 min) retention time 1.91 min.

LRMS: m/z 259 (M+1).

### PREPARATION 34

### 2-Ethynyl-6-(trifluoromethyl)pyridin-3-amine

The title compound of Preparation 33 (295 mg, 1.14 mmol) was dissolved in 10 ml methanol. Potassium carbonate (0.24 g, 1.71 mmol) was added and the mixture was stirred at room temperature for 2 h. The mixture was partitioned between ethyl acetate and water. The organic layer was washed with water, brine, dried over anhydrous magnesium sulfate, filtered and evaporated under reduced pressure gradient to give 180 mg (0.97 mmol, 86%) of the title compound as an oil. Purity 99%.

¹H NMR (300 MHz, CHLOROFORM-*d*) δ ppm 7.44 (d, J=8.51 Hz, 1 H), 7.08 (d, *J*=8.79 Hz, 1 H), 4.61 (br. s., 2 H), 3.55 (s, 1 H).

UPLC/MS (3 min) retention time 1.25 min.

LRMS: m/z 187 (M+1).

### PREPARATION 35

### 5-(Trifluoromethyl)-1H-pyrrolo[3,2-b]pyridine

Potassium tert-butoxide (228 mg, 2.03 mmol) was suspended in 0.5 ml 1-methyl-2-under nitrogen atmosphere. A solution of the title compound of Preparation 34 (180 mg, 0.97 mmol) in 0.5 ml 1-methyl-2-pyrrolidinone was added and the mixture was stirred at room temperature overnight. The mixture was partitioned between ethyl acetate and water. The organic phase was washed with water, brine, dried over anhydrous magnesium sulfate, filtered and evaporated under reduced pressure. The resulting residue was purified using the Isolera Purification System (ether-hexane gradient, 0:100 rising to 50:50) to give 115 mg (0.62 mmol, 64%) of the title compound as a white solid. Purity 99%.

¹H NMR (300 MHz, CHLOROFORM-*d*) δ ppm 8.66 (br. s., 1 H), 7.83 (d, *J*=8.51 Hz, 1 H), 7.62 (t, *J*=3.02 Hz, 1 H), 7.55 (d, *J*=8.51 Hz, 1 H), 6.84 - 6.92 (m, 1 H).

UPLC/MS (3 min) retention time 1.23 min.

LRMS: m/z 187 (M+1).

### PREPARATION 36

### 3-Bromo-5-(trifluoromethyl)-1H-pyrrolo[3,2-b]pyridine

The title compound of Preparation 35 (100 mg, 0.54 mmol) was treated with copper(II) bromide (360 mg, 1.61 mmol) according to the method of Preparation 21 to give 151 mg of the crude title compound as an oil. Purity 87%.

¹H NMR (300 MHz, CHLOROFORM-*d*) δ ppm 8.77 (br. s., 1 H), 7.84 (d, *J*=8.51 Hz, 1 H), 7.66 (d, *J*=3.57 Hz, 1 H), 7.60 (d, *J*=8.51 Hz, 1 H).

UPLC/MS (3 min) retention time 1.52 min.

LRMS: m/z 265, 267 (M+1).

### PREPARATION 37

### Ethyl [3-bromo-5-(trifluoromethyl)-1H-pyrrolo[3,2-b]pyridin-1-yl]acetate

The crude title compound of Preparation 36 (151 mg) was treated with sodium hydride (60% dispersion in oil, 24 mg, 0.63 mmol) and ethyl 2-bromoacetate (0.1 ml, 0.63 mmol) according to the method of Preparation 16. The resulting residue was partially purified using the Isolera Purification System (ethyl acetate-hexane gradient, 0:100 rising to 50:50) to give 75 mg of the crude title compound as an oil. Purity 85%.

¹H NMR (300 MHz, CHLOROFORM-*d*) δ ppm 7.71 (d, *J*=8.60 Hz, 1 H), 7.60 (d, *J*=8.60 Hz, 1 H), 7.53 (s, 1 H), 4.88 (s, 2 H), 4.24 (q, *J*=7.16 Hz, 2 H), 1.28 (t, *J*=7.03 Hz, 3H).

UPLC/MS (3 min) retention time 1.71 min.

LRMS: m/z 351, 353 (M+1).

### PREPARATION 38

### Ethyl [3-(2-{[(cyclopropylcarbonyl)(ethyl)amino]methyl}-4-fluorophenyl)-5-(trifluoromethyl)-1H-pyrrolo[3,2-b]pyridin-1-yl]acetate

The crude title compound of Preparation 37 (75 mg) was treated with the title compound of Preparation 4 (89 mg, 0.26 mmol), aqueous caesium carbonate solution (2 M, 0.21 ml, 0.4 mmol) and [1,1'-bis(diphenylphosphino)ferrocene]dichloropalladium (II) complex with dichloromethane (19 mg, 0.02 mmol) according to the method of Preparation 23 followed by purification using the Isolera Purification System (methanol-dichloromethane gradient, 0:100 rising to 5:95) to give 62 mg (0.13 mmol, 23% over three steps) of the title compound as a white solid. Purity 98%.

UPLC/MS (3 min) retention time 1.97 min.

LRMS: m/z 492 (M+1).

### PREPARATION 39

### 3-Bromo-1H-pyrrolo[3,2-c]pyridine

1*H*-Pyrrolo[3,2-c]pyridine (200 mg, 1.69 mmol) was treated with copper(II) bromide (1.13 g, 5.06 mmol) according to the method of Preparation 21 to give 280 mg (1.42 mmol, 84%) of the title compound as a pale yellow solid. Purity 100%.

¹H NMR (300 MHz, DMSO-*d*) δ ppm 8.71 (br. s., 1 H), 8.26 (d, J=5.49 Hz, 1 H), 7.67 (s, 1 H), 7.42 (d, *J*=5.77 Hz, 1 H).

HPLC/MS (9 min) retention time 0.58 min.

LRMS: m/z 197, 199 (M+1).

### PREPARATION 40

### Ethyl (3-bromo-1H-pyrrolo[3,2-c]pyridin-1-yl)acetate

The title compound of Preparation 39 (280 mg, 1.42 mmol) was treated with sodium hydride (60% dispersion in oil, 63 mg, 1.58 mmol) and ethyl 2-bromoacetate (0.17 ml, 1.54 mmol) according to the method of Preparation 16 to give 303 mg (1.07 mmol, 75%) of the title compound as a pale yellow solid. Purity 94%.

¹H NMR (300 MHz, CHLOROFORM-d) δ ppm 8.89 (s, 1 H), 8.42 (d, *J*=5.77 Hz, 1 H), 7.13 - 7.20 (m, 2 H), 4.83 (s, 2 H), 4.25 (q, *J*=7.14 Hz, 2 H), 1.29 (t, *J*=7.14 Hz, 4 H).

UPLC/MS (3 min) retention time 0.69 min.

LRMS: m/z 283, 285 (M+1).

### PREPARATION 41

### 2-Chloro-3-iodopyridin-4-amine

2-Chloro-4-aminopyridine (1.8 g, 14 mmol) was dissolved in 28 ml dimethylformamide. N-Iodosuccinimide (3.15 g, 14 mmol) was added and the mixture was stirred at room temperature overnight. Additional N-iodosuccinimide (3.15 g, 14 mmol) was added and the mixture wasa stirred at 55 ºC overnight. The mixture was evaporated to dryness and the residue was partitioned between ethyl acetate and water. The aqueous was extracted with ethyl acetate and the combined organics were dried over sodium sulphate, filtered and evaporated under reduced pressure. Purification by flash chromatography (ethyl acetate-hexane gradient, 15:85 rising to 25:75) gave 1.5 g (5.9 mmol, 42%) of the title compound as a purple solid.

¹H NMR (300 MHz, CHLOROFORM-*d*) δ ppm 8.34 (1 H, s), 6.63 (1 H, s), 4.75 (2 H, br. s.).

### PREPARATION 42

### tert-Butyl (2-chloro-3-iodopyridin-4-yl)carbamate

The title compound of Preparation 41 (6.4 g, 25 mmol) was dissolved in 25 ml tetrahydrofuran and the mixture was cooled in an ice-bath. Di-tert-butylcarbonate (6.0 g, 27.5 mmol) was added and the mixture was stirred for 1 h. The mixture was partitioned between saturated ammonium chloride solution and ethyl acetate. The organics were washed sequentially with 0.1N hydrochloric acid, water and brine. The organics were dried over sodium sulphate, filtered and evaporated under reduced pressure. The solid was recrystallised from ethanol and was dried in the oven to give 5.0 g (14 mmol, 56%) of the title compound. Purity 100%.

¹H NMR (400 MHz, CHLOROFORM-*d*) δ ppm 8.15 (1 H, d, *J*=5.5 Hz), 8.03 (1 H, d, *J*=5.5 Hz), 7.32 (1 H, br. s.), 1.55 (9H, s).

UPLC/MS (3 min) retention time 1.92 min.

LRMS: *m*/*z* 355 (M+1).

### PREPARATION 43

### tert-Butyl (2-chloro-3-prop-1-yn-1-ylpyridin-4-yl)carbamate

### Method A

The title compound of Preparation 42 (2.2 g, 6.2 mmol), copper(I) iodide (60 mg, 0.32 mmol) and (bis(triphenylphosphine) palladium(II) dichloride were suspended in 20 ml de-gassed dimethylformamide in a pressure tube. Nitrogen was bubbled through the mixture for several minutes and the mixture was cooled in a dry ice-acetonitrile bath. Propyne (0.9 ml, 15.8 mmol) was condensed into 2 ml triethylamine, cooled in a dry ice-acetonitrile bath and the solution was cannulated into the pressure tube. The tube was sealed and the mixture was stirred at 50 ºC for 5 h. The mixture was allowed to cool to room temperature and nitrogen was bubbled through the mixture for several minutes. The mixture was partitioned between ether and water and the organics were washed with water, dried over magnesium sulphate, filtered and evaporated under reduced pressure. The residue was purified by flash chromatography (ethyl acetate-hexane gradient, 0:100 rising to 10:90) to give 1.44 g (5.4 mmol, 87%) of the title compound as a yellow solid. Purity 100%.

### Method B

The title compound of Preparation 42 (0.88 g, 2.5 mmol), tributyl(prop-1-ynyl)stannane (0.90 ml, 2.95 mmol), tetrakis(triphenylphosphine)palladium (0.20 g, 0.17 mmol) and copper(I) iodide (57 mg, 0.30 mmol) were suspended in 6 ml de-gassed toluene in a pressure tube. Argon was bubbled through the mixture for several minutes, then the tube was sealed and the mixture was stirred at 100 ºC overnight. The mixture was allowed to cool to room temperature and 1.5 ml 0.88N ammonium hydroxide solution and 1.5 ml saturated ammonium chloride solution were added. The phases were separated and the aqueous was extracted twice with dichloromethane. The combined organics were dried over sodium sulphate, filtered and evaporated. The residue was purified using the SP1 Purification System (ethyl acetate-hexane gradient, 0:100 rising to 5:95) to give 0.46 g (1.7 mmol, 70%) of the title compound as a white solid. Purity 100%.

¹H NMR (400 MHz, CHLOROFORM-*d*) δ ppm 8.13 (1 H, d, *J*=5.9 Hz), 8.05 (1 H, d, *J*=5.9 Hz), 7.42 (1 H, br. s.), 2.25 (3 H, s), 1.55 (9 H, s).

UPLC/MS (3 min) retention time 1.90 min.

LRMS: *m*/*z* 267 (M+1).

### PREPARATION 44

### 4-Chloro-2-methyl-1H-pyrrolo[3,2-c]pyridine

The title compound of Preparation 43 (0.35 g, 1.3 mmol) was dissolved in 9 ml de-gassed dimethylformamide in a pressure tube and nitrogen was bubbled through the mixture for several minutes. Copper(I) iodide (13 mg, 0.07 mmol) was added, the tube was sealed and the mixture was stirred at 110 ºC overnight. The mixture was allowed to cool and was partitioned between ether and water. The aqueous was extracted twice with ether and the organics were washed with water and dried over sodium sulphate. Filtration and evaporation under reduced pressure gave 225 mg (1.35 mmol, 87%) of the title compound as a pale yellow solid. Purity 96%.

¹H NMR (400 MHz, CHLOROFORM-*d*) δ ppm 10.10 (1H, br. s.), 7.96 (1 H, d, *J*=5.5 Hz), 7.16 (1 H, d, *J*=5.5 Hz), 6.31 (1 H, s), 2.22 (3 H, s).

UPLC/MS (3 min) retention time 1.01 min.

LRMS: *m*/*z* 167 (M+1).

### PREPARATION 45

### 4-Chloro-3-iodo-2-methyl-1H-pyrrolo[3,2-c]pyridine

The title compound of Preparation 44 (200 mg, 1.20 mmol) was dissolved in 2.5 ml dimethylformamide. *N*-Iodosuccinimide (270 mg, 1.20 mmol) was added portion-wise to and the mixture was stirred at room temperature for 90 min. The reaction mixture was then diluted water and stirred for 20 min. The resultant precipitate was collected by filtration, washed with water and dried under vaccuum to give 321 mg (1.10 mmo, 91%) of the title compound as an off-white solid. Purity 90%.

¹H NMR (300 MHz, CHLOROFORM-*d*) δ ppm 11.35 (br s, 1 H), 7.96 (d, J = 5.8 Hz, 1 H), 7.23 (d, J = 5.8 Hz, 1H), 2.50 (s, 3H).

UPLC/MS (3 min) retention time 1.55 min.

LRMS: m/z 293 (M+1).

### PREPARATION 46

### Ethyl (4-chloro-3-iodo-2-methyl-1H-pyrrolo[3,2-c]pyridin-1-yl)acetate

The title compound of Preparation 45 (317 mg, 1.08 mmol) was suspended in 5 ml dimethylformamide. Sodium hydride (60% dispersion in oil, 0.044 g, 1.10 mmol) was added portion-wise and the mixture was stirred for 30 min at room temperature. Ethyl 2-bromoacetate (0.120 ml, 1.08 mmol) was added and the mixture was stirred overnight. The mixture was partitioned between water and ethyl acetate. The organic phase was washed with brine, dried over magnesium sulphate, filtered and evaporated. The residue was purified using the Isolera purification system (ethyl acetate-hexane gradient, 0:100 rising to 33:67) to give 231 mg (0.61 mmol, 56%) of the title compound as a white solid. Purity 100%.

¹H NMR (300 MHz, CHLOROFORM-*d*) δ ppm 8.07 (d, J = 5.5 Hz, 1 H), 7.09 (d, J = 5.5 Hz, 1H), 4.85 (s, 2H), 4.34 (q, J = 7.1 Hz, 2H), 2.51 (s, 3H), 1.28 (t. J=7.1 Hz, 3H).

UPLC/MS (3 min) retention time 1.72 min.

LRMS: m/z 379 (M+1).

### PREPARATION 47

### Ethyl {4-chloro-3-[2-{[(cyclopropylcarbonyl)(ethyl)amino]methyl}-4-(trifluoromethyl)phenyl]-2-methyl-1H-pyrrolo[3,2-c]pyridin-1-yl}acetate

The title compound of Preparation 46 (220 mg, 0.58 mmol) and the title compound of Preparation 4 (346 mg, 0.87 mmol) were dissolved in 8 ml dimethylformamide in a microwave tube. Potassium phosphate (370 mg, 1.74 mmol) and 2-dicyclohexyl-phosphino-2',6'-dimethoxybiphenyl (24 mg, 0.06 mmol) were added and the mixture was submitted to three vacuum-argon cycles. Tris(dibenzylideneacetone)-dipalladium (37 mg, 0.04 mmol) was then added and the mixture was further submitted to three vacuum-argon cycles. The reaction mixture was heated in the microwave at 120ºC for 35 min. The mixture was cooled to room temperature, filtered through Celite, and the Celite washed with ether. The filtrate was washed with water and the aqueous phase extracted twice with ether. The combined organics were washed sequentially with water, brine. dried over sodium sulphate, filtered and evaporated under reduced pressure. The residue was purified using the SP1 Purification System (ethanol-ether-hexane gradient, 0:0:100 rising to 1:9:90) to give 106 mg (0.20 mmol, 35%) of the title compound. Purity 96%.

UPLC/MS (3 min) retention time 1.92 min.

LRMS: m/z 522 (M+1).

### PREPARATION 48

### Ethyl {3-[2-{[(cyclopropylcarbonyl)(ethyl)amino]methyl}-4-(trifluoromethyl)phenyl]-2,4-dimethyl-1H-pyrrolo[3,2-c]pyridin-1-yl}acetate

The title compound of Preparation 47 (102 mg, 0.20 mmol) and methyl boronic acid (30 mg, 0.50 mmol) were dissolved in 1 ml 1,2-dimethoxyethane in a microwave tube. Potassium carbonate (81 mg, 0.59 mmol) was added and the reaction mixture was submitted to three vacuum-argon cycles. [1,1'-bis(diphenylphosphino)ferrocene]-dichloropalladium(II), complex with dichloromethane (24 mg, 0.03 mmol) was then added and the mixture was further submitted to three vacuum-argon cycles. The reaction mixture was heated in the microwave at 120 ºC for 4 h. The mixture was cooled to room temperature, filtered through Celite, and the filtrate evaporated to give 60 mg of the crude title compound as a dark oil. Purity 72%.

UPLC/MS (3 min) retention time 1.54 min.

LRMS: m/z 502 (M+1).

### PREPARATION 49

### N-Ethyl-N-[2-(1H-pyrrolo[2,3-b]pyridin-1-yl)-5-(trifluoromethyl)benzyl]cyclopropanecarboxamide

1-*H*-Pyrrolo[2,3*b*]pyridine (124 mg, 1.05 mmol), the title compound of Preparation 3 (400 mg, 1.14 mmol), copper(I) iodide (20 mg, 0.11 mmol) and potassium phosphate (440 mg, 2.07 mmol) were suspended in 1.5 ml dioxane in a Schlenk tube and were submitted to three vacuum-argon cycles. *N,N'*-Dimethylcyclohexane-1,2-diamine (0.025 ml, 0.16 mmol) was added and the resulting mixture was submitted to three further vacuum-argon cycles. The reaction was stirred at 130 ºC under argon for 20 h. Additional copper(I) iodide (20 mg, 0.11 mmol) and *N,N*-dimethylcyclohexane-1,2-diamine (0.025 ml, 0.16 mmol) was added, the mixture was submitted to three vacuum-argon cycles and was stirred at 130 ºC for 48 h. The mixture was allowed to cool and was filtered through Celite, eluting with ethyl acetate and dioxane. The filtrate was evaporated under reduced pressure and the residue was partially purified using the Isolera Purification System (ether-hexane gradient, 0:100 rising to 10:90) to give 165 mg of the crude title compound as a white solid. Purity 78%.

Partial H NMR (300 MHz, CHLOROFORM-*d*) 66:33 mixture of two rotamers.

Major rotamer δ ppm 6.69 (1 H, d, *J*=3.3 Hz), 4.45 (s, 2 H).

Minor rotamer δ ppm 6.75 (1 H, d, *J*=3.6 Hz), 4.59 (br. s., 2H).

UPLC/MS (3 min) retention time 1.87 min.

LRMS: m/z 388 (M+1).

### PREPARATION 50

### N-[2-(3-Bromo-1H-pyrrolo[2,3-b]pyridin-1-yl)-5-(trifluoromethyl)benzyl]-N-ethylcyclopropanecarboxamide

The crude title compound of Preparation 49 (104 mg) was dissolved in 0.5 ml ethyl acetate. *N*-Bromosuccinimide (48 mg, 0.27 mmol) was added portion-wise and the mixture was stirred at room temperature for 90 min. The mixture was partitioned between ethyl acetate and water. The aqueous phase was extracted twice with ethyl acetate and the combined organics were washed with brine, dried over anhydrous magnesium sulphate, filtered and evaporated under reduced pressure to give 130 mg (0.28 mmol, 42% over two steps). Purity 95%.

Partial H NMR (300 MHz, CHLOROFORM-d) 73:27 mixture of two rotamers.

Major rotamer δ ppm 4.46 (br. s., 2H).

Minor rotamer δ ppm 4.61 (br. s., 2H).

UPLC/MS (3 min) retention time 2.06 min.

LRMS: m/z 466, 468 (M+1).

### PREPARATION 51

### tert-Butyl {1-[2-{[(cyclopropycarbonyl)(ethyl)amine]methyl}-4-(trifluoromethyl)phenyl]-1H-pyrrolo[2,3-b]pyridin-3-yl}acetate

The title compound of Preparation 50 (130 mg, 0.28 mmol) was dissolved in 1.2 ml tetrahydrofuran under argon in a microwave tube. (2-*tert*-Butoxy-2-oxoethyl)zinc(II) chloride (0.5 M solution in ether, 1.68 ml, 0.84 mmol) was added and the mixture was submitted to three vacuum-argon cycles. 1,2,3,4,5-Pentaphenyl-1'(di-tert-butylphosphino)ferrocene (Q-Phos) (9.8 mg, 0.01 mmol) and bis(dibenzylideneacetone)palladium (8 mg, 0.01 mmol) were added and the resulting mixture was submitted to three further vacuum-argon cycles. The mixture was stirred at 100 ºC for 1 h under microwave irradiation at 100ºC. The mixture was allowed to cool and was diluted with ether and water and filtered through Celite, eluting with more ether and water. The organic layer was washed with brine, dried over anhydrous magnesium sulphate, filtered and evaporated under reduced pressure. The resulting residue was purified using the Isolera Purification System (ethyl acetate-hexane gradient, 0:100 rising to 70:30) to give 76 mg (0.15 mmol, 54%) of the title compound as a colourless oil. Purity 100%.

Partial ¹H NMR (300 MHz, CHLOROFORM-*d*) 60:40 mixture of two rotamers.

Major rotamer δ ppm 4.46 (s, 2 H), 3.73 (s, 2 H).

Minor rotamer δ ppm 4.61 (br. s., 2 H), 3.76 (s, 2 H).

UPLC/MS (3 min) retention time 2.09 min.

LRMS: m/z 502 (M+1).

### PREPARATION 52

### N-Ethyl-N-[5-fluoro-2-(1H-pyrrolo[2,3-b]pyridin-1-yl)benzyl]cyclopropanecarboxamide

The title compound of Preparation 6 (420 mg, 1.39 mmol) was treated with 1-*H-*pyrrolo[2,3*b*]pyridine (150 mg, 1.27 mmol), copper(I) iodide (20 mg, 0.13 mmol), potassium phosphate (540 mg, 2.54 mmol) and *N,N'*-dimethylcyclohexane-1,2-diamine (0.030 ml, 0.19 mmol) according to the method of Preparation 49. The resulting residue was purified using the Isolera Purification System (ether-hexane gradient, 0:100 rising to 100:0) to give 198 mg of the crude title compound as a colourless oil. Purity 87%.

Partial ¹H NMR (300 MHz, CHLOROFORM-*d*) 60:40 mixture of two rotamers.

Major rotamer δ ppm 6.65 (1 H, d, *J*=3.6 Hz).

Minor rotamer δ ppm 6.71 (1 H, d, *J*=3.6 Hz).

UPLC/MS (3 min) retention time 1.71 min.

LRMS: m/z 338 (M+1).

### PREPARATION 53

### N-[2-(3-Bromo-1H-pyrrolo[2,3-b]pyridin-1-yl)-5-fluorobenzyl]-N-ethylcyclopropanecarboxamide

The crude title compound of Preparation 52 (95 mg) was treated with *N-*bromosuccinimide (50 mg, 0.28 mmol) according to the method of Preparation 50 to give 110 mg (0.26 mmol, 39% over two steps) of the title compound as a brown solid. Purity 100%.

UPLC/MS (3 min) retention time 1.95 min.

LRMS:m/z416,418(M+1).

### PREPARATION 54

### tert-Butyl [1-(2-{[(cyclopropylcarbonyl)(ethyl)amino]methyl}-4-fluorophenyl)-1H-pyrrolo[2,3-b]pyridin-3-yl]acetate

The title compound of Preparation 53 (110 mg, 0.26 mmol) was treated with (2-*ter*t-butoxy-2-oxoethyl)zinc(II) chloride (0.5 M in ether, 1.6 ml, 0.80 mmol), 1,2,3,4,5-Pentaphenyl-1'-(di-tert-butylphosphino)ferrocene (Q-Phos) (9.3 mg, 0.01 mmol) and bis(dibenzylideneacetone)palladium (7.5 mg, 0.01 mmol) according to the method of Preparation 51. The resulting residue was purified using the Isolera Purification System (ether-hexane gradient, 0:100 rising to 100:0) to give 45 mg (0.10 mmol, 38%) of the title compound as a colourless oil. Purity 93%.

Partial ¹H NMR (300 MHz, CHLOROFORM-d) 57:43 mixture of two rotamers.

Major rotamer δ ppm 3.73 (s, 2 H).

Minor rotamer δ ppm 3.76 (s, 2 H).

UPLC/MS (3 min) retention time 1.98 min.

LRMS:m/z452(M+1).

### PREPARATION 55

### 1H-Pyrrolo[2,3-b]pyridine 7-oxide

1H-Pyrrolo[2,3b]pyridine (9.2 g, 78 mmol) was dissolved in 260 ml dimethoxyethane and the solution was cooled in an ice-bath. 3-Chloroperbenzoic acid (77% purity, 31 g, 138 mmol) was added portion-wise and the mixture was stirred at room temperature for 2 h. The solvent volume was reduced to half under reduced pressure and the precipitate formed was collected by filtration and washed with ether. The solid was suspended in 150 ml water, the aqueous basifed to pH 9 with saturated potassium carbonate solution and left in the refrigerator overnight. The precipitate was collected by filtration, washed with hexane and dried in vacuo to obtain 7.0 g (52 mmol, 68%) of the title compound as a white solid. Purity 98%.

¹H NMR (300 MHz, DMSO-*d*) δ ppm 8.13 (d, *J*=5.28 Hz, 1H), 7.65 (d, *J*=8.22 Hz, 1 H), 7.46 (d, J=2.93 Hz, 1 H), 7.01 - 7.13 (m, 1 H), 6.58 (d, *J*=2.93 Hz, 1 H).

UPLC/MS (3 min) retention time 0.61 min.

LRMS: m/z 135 (M+1).

### PREPARATION 56

### Ethyl 6-chloro-1H-pyrrolo[2,3-b]pyridine-1-carboxylate

The title compound of Preparation 55 (10.5 g, 78 mmol) was dissolved in 800 ml tetrahydrofuran under a nitrogen atmosphere. 1,1,1,3,3,3-Hexamethyldisilazane (18.2 ml, 86 mol) was added with stirring. Ethyl chloroformate (26.5 ml, 280 mmol) was added drop-wise and the mixture was stirred at room temperature for 2 h. The solvent was evaporated under reduced pressure, the residue was re-dissolved in ethyl acetate and the organics washed with saturated sodium bicarbonate solutions, brine and dried over anhydrous sodium sulphate. The organic were filtered, evaporated under reduced pressure and the residue was purified using the Isolera Purification System (ether-hexane gradient, 0:100 rising to 30:70) to give 10.2 g (45 mmol, 58%) of the title compound as a colourless oil. Purity 100%.

¹H NMR (300 MHz, CHLOROFORM-*d*) δ ppm 7.84 (d, *J*=8.22 Hz, 1 H), 7.72 (d, *J*=4.11 Hz, 1 H), 7.24 - 7.32 (m, 1 H), 6.55 (d, *J*=3.52 Hz, 1 H), 4.55 (q, *J*=7.04 Hz, 2 H), 1.49 (t, *J*=7.04 Hz, 3 H).

UPLC/MS (3 min) retention time 1.59 min.

LRMS: m/z 225, 227(M+1).

### PREPARATION 57

### 6-Chloro-1H-pyrrolo[2,3-b]pyridine

The title compound of Preparation 56 (4.96 g, 22.0 mmol) was dissolved in 225 ml methanol. 1N Sodium hydroxide solution (75 ml, 75 mmol) was added and the mixture was stirred at room temperature for 4 h. The methanol was evaporated under reduced pressure and the aqueous layer was extracted three times with dichloromethane. The combined organics were dried over anhydrous magnesium sulphate, filtered and evaporated under under reduced pressure to give 3.18 g (20.8 mmol, 95%) of the title compound as a white solid. Purity 94%.

¹H NMR (300 MHz, CHLOROFORM-*d*) δ ppm 10.14 (br. s., 1 H), 7.91 (d, *J*=8.22 Hz, 1 H), 7.36 (dd, *J*=3.52, 2.35 Hz, 1 H), 7.12 (d, *J*=8.22 Hz, 1 H), 6.52 (dd, 1 H).

UPLC/MS (3 min) retention time 1.38 min.

LRMS: m/z 153,155 (M+1).

### PREPARATION 58

### 6-Methyl-1H-pyrrolo[2,3-b]pyridine

The title compound of Preparation 57 (1.00 g, 6.55 mmol), 2,4,6-trimethyl-1,3,5,2,4,6-trioxatriborinane (1.0 ml, 7.2 mmol) and potassium carbonate (2.72 g, 19.7 mmol) were suspended in 13 ml dimethoxyethane in a microwave reactor and submitted to three vacuum-argon cycles. [1,1'-Bis(diphenylphosphino)ferrocene]-dichloropalladium (II) complex with dichloromethane (0.29 g, 0.33 mmol) was added and the resulting mixture was submitted to three further vacuum-argon cycles. The mixture was stirred at 120ºC for 2h under microwave irradiation. The mixture was allowed to cool and was filtered through Celite, eluting with ethyl acetate. The filtrate was evaporated under reduced pressure and the residue was purified using the Isolera Purification System (ethyl acetate-hexane gradient, 0:100 rising to 50:50) to give 0.57 g (4.31 mmol, 65%) of the title compound as a beige solid. Purity 98%.

¹H NMR (300 MHz, CHLOROFORM-*d*) δ ppm 10.87 (br. s., 1 H), 7.86 (d, *J*=7.63 Hz, 1 H), 7.29 (dd, *J*=3.52, 2.35 Hz, 1 H), 6.97 (d, *J*=7.63 Hz, 1 H), 6.47 (dd, *J*=3.52,1.76 Hz, 1 H), 2.69 (s, 3 H).

HPLC/MS (15 min) retention time 2.57 min.

LRMS:m/z133(M+1).

### PREPARATION 59

### N-Ethyl-N-[2-(6-methyl-1H-pyrrolo[2,3-b]pyridin-1-yl)-5-(trifluoromethyl)benzyl]cyclopropanecarboxamide

The title compound of Preparation 58 (1.48 g, 11.2 mmol) and the title compound of Preparation 3 (3.92 g, 11.20 mmol) were treated with copper(I) iodide (0.21 g, 1.12 mmol), potassium phosphate (4.75 g, 22.38 mmol) and *N,N*-dimethylcyclohexane-1,2-diamine (0.27 ml, 1.68 mmol) according to the method of Preparation 49. The resulting residue was purified using the Isolera Purification System (ether-hexane gradient, 0:100 rising to 100:0) to give 3.40 g (8.48 mmol, 76%) of the title compound as a colourless oil. Purity 95%.

Partial ¹H NMR (300 MHz, CHLOROFORM-d) 55:45 mixture of two rotamers.

Major rotamer δ ppm 6.68 (1 H, d, *J*=3.3 Hz), 2.53 (s, 3 H).

Minor rotamer δ ppm 6.62 (1 H, d, *J*=3.3 Hz), 2.57 (s, 3 H).

HPLC/MS (15 min) retention time 8.80 min.

LRMS:m/z402(M+1).

### PREPARATION 60

### N-[2-(3-Bromo-6-methyl-1H-pyrrolo[2,3-b]pyridin-1-yl)-5-(trifluoromethyl)benzyl]-N-ethylcyclopropanecarboxamide

The title compound of Preparation 59 (0.74 g, 1.84 mmol) was treated with *N-*bromosuccinimide (0.33 g, 0.28 mmol) according to the method of Preparation 50. The resulting residue was purified using the Isolera Purification System (ether-hexane gradient, 0:100 rising to 50:50) to give 0.71 g (1.48 mmol, 80%) of the title compound as a colourless oil. Purity 97%.

Partial ¹H NMR (300 MHz, CHLOROFORM-d) 50:50 mixture of two rotamers.

Rotamer 1 δ ppm 2.58 (s, 3 H).

Rotamer 2 δ ppm 2.55 (s, 3 H).

UPLC/MS (3 min) retention time 2.17 min.

LRMS: m/z 480, 482 (M+1).

### PREPARATION 61

### tert-Butyl {1-[2-{[(cyclopropylcarbonyl)(ethyl)amino]methyl}-4-(trifluoromethyl)phenyl]-6-methyl-1H-pyrrolo[2,3-b]pyridin-3-yl}acetate

The title compound of Preparation 60 (0.70 g, 1.46 mmol) was treated with (2-*tert-*butoxy-2-oxoethyl)zinc(II) chloride (0.5 M in ether, 8.80 ml, 4.40 mmol), 1,2,3,4,5-pentaphenyl-1'-(di-tert-butylphosphino)ferrocene (Q-Phos) (0.052 g, 0.07 mmol) and bis(dibenzylideneacetone)palladium (0.042 g, 0.07 mmol) according to the method of Preparation 51. The resulting residue was purified using the Isolera Purification System (ether-hexane gradient, 0:100 rising to 100:0) to give 0.49 g (0.95 mmol, 65%) of the title compound as a colourless oil. Purity 90%.

Partial ¹H NMR (300 MHz, CHLOROFORM-d) 50:50 mixture of two rotamers.

Rotamer 1 δ ppm 3.73 (s, 2 H), 2.56 (s, 3 H).

Rotamer 2 δ ppm 3.69 (s, 2 H), 2.53 (s, 3 H).

UPLC/MS (3 min) retention time 2.18 min.

LRMS: m/z 516 (M+1).

### PREPARATION 62

### 6-Cyclopropyl-1H-pyrrolo[2,3-b]pyridine

The title compound of Preparation 57 (300 mg, 1.97 mmol) was treated with cyclopropylboronic acid (0.34 g, 3.93 mmol), potassium phosphate (1.25 g, 5.89 mmol), tricyclohexylphosphine (0.088 g, 0.31 mmol) and palladium(II) acetate (0.044g, 0.20 mmol) according to the method of Preparation 26. The resulting residue was purified using the Isolera Purification System (ether-hexane gradient, 0:100 rising to 100:0) to give 110 mg of the crude title compound as a yellow solid. Purity 88%.

¹H NMR (300 MHz, CHLOROFORM-d) δ ppm 9.68 (br. s., 1 H), 7.82 (d, *J*=8.2 Hz, 1 H), 7.25 (d, *J*=3.6, 2.2 Hz, 1 H), 6.96 (d, *J*=8.2 Hz, 1 H), 6.45 (dd, *J*=3.6, 2.2 Hz, 1 H), 2.08 - 2.23 (m, 1 H), 1.00-1.10 (m, 4 H)

UPLC/MS (3 min) retention time 0.96 min.

LRMS: m/z 159 (M+1).

### PREPARATION 63

### N-[2-(6-Cyclopropyl-1H-pyrrolo[2,3-b]pyridin-1-yl)-5-(trifluoromethyl)benzyl]-N-ethylcyclopropanecarboxamide

The crude title compound of Preparation 62 (110 mg) and the title compound of Preparation 3 (250 mg, 0.71 mmol) were treated with copper(I) iodide (13.5 mg, 0.07 mmol), potassium phosphate (300 g, 1.43 mmol) and *N,N*-dimethylcyclohexane-1,2-diamine (0.034 ml, 0.22 mmol) according to the method of Preparation 49. The resulting residue was purified using the Isolera Purification System (ether-hexane gradient, 0:100 rising to 100:0) to give 110 mg (0.26 mmol, 13% over two steps) of the title compound as a colourless oil. Purity 94%.

Partial ¹H NMR (300 MHz, CHLOROFORM-d) 66:33 mixture of two rotamers.

Major rotamer δ ppm 4.48 (s, 2 H).

Minor rotamer δ ppm 4.62 (br.s., 2 H).

HPLC/MS (15 min) retention time 9.47 min.

LRMS: m/z 428 (M+1).

### PREPARATION 64

### N-[2-(3-Bromo-6-cyclopropyl-1H-pyrrolo[2,3-b]pyridin-1-yl)-5-(trifluoromethyl)benzyl]-N-ethylcyclopropanecarboxamide

The title compound of Preparation 63 (110 mg, 0.26 mmol) was treated with N-bromosuccinimide (44 mg, 0.25 mmol) according to the method of Preparation 50 to give 120 mg of the crude title compound as a colourless oil. Purity 73%.

Partial ¹H NMR (300 MHz, CHLOROFORM-d 66:33 mixture of two rotamers.

Major rotamer δ ppm 4.47 (s, 2 H).

Minor rotamer δ ppm 4.60 (br. s., 2 H).

UPLC/MS (3 min) retention time 2.29 min.

LRMS: m/z 506, 508 (M+1).

### PREPARATION 65

### tert-Butyl {6-cyclopropyl-1-{[2-{(cyclopropylcarbonyl)(ethyl)amino]methyl}-4-(trifluoromethyl)phenyl]-1H-pyrrolo[2,3-b]pyridin-3-yl}acetate

The crude title compound of Preparation 64 (110 mg) was treated with (2-*tert*-butoxy-2-oxoethyl)zinc(II) chloride (0.5 M in ether, 1.30 ml, 0.65 mmol), 1,2,3,4,5-pentaphenyl-1'-(di-tert-butylphosphino)ferrocene (Q-Phos) (7.6 mg, 0.01 mmol) and bis(dibenzylideneacetone)palladium (6.2 mg, 0.01 mmol) according to the method of Preparation 51. The resulting residue was purified using the Isolera Purification System (ether-hexane gradient, 0:100 rising to 80:20) to give 42 mg (0.077 mmol, 30% over two steps) of the title compound as a colourless oil. Purity 93%.

Partial ¹H NMR (300 MHz, CHLOROFORM-d) 66:33 mixture of two rotamers.

Major rotamer δ ppm 4.49 (s, 2 H), 3.67 (s, 2 H).

Minor rotamer δ ppm 4.68 (br. s., 2 H), 3.70 (s, 2 H).

HPLC/MS (15 min) retention time 10.13 min.

LRMS: m/z 542 (M+1).

### PREPARATION 66

### N-Ethyl-N-[5-fluoro-2-(6-methyl-1H-pyrrolo[2,3-b] pyridin-1-yl)benzyl]cyclopropanecarboxamide

The title compound of Preparation 58 (160 mg, 1.21 mmol) and the title compound of Preparation 6 (360 mg, 1.21 mmol) were treated with copper(I) iodide (23 mg, 0.12 mmol), potassium phosphate (510 mg, 2.42 mmol) and *N,N*-dimethylcyclohexane-1,2-diamine (0.042 ml, 0.27 mmol) according to the method of Preparation 49. The resulting residue was purified using the Isolora Purification System (ether-hexane gradient, 0:100 rising to 100:0) to give 170 mg (0.48 mmol, 40%) of the title compound as a colourless oil. Purity 100%.

Partial ¹H NMR (300 MHz, CHLOROFORM-d) 50:50 mixture of two rotamers.

Rotamer 1 δ ppm 2.56 (s, 3 H)

Rotamer 2 δ ppm 2.52 (s, 3 H)

UPLC/MS (3 min) retention time 1.87 min.

LRMS: m/z 352 (M+1).

### PREPARATION 67

### N-[2-(3-Bromo-6-methyl-1H-pyrrolo[2,3-b]pyridin-1-yl)-5-fluorobenzyl]-N-ethylcyclopropanecarboxamide

The title compound of Preparation 66 (170 mg, 0.48 mmol) was treated with *N-*bromosuccinimide (86 mg, 0.48 mmol) according to the method of Preparation 50. The resulting residue was purified using the Isolera Purification System (ether-hexane gradient, 0:100 rising to 60:40) to give 120 mg of the crude title compound and as a colourless oil. Purity 70%.

UPLC/MS (3 min) retention time 2.09min.

LRMS: m/z 430, 432 (M+1).

### PREPARATION 68

### tert-Butyl [1-(2-{[(cyclopropylcarbonyl)(ethyl)amino]methyl}-4-fluorophenyl)-6-methyl-1H-pyrrolo[2,3-b]pyridin-3-yl]acetate

The crude title compound of Preparation 67 (120 mg) was treated with (2-*tert*-butoxy-2-oxoethyl)zinc(II) chloride (0.5 M in ether, 2.30 ml, 1.15 mmol), 1,2,3,4,5-pentaphenyl-1'-(di-tert-butylphosphino)ferrocene (Q-Phos) (10.0 mg, 0.01 mmol) and bis(dibenzylideneacetone)palladium (8.0 mg, 0.01 mmol) according to the method of Preparation 51. The resulting residue was purified using the Isolera Purification System (ether-hexane gradient, 0:100 rising to 80:20) to give 54 mg (0.12 mmol, 24% over two steps) of the title compound as a colourless oil. Purity 100%.

Partial ¹H NMR (300 MHz, CHLOROFORM-d) 50:50 mixture of two rotamers.

Rotamer 1 δ ppm 3.71 (s, 2 H), 2.51 (s, 3 H).

Rotamer 2 δ ppm 3.68 (s, 2 H), 2.55 (s, 3 H).

HPLC/MS (15 min) retention time 9.17 min.

LRMS: m/z 466 (M+1).

### PREPARATION 69

### N-Ethyl-N-[2-(6-methyl-1H-pyrrolo[2,3-b]pyridin-1-yl)benzyl]cyclopropanecarboxamide

The title compound of Preparation 58 (160 mg, 1.21 mmol) and the title compound of Preparation 11 (340 mg, 1.21 mmol) was treated with copper(I) iodide (23 mg, 0.12 mmol), potassium phosphate (510 mg, 2.42 mmol) and *N,N*-dimethylcyclohexane-1,2-diamine (0.042 ml, 0.27 mmol) according to the method of Preparation 49. The resulting residue was purified by reverse-phase chromatography using the Isolera Purification system to give 120 mg (0.36 mmol, 30%) of the title compound as a colourless oil. Purity 100%.

Partial ¹H NMR (300 MHz, CHLOROFORM-d) 66:33 mixture of two rotamers.

Major rotamer δ ppm 2.52 (s, 3 H)

Minor rotamer δ ppm 2.55 (s, 3 H).

UPLC/MS (3 min) retention time 1.87 min.

LRMS: m/z 334 (M+1).

### PREPARATION 70

### N-[2-(3-Bromo-6-methyl-1H-pyrrolo[2,3-b]pyridin-1-yl)benzyl]-N-ethylcyclopropanecarboxamide

The title compound of Preparation 69 (120 mg, 0.36 mmol) was treated with N-bromosuccinimide (48 mg, 0.27 mmol) according to the method of Preparation 50. The resulting residue was purified using the Isolera Purification System (ethyl acetate-hexane gradient, 0:100 rising to 50:50) to give 120 mg of the crude title compound as a colourless oil. Purity 78%.

UPLC/MS (3 min) retention time 2.10 min.

LRMS: m/z 412, 414 (M+1).

### PREPARATION 71

### tert-Butyl[1-(2-{[(cyclopropylcarbonyl)(ethyl)amino]methyl}phenyl)-6-methyl-1H-pyrrolo[2,3-b]pyridin-3-yl]acetate

The crude title compound of Preparation 70 (120 mg) was treated with (2-tert-butoxy-2-oxoethyl)zinc(II) chloride (2.30 ml of a 0.5 M solution in ether, 1.15 mmol), 1,2,3,4,5-pentaphenyl-1'-(di-tert-butylphosphino)ferrocene (Q-Phos) (10 mg, 0.01 mmol) and bis(dibenzylideneacetone)palladium (8 mg, 0.01 mmol) according to the method of Preparation 51. The resulting residue was purified using the Isolera Purification System (ether-hexane gradient, 0:100 rising to 80:20) to give 68 mg of the crude title compound as a colourless oil. Purity 85%.

UPLC/MS (3 min) retention time 2.08 min.

LRMS: m/z 448 (M+1).

### PREPARATION 72

### N-Ethyl-N-[2-(6-methoxy-1H-pyrrolo[2,3-b]pyridin-1-yl)-5-(trifluoromethyl)benzyl]cyclopropanecarboxamide

6-Methoxy-1H-pyrrolo[2,3-b]pyridine (148 mg, 1.00 mmol), potassium phosphate (427 mg, 2.01 mmol) and the title compound of Preparation 3 (350 mg, 1.00 mmol) were suspended in 2 ml dioxane in a Schlenk vessel. The mixture was subjected to three vacuum-argon cycles. Copper(I) iodide (019 mg, 0.10 mmol) and trans-N¹,N²-dimethylcyclohexane-1,2-diamine (0.024 ml, 0.15 mmol,) were added and the mixture was further submitted to three vacuum-argon cycled. The reaction vessel was sealed and the mixture was stirred and heated to 130ºC for 21 h. The mixture was allowed to cool to room temperature, was diluted with ethyl acetate and filtered through Celite. The filtrate was evaporated under reduced pressure and the residue was purified using the Isolera purification system (ethyl acetate-hexane gradient, 0:100 rising to 15:85) to give 342 mg of the title compound (0.82 mmol, 82%) as a clear oil. Purity 96%.

Partial ¹H NMR (300 MHz, CHLOROFORM-d) 60:40 mixture of two rotamers.

Major rotamer δ ppm 4.55 (s, 2H), 3.84 (s, 2H).

Minor rotamer δ ppm 4.65 (s, 2H), 3.78 (s, 2H).

UPLC/MS (3 min) retention time 2.09 min.

LRMS: m/z 418 (M+1).

### PREPARATION 73

### N-[2-(3-Bromo-6-methoxy-1H-pyrrolo[2,3-b]pyridin-1-yl)-5-(trifluoromethyl)benzyl]-N-ethylcyclopropanecarboxamide

The title compound of Preparation 72 (334 mg, 0.80 mmol) was dissolved in 5 ml ethyl acetate and was cooled in an ice-bath. *N*-Bromosuccinimide (143 mg, 0.80 mmol) was added portionwise and the mixture was stirred at room temperature overnight. Further *N*-bromosuccinimide (40 mg, 0.22 mmol) was added to the cooled mixture and stirring was continued for a further 2 h. The reaction mixture was then diluted with water and extracted with ethyl acetate. The organic extract was washed with brine, dried over magnesium sulfate and evaporated under reduced pressure. The residue was purified using the Isolera purification system (ether-hexane gradient, 0:100 rising to 15:85) to give 273 mg of the crude title compound as an oil. Purity 75%.

Partial ¹H NMR (300 MHz, CHLOROFORM-d) 65:35 mixture of two rotamers.

Major rotamer δ ppm 4.55 (s, 2H), 3.84 (s, 2H).

Minor rotamer δ ppm 4.65 (s, 2H), 3.78 (s, 2H).

UPLC/MS (3 min) retention time 2.23 min.

LRMS: m/z 496, 498 (M+1).

### PREPARATION 74

### tert-Butyl {1-[2-{[(cyclopropylcarbonyl)(ethyl)amino]methyl}-4-(trifluoromethyl)phenyl]-6-methoxy-1H-pyrrolo[2,3-b]pyridin-3-yl}acetate

The crude title compound of Preparation 73 (263 mg), (2-tert-butoxy-2-oxoethyl)zinc(II) chloride (0.5 M in ether, 2.65 ml, 1.33 mmol), 1,2,3,4,5-pentaphenyl-1'-(di-tert-butylphosphino)ferrocene (15 mg, 0.02 mmol) and bis(dibenzylideneacetone)palladium (12 mg, 0.02 mmol) were mixed together under an argon atmosphere. The mixture was stirred and heated to 100 ºC under microwave irradiation fro 1 h. Further (2-tert-butoxy-2-oxoethyl)zinc(II) chloride (0.5 M in ether, 1.3 ml, 0.65 mmol), 1,2,3,4,5-pentaphenyl-1'-(di-tert-butylphosphino)ferrocene (15 mg, 0.01 mmol) and bis(dibenzylideneacetone)palladium (12 mg, 0.01 mmol) were added and the mixture was heated at 100 ºC heating for a further 1.5 h. The mixture was diluted with ethyl acetate, filtered through Celite. The filtrate was washed with water, brine, dried over magnesium sulfate and evaporated under reduced pressure. The residue was purified using the Isolera purification system (ether-hexane gradient, 0:100 rising to 75:25) to give 183 mg of the crude title compound as an oil. Purity 80%.

Martial ¹H NMR (300 MHz, CHLOROFORM-d) 53:47 mixture of two rotamers.

Major rotamer δ ppm 4.57 (s, 2H), 3.84 (s, 2H).

Minor rotamer δ ppm 4.67 (s, 2H), 3.78 (s, 2H).

UPLC/MS (3 min) retention time 2.23 min.

LRMS: m/z 532 (M+1).

### PREPARATION 75

### Methyl 6-iodo-1H-pyrrolo[2,3-b]pyridine-1-carboxylate

The title compound of Preparation 55 (1.60 g, 11.9 mmol) was dissolved in 100 ml tetrahydrofuran under a nitrogen atmosphere. 1,1,1,3,3,3-Hexamethyldisilazane (2.50 ml, 12.0 mol) and iodotrimethylsilane (5.11 ml, 35.8 mmol) were added with stirring. Methyl chloroformate (1.80 ml, 23.3 mmol) was added drop-wise and the mixture was stirred at room temperature for 5 h. The solvent was evaporated under reduced pressure, the residue was re-dissolved in ethyl acetate and the organics washed with saturated sodium bicarbonate solution, brine and dried over anhydrous sodium sulphate. The organics were filtered, evaporated under reduced pressure and the residue was purified using the Isolera Purification System (ether-hexane gradient, 0:100 rising to 100:0) to give 0.85 g (2.81 mmol, 24%) of the title compound as a brown solid. Purity 100%.

¹H NMR (300 MHz, CHLOROFORM-*d*) δ ppm 7.68 (1 H, d, *J*=4.1 Hz), 7.63 (1 H, d, *J*=7.5 Hz), 7.55 (1 H, d, *J*=7.5 Hz), 6.55 (1 H, d, *J*=4.1 Hz), 4.10 (3 H,s).

UPLC/MS (3 min) retention time 1.56 min.

LRMS: m/z 303 (M+1).

### PREPARATION 76

### Methyl 6-(trifluoromethyl)-1H-pyrrolo[2,3-b]pyridine-1-carboxylate

The title compound of Preparation 75 (200 mg, 0.66 mmol) and methyl 2,2-difluoro-2-(fluorosulfonyl)acetate (336 µl, 2.64 mmol) were dissolved in 3 ml dimethylformamide in a Schlenk vessel and the mixture was subjected to three vacuum-argon cycles. Copper iodide was added and the reaction vessel was sealed and the mixture was stirred and heated to 115 ºC for 3 h. The mixture was allowed to cool to room temperature and was diluted with ethyl acetate. The mixture was washed sequentially with water, 10% lithium chloride solution and brine, was dried over magnesium sulphate, filtered and evaporated under reduced pressure. The residue was purified using the Isolera purification system (ether-hexane gradient, 0:100 rising to 10:90) to give 105 mg (0.43 mmol, 65%) of the title compound as a white solid. Purity 100%.

¹H NMR (300 MHz, CHLOROFORM-*d*) δ ppm 8.05 (d, *J*=8.2 Hz, 1 H), 7.92 (d, *J*=4.1 Hz, 1 H), 7.62 (d, *J*=8.2 Hz, 1 H), 6.66 (d, *J*=4.1 Hz, 1 H), 4.13 (s, 3 H).

UPLC/MS (3 min) retention time 1.58 min.

LRMS: m/z 245 (M+1).

### PREPARATION 77

### 6-(Trifluoromethyl)-1H-pyrrolo[2,3-b]pyridine

The title compound of Preparation 76 (200 mg, 0.82 mmol) was treated with 2.0 M aqueous sodium hydroxide solution (4.1 ml, 8.2 mmol) according to the method of Preparation 57 to give 140 mg (0.75 mmol, 92%) of the title compound as a white solid. Purity 100%.

¹H NMR (300 MHz, CHLOROFORM-*d*) δ ppm 11.52 (br. s., 1 H), 8.13 (d, J=8.2 Hz, 1 H), 7.57 - 7.64 (m, 1 H), 7.52 (d, *J*=8.2 Hz, 1 H), 6.59 - 6.67 (m, J=3.5 Hz, 1 H).

UPLC/MS (3 min) retention time 1.53 min.

LRMS: m/z 187 (M+1).

### PREPARATION 78

### N-Ethyl-N-(5-(trifluoromethyl)-2-(6-(trifluoromethyl)-1H-pyrrolo[2,3-b]pyridin-1-yl)benzyl)cyclopropanecarboxamide

The title compound of Preparation 77 (70 mg, 0.38 mmol) and the title compound of Preparation 3 (197 mg, 0.56 mmol) were dissolved in 1 ml acetonitrile. Salicylaldoxime (5 mg, 0.04 mmol), caesium carbonate (247 mg, 0.76 mmol) and copper(I) oxide (6 mg, 0.04 mmol) were added under argon atmosphere. The mixture was stirred and heated in the microwave to 150 ºC for 20 h. The mixture allowed to cool and was diluted with ethyl acetate, filtered through Celite and the filtrate washed with water. The aqueous phase was extracted twice with ethyl acetate and the combined organics were washed with brine, dried over sodium sulphate, filtered and evaporated under reduced pressure. The residue was purified using the Isolora purification system (ether-hexane gradient, 0:100 rising to 100:0) to give 95 mg (0.21 mmol, 55%) of the title compound as a colourless oil. Purity 90%.

Partial ¹H NMR (300 MHz, CHLOROFORM-d) 56:44 mixture of two rotamers.

Major rotamer δ ppm 6.77 (d, *J*=3.5 Hz, 1 H)

Minor rotamer δ ppm 6.85 (d, *J*=3.5 Hz, 1 H)

HPLC/MS (5 min) retention time 3.68 min.

LRMS: m/z 456 (M+1).

### PREPARATION 79

### N-(2-(3-Bromo-6-(trifluoromethyl)-1H-pyrrolo[2,3-b]pyridin-1-yl)-5-(trifluoromethyl)benzyl)-N-ethylcyclopropanecarboxamide

The title compound of Preparation 78 (95 mg, 0.21 mmol) was treated with N-bromosuccinimide (40 mg, 0.22 mmol) according to the method of Preparation 50. The crude product was purified using the Isolera purification system (ether-hexane gradient, 0:100 rising to 100:0) to give 77 mg (0.14 mmol, 69%) of the title compound as a colourless oil. Purity 95%.

Partial ¹H NMR (300 MHz, CHLOROFORM-d) 59:41 mixture of two rotamers.

Major rotamer δ ppm 8.12 (d, *J*=8.2 Hz, 1 H), 4.47 (s, 2H).

Minor rotamer δ ppm 8.16 (d, *J*=8.2 Hz, 1 H).

UPLC/MS (3 min) retention time 2.19 min.

LRMS: m/z 534, 536 (M+1).

### PREPARATION 80

### tert-Butyl-2-(1-(2-((N-ethylcyclopropanecarboxamido)methyl)-4-(trifluoromethyl)phenyl)-6-(trifluoromethyl)-1H-pyrrolo[2,3-b]pyridin-3-yl)acetate

The title compound of Preparation 79 (75 mg, 0.14 mmol), (2-tert-butoxy-2-oxoethyl)zinc(II) chloride (0.5 M in ether, 0.82 ml, 0.41 mmol), 1,2,3.4..5-Pentaphenyl-1'-(di-tert-butylphosphino)ferrocene (5.0 mg, 0.01 mmol) and bis(dibenzylideneacetone)palladium (5.0 mg, 0.01 mmol) were mixed together under argon atmosphere. The mixture was heated in the microwave at 100 ºC for 1.5 h. The mixture was then partitioned between water and ethyl acetate and the aqueous layer was extracted twice with ethyl acetate. The combined organic extract was washed with brine, dried over magnesium sulphate, filtered and evaporated under reduced pressure. The residue was purified using the Isolera purification system (ether-hexane gradient, 0:100 rising to 100:0) to give 55 mg (0.097 mmol, 67%) of the title compound as a beige solid. Purity 90%.

Partial ¹H NMR (300 MHz, CHLOROFORM-d) 53:47 mixture of two rotamers.

Major rotamer δ ppm 3.74 (s, 2 H).

Minor rotamer δ ppm 3.79 (s, 2 H).

UPLC/MS (3 min) retention time 2.23 min.

LRMS: m/z 570 (M+1).

### PREPARATION 81

### Methyl pent-4-ynoate

Pent-4-yonic acid (1.5 g, 15.3 mmol) was dissolved in 50 ml methanol and the mixture was cooled in an ice bath. Thionyl chloride (1.3 ml, 18.3 mmol) was added and the mixture was stirred at room temperature overnight. The mixture was partitioned between dichloromethane, and water. The organic phase was washed sequentially with 4% w/v sodium bicarbonate solution, water and brine, was dried over anhydrous magnesium sulfate, filtered and evaporated under reduced pressure to give 1,61 g (14.4 mmol, 94%) of the title compound as a colourless oil. Purity 95%.

¹H NMR (300 MHz, CHLOROFORM-*d*) δ ppm 3.71 (s, 3 H), 3.65 (s, 1 H), 2.44 - 2.65 (m, 4 H).

### PREPARATION 82

### Methyl 5-pyrimidin-2-ylpent-4-ynoate

2-Chloropyrimidine (300 mg, 2.62 mmol) and the title compound of Preparation 81 (352 mg, 3.14 mmol) were dissolved in 12 ml triethylamine in a Schenk vessel. Copper(I) iodide (199 mg, 1.05 mmol) was added and the reaction mixture was submitted to three vacuum-argon cycles. Bis(triphenylphosphine)palladium(II) dichloride (367 mg, 0.5 mmol) was added and the mixture was submitted to three further vacuum-argon cycles. The reaction vessel was sealed and the contents were stirred at 80 ºC for 3 h. The mixture was poured into saturated ammonium chloride solution and extracted with ethyl acetate. The organic phase was washed sequentially with water and brine, dried over anhydrous magnesium sulfate, filtered and evaporated under reduced pressure. The resulting residue was purified using the Isolera Purification System (ethyl acetate-hexane gradient, 0:100 rising to 30:70) to give 71 mg (0.37 mmol, 14%) of the title compound. Purity 98%.

¹H NMR (300 MHz, CHLOROFORM-*d*) δ ppm 8.70 (d, *J*=4.94 Hz, 2 H), 7.24 (t, *J*=4.94 Hz, 1 H), 3.72 (s, 3 H), 2.76 - 2.88 (m, 2 H), 2.64 - 2.77 (m, 2 H).

UPLC/MS (3 min) retention time 0.90 min.

LRMS: m/z 191 (M+1).

### PREPARATION 83

### Methyl pyrrolo[1,2-a]pyrimidin-6-ylacetate

The title compound of Preparation 82 (350 mg, 1.84 mmol) was dissolved in 40 ml dimethylacetamide in a microwave reactor. Copper(I) bromide (267 mg, 1.86 mmol) and triethylamine (5.7 ml, 41 mmol) were added and the mixture was stirred at 150 ºC for 1 h under microwave irradiation. The mixture was poured into saturated ammonium chloride solutions and was extracted with ethyl acetate. The organic phase was washed with water, brine, dried over anhydrous magnesium sulfate, filtered and evaporated under reduced pressure. The resulting residue was purified using the Isolera Purification System (ethyl acetate-hexane gradient, 0:100 rising to 30:70) to give 91 mg (0.48 mmol, 26%) of the title compound. Purity 99%.

¹H NMR (300 MHz, CHLOROFORM-*d*) δ ppm 8.19 (d, *J*=7.14 Hz, 1 H), 8.12 (d, *J*=3.85 Hz, 1 H), 6.91 (d, *J*=4.12 Hz, 1 H), 6.64 (d, *J*=4.12 Hz, 1 H), 6.59 (dd, *J*=7.14, 3.85 Hz, 1 H), 3.93 (s, 2 H), 3.71 (s, 3 H).

UPLC/MS (3 min) retention time 1.02 min.

LRMS: m/z 191 (M+1).

### PREPARATION 84

### Methyl (8-bromopyrrolo[1,2-a]pyrimidin-6-yl)acetate

The title compound of Preparation 83 (90 mg, 0.47 mmol) was dissolved in 6 ml dimethylformamide and the mixture was cooled in an ice bath. *N*-Bromosuccinimide (84 mg, 0.47 mmol) was added and the mixture was stirred at 0 ºC for 1 h. The mixture was diluted with ethyl acetate, washed with water, brine, dried over anhydrous magnesium sulfate, filtered and evaporated under reduced pressure to give 116 mg (0.43 mmol, 91%) of the title compound as a white solid. Purity 99%.

¹H NMR (300 MHz, CHLOROFORM-*d*) δ ppm 8.20 (dd, *J*=3.85, 1.65 Hz, 1 H), 8.16 (d, *J*=7.14 Hz, 1 H), 6.94 (s, 1 H), 6.62 (dd, *J*=7.14, 3.85 Hz, 1 H), 3.90 (s, 2 H), 3.72 (s, 3 H).

UPLC/MS (3 min) retention time 1.29 min.

LRMS: m/z 269, 271 (M+1).

### PREPARATION 85

### Methyl {8-[2-{[(cyclopropylcarbonyl)(ethyl)amino]methyl}-4-(trifluoromethyl)phenyl]pyrrolo[1,2-a]pyrimidin-6-yl}acetate

The title compound of Preparation 84 (56 mg, 0.21 mmol) was treated with the title compound of Preparation 4 (98 mg, 0.25 mmol), aqueous caesium carbonate solution (2M, 0.21 ml, 0.4 mmol) and [1,1'-bis(diphenylphosphino)ferrocene]dichloro-palladium (II) complex with dichloromethane (18 mg, 0.02 mmol) according to the method of Preparation 23 followed by purification using the Isolera Purification System (methanol-dichloromethane gradient, 0:100 rising to 3:97) to give 57 mg (0.12 mmol, 59%) of the title compound as a white solid. Purity 98%.

Partial ¹H NMR (300 MHz, CHLOROFORM-*d*) 55:45 mixture of two rotamers.

Major rotamer δ ppm 4.84 (s, 2 H), 4.01 (s, 2 H), 3.77 (s, 3 H).

Minor rotamer δ ppm 4.77 (s, 2 H), 3.98 (s, 2 H), 3.75 (s, 3 H).

UPLC/MS (3 min) retention time 1.88 min.

LRMS: m/z 460 (M+1).

### PREPARATION 86

### Methyl [8-(2-{[(cyclopropylcarbonyl)(ethyl)amino]methyl}-4-fluorophenyl)pyrrolo[1,2-a]pyrimidin-6-yl]acetate

The title compound of Preparation 84 (78 mg, 0.29 mmol) was treated with the title compound of Preparation 7(111 mg, 0.32 mmol), aqueous caesium carbonate solution (2M, 0.29 ml, 0.6 mmol) and [1,1'-bis(diphenylphosphino)ferrocene]dichloro-palladium (II) complex with dichloromethane (26 mg, 0.03 mmol) according to the method of Preparation 23 followed by purification using the Isolera Purification System (methanol-dichloromethane gradient, 0:100 rising to 5:95) to give 60 mg (0.15 mmol, 51 %) of the title compound as a white solid. Purity 97%.

Partial ¹H NMR (300 MHz, CHLOROFORM-d) 60:40 mixture of two rotamers.

Major rotamer δ ppm 4.74 (s, 2 H), 3.99 (s, 2 H), 3.76 (s, 3 H).

Minor rotamer δ ppm 4.67 (s, 2 H), 3.96 (s, 2 H), 3.74 (s, 3 H).

UPLC/MS (3 min) retention time 1.71 min.

LRMS: m/z 410 (M+1).

### PREPARATION 87

### Methyl 5-[4-(trifluoromethyl)pyrimidin-2-yl]pent-4-ynoate 2-Chloro-4-(trifluoromethyl)pyrimidine (0.5 ml, 4.14 mmol) was treated with the title compound of Preparation 81 (0.56 g, 4.97 mmol), copper (I) iodide (32 mg, 0.17 mmol) and bis(triphenylphosphine)palladium(II) chloride (58 mg, 0.08 mmol) according to the method of Preparation 82 followed by purification using the Isolera Purification System (ether-hexane gradient, 0:100 rising to 50:50) to give 0.64 g (2.48 mmol, 60%) of the title compound as a pale yellow solid. Purity 95%.

¹H NMR (300 MHz, CHLOROFORM-*d*) δ ppm 8.95 (d, *J*=4.94 Hz, 1 H), 7.55 (d, *J*=4.94 Hz, 1 H), 3.73 (s, 3 H), 2.79 - 2.89 (m, 2 H), 2.64 - 2.77 (m, 2 H).

UPLC/MS (3 min) retention time 1.45 min.

LRMS: m/z 259 (M+1).

### PREPARATION 88

### Methyl [2-(trifluoromethyl)pyrroloo[1,2-a]pyrimidin-6-yl]acetate

The title compound of Preparation 87 (286 mg, 1.11 mmol) was treated with copper(I) bromide (160 mg, 1.11 mmol) and triethylamine (2 ml) according to the method of Preparation 83 followed by reverse-phase purification using the Isolera Purification System (methanol-water gradient, 0:100 rising to 100:0) to give 35 mg (0.135 mmol, 12%) of the title compound as a pale yellow solid. Purity 95%.

¹H NMR (400 MHz, CHLOROFORM-*d*) δ ppm 8.33 (d, *J*=7.03 Hz, 1 H), 7.07 (d, *J*=4.30 Hz, 1 H), 6.85 - 6.92 (m, 2 H), 3.98 (s, 2 H), 3.72 (s, 3 H).

UPLC/MS (3 min) retention time 1.54 min.

LRMS: m/z 259 (M+1).

### PREPARATION 89

### Methyl [8-bromo-2-(trifluoromethyl)pyrrolo[1,2-a]pyrimidin-6-yl]acetate

The title compound of Preparation 88 (67 mg, 0.26 mmol) was treated with N-bromosuccinimide (42 mg, 0.24 mmol) according to the method of Preparation 84 to give 77 mg (0.23 mmol, 88%) of the title compound as a pale yellow solid. Purity 95%.

¹H NMR (300 MHz, CHLOROFORM-*d*) δ ppm 8.30 (d, *J*=7.63 Hz, 1 H), 7.10 (s, 1 H), 6.88 - 6.94 (m, 1 H), 3.95 (s, 2 H), 3.72 (s, 3 H).

HPLC/MS (15 min) retention time 7.68 min.

LRMS: m/z 337, 339 (M+1).

### PREPARATION 90

### Methyl [8-[2-{[(cyclopropylcarbonyl)(ethyl)amino]methyl}-4-(trifluoromethyl)phenyl]-2-(trifluoromethyl)pyrrolo[1,2-a]pyrimidin-6-yl]acetate

The title compound of Preparation 89 (77 mg, 0.23 mmol) was treated with the title compound of Preparation 4 (91 mg, 0.23 mmol), caesium carbonate (2M aqueous solution, 0.35 ml, 0.70 mmol), [1,1'-bis(diphenylphosphino)ferrocene]dichloro-palladium(II), complex with dichloromethane (20 mg, 0.02 mmol) according to the method of Preparation 23 followed by purification using the Isolera Purification System (ether-hexane, 50:50 rising to 100:0) to give 65 mg (0.12 mmol, 54%) of the title compound as a pale yellow solid. Purity 95%.

Partial ¹H NMR (300 MHz, CHLOROFORM-d) 60:40 mixture of two rotamers.

Major rotamer δ ppm 4.85 (s, 2 H), 4.06 (s, 2 H), 3.78 (s, 3 H).

Minor rotamer δ ppm 4.74 (s, 2 H), 4.01 (s, 2 H), 3.76 (s, 3 H).

HPLC/MS (9 min) retention time 7.10 min.

LRMS: m/z 528 (M+1).

### PREPARATION 91

### Methyl 5-(4-methylpyrimidin-2-yl)pent-4-ynoate

2-Chloro-4-methylpyrimidine (256 mg, 2.0 mmol) was treated with the title compound of Preparation 81 (268 mg, 2.4 mmol), copper(I) iodide (15.2 mg, 0.08 mmol), bis(triphenylphosphine)palladium(II) bichloride (28 mg, 0.04 mmol) and triethylamine (12 ml) according to the method of Preparation 82. The resulting residue was purified using the Isolera Purification System (ethyl acetate-hexane gradient, 0:100 rising to 30:70) to give 180 mg (0.88 mmol, 44%) as an oil. Purity 98%.

¹H NMR (300 MHz, CHLOROFORM-*d*) δ ppm 8.53 (d, *J*=4.70 Hz, 1 H), 7.09 (d, *J*=5.28 Hz, 1 H), 3.72 (s, 3 H), 2.75 - 2.86 (m, 2 H), 2.65 - 2.75 (m, 2 H), 2.53 (s, 3 H).

UPLC/MS (3 min) retention time 1.02 min.

LRMS: m/z 205 (M+1).

### PREPARATION 92

### Methyl (2-methylpyrrolo[1,2-a]pyrimidin-6-yl)acetate

The title compound of Preparation 91 (260 mg, 1.27 mmol) was dissolved in 24 ml dimethylacetamide in a microwave reactor. Copper(I) bromide (184 mg, 1.29 mmol) and triethylamine (3.5 ml, 25 mmol) were added and the mixture was stirred at 180 ºC for 15 min under microwave irradiation, according to the method of Preparation 83. The resulting residue was purified using the Isolera Purification System (ether-hexane gradient, 0:100 rising to 40:60) to give 77 mg (0.38 mmol, 30%) as a white solid. Purity 100%.

¹H NMR (300 MHz, CHLOROFORM-*d*) δ ppm 8.08 (d, *J*=7.04 Hz, 1 H), 6.81 (d, *J*=4.11 Hz, 1 H), 6.42 - 6.52 (m, 2 H), 3.89 (s, 2 H), 3.70 (s, 3 H), 2.51 (s, 3 H).

UPLC/MS (3 min) retention time 1.04 min.

LRMS: m/z 205 (M+1).

### PREPARATION 93

### Methyl (8-bromo-2-methyl pyrrolo[1,2-a]pyrimidin-6-yl)acetate

The title compound of Preparation 92 (77 mg, 0.38 mmol) was dissolved in 3 ml dichloromethane and the mixture was cooled to -10 ºC in an ice-acetone bath. *N-*Bromosuccinimide (67 mg, 0.38 mmol) was added and the mixture was stirred at -10 ºC for 1 h. The mixture was diluted with ethyl acetate, washed sequentially with water and brine, dried over anhydrous magnesium sulfate, filtered and evaporated under reduced pressure. The resulting residue was purified using the Isolera Purification System (ether-hexane gradient, 0:100 rising to 40:60) to give 70 mg (0.25 mmol, 66%) as a white solid. Purity 99%.

¹H NMR (300 MHz, CHLOROFORM-*d*) δ ppm 8.03 (d, *J*=7.63 Hz, 1 H), 6.83 (s, 1 H), 6.49 (d, *J*=7.04 Hz, 1 H), 3.86 (s, 2 H), 3.70 (s, 3 H), 2.55 (s, 3 H).

UPLC/MS (3 min) retention time 1.39 min.

LRMS: m/z 283, 285 (M+1).

### PREPARATION 94

### Methyl {8-[2-{[(cyclopropylcarbonyl)(ethyl)amino]methyl}-4-(trifluoromethyl)phenyl]-2-methylpyrrolo[1,2-a]pyrimidin-6-yl}acetate

The title compound of Preparation 93 (70 mg, 0.25 mmol) was treated with the title compound of Preparation 4 (117 mg, 0.3 mmol), aqueous caesium carbonate solution (2M, 0.25 ml, 0.5 mmol) and [1,1'-bis(diphenylphosphino)ferrocene]dichloro-palladium (II) complex with dichloromethane (22 mg, 0.02 mmol) according to the method of Preparation 23 followed by purification using the Isolera Purification System (methanol-dichloromethane gradient, 0:100 rising to 5:95) to give 60 mg (0.13 mmol, 51%) of the title compound as a white solid. Purity 96%.

Partial ¹H NMR (300 MHz, CHLOROFORM-d) 65:35 mixture of two rotamers.

Major rotamer δ ppm 4.92 (s, 2 H), 3.96 (s, 2 H), 3.76 (s, 3 H).

Minor rotamer δ ppm 4.81 (s, 2 H), 3.93 (s, 2 H), 3.73 (s, 3 H).

UPLC/MS (3 min) retention time 1.97 min.

LRMS: m/z 474 (M+1).

### PREPARATION 95

### Ethyl (2Z)-3-amino-3-pyridin-2-ylacrylate

2-Cyanopyridine (2.7 g, 25.9 mmol) was dissolved in 55 ml 1,2-dichloroethane. Potassium ethyl malonate (6.6 g, 38.9 mmol), zinc chloride (2.6 g, 13.0 mmol) and *N*,*N*-diisopropylethylamine were added and the reaction mixture was stirred at reflux for 6 h under nitrogen atmosphere using a Dean-Stark head. The mixture was allowed to cool and the organics were washed with 25 ml saturated aqueous ammonium chloride solutions. The organic phase was dried over sodium sulphate, filtered and evaporated under reduced pressure. The residue was purified using the SP1 Purification System (ethyl acetate-hexane gradient, 0:100 rising to 20:80) to give 4.4 g (22.9 mmol, 88%) of the title compound as a yellow solid. Purity 100%.

¹H NMR (400 MHz, CHLOROFORM-d) δ ppm 1.32 (t, *J*=7.23 Hz, 3 H), 4.21 (q, *J*=7.03 Hz, 2 H), 5.35 (s, 1 H), 7.32 - 7.41 (m, 1 H), 7.73 - 7.82 (m, 2 H), 8.64 (d, *J*=4.69 Hz, 1 H).

UPLC/MS (3 min) retention time 1.43 min.

LRMS: m/z 193 (M+1).

### PREPARATION 96

### Ethyl 3-amino-3-pyridin-2-ylpropanoate

The title compound of Preparation 95 (400 mg, 1.85 mmol) was dissolved in 14 ml ethanol. Palladium hydroxide (20% on carbon, 195 mg, 0.28 mmol) and acetic acid (0.212 ml, 3.71 mmol) were added and the mixture was stirred at room temperature under 14 psi hydrogen atmosphere for 5 h. The catalyst was removed by filtration and the filtrate was evaporated under reduced pressure. The residue was dissolved in dichloromethane and washed sequentially with saturated sodium carbonate solution and brine. The organic phase was dried over sodium sulphate, filtered and evaporated under reduced pressure to give 280 mg (1.44 mmol, 69%) of the title compound as an oil. Purity 92%.

¹H NMR (400 MHz, CHLOROFORM-d) δ ppm 1.24 (t, *J*=7.03 Hz, 3 H), 2.65 - 2.80 (m, 1 H), 2.80 - 2.91 (m, 1 H), 4.15 (q, *J*=7.03 Hz, 2 H), 4.44 (m, 1 H), 7.18 (m, 1 H), 7.36 (d, *J*=7.42 Hz, 1 H), 7.67 (t, *J*=6.84 Hz, 1 H), 8.57 (br. s., 1 H).

UPLC/MS (3 min) retention time 0.56 min.

LRMS: m/z 195 (M+1).

### PREPARATION 97

### Ethyl 3-(formylamino)-3-pyridin-2-ylpropanoate

The title compound of Preparation 96 (100 mg, 0.43 mmol) and formic acid (0.142 ml, 3.65 mmol) were stirred at reflux for 4 h. The mixture of reaction was evaporated under reduced pressure to give 95 mg of the crude title compound as a dark oil. Purity 83%.

UPLC/MS (3 min) retention time 0.79 min.

LRMS: m/z 223 (M+1).

### PREPARATION 98

### Ethyl imidazo[1,5-a]pyridin-1-ylacetate

### Method A

The crude title compound of Preparation 97 (80 mg) was dissolved in 0.5 ml toluene. Phosphorous oxychloride (0.125 ml, 1.37 mmol) was slowly added and the mixture was stirred at 100 ºC for 3 h. The solvent was evaporated under reduced pressure and the residue was diluted with water and basified to pH 9 with 2N sodium hydroxide solution. The aqueous phase was extracted twice with dichloromethane and the combined organics were washed with water, dried over sodium sulphate, filtered and evaporated under reduced pressure to give 45 mg of the crude title compound as a dark oil. Purity 83%.

### Method B

The title compound of Preparation 96 (800 mg, 4.12 mmol) was dissolved in 20 ml n-butyl acetate. Formic acid (0.179 ml, 4.74 mmol) was added followed by drop-wise addition of propane phosphoric acid anhydride (T3P) (6.40 ml, 10.76 mmol) and the mixture was stirred at room temperature for 1 h and then at reflux for 2 h. The mixture was allowed to cool to room temperature and was washed twice with saturated sodium bicarbonate solution. The organics were dried over sodium sulphate, filtered and evaporated under reduced pressure. The residue was purified by reverse-phase chromatography using the SP1 Purification system to give 150 mg (0.73 mmol, 18%) of the title compound as a brown oil. Purity 100%.

¹H NMR (400 MHz, CHLOROFORM-d) δ ppm 1.26 (t, *J*=7.23 Hz, 3 H), 3.92 (s, 2 H), 4.17 (q, *J*=7.16 Hz, 2 H), 6.54 (t, *J*=6.64 Hz, 1 H), 6.69 (dd, *J*=8.99, 6.64 Hz, 1 H), 7.43 (d, *J*=9.38 Hz, 1 H), 7.89 (d, *J*=7.03 Hz, 1 H), 8.06 (s, 1 H).

UPLC/MS (3 min) retention time 0.81 min.

LRMS: m/z 205 (M+1).

### PREPARATION 99

### Ethyl (3-bromolmidazo[1,5-a]pyridin-1-yl)acetate

The title compound of Preparation 98 (150 mg, 0.73 mmol) was dissolved in 6 ml dimethylformamide. *N*-Bromosuccinimide (130 mg, 0.73 mmol) was added and the mixture was stirred at 55 ºC for 2 h. The mixture was allowed to cool and was poured into water. The organic phase was washed with water, dried over sodium sulphate, filtered and evaporated under reduced pressure to give 121 mg (0.43 mmol, 58%) of the title compound as a dark oil. Purity 100%.

¹H NMR (400 MHz, CHLOROFORM-d) δ ppm 1.26 (t, *J*=7.23 Hz, 3 H), 3.92 (s, 2 H), 4.17 (q, *J*=7.29 Hz, 2 H), 6.66 (t, *J*=6.45 Hz, 1 H), 6.76 (dd, *J*=8.99, 6.64 Hz, 1 H), 7.42 (d, *J*=9.38 Hz, 1 H), 7.82 (d, *J*=7.03 Hz, 1 H).

UPLC/MS (3 min) retention time 1.42 min.

LRMS: m/z 283, 285 (M+ 1).

### PREPARATION 100

### Ethyl {3-[2-{[(cyclopropylcarbonyl)(ethyl)amino]methyl}-4-(trifluoromethyl)phenyl]imidazo[1,5-a]pyridin-1-yl}acetate

The title compound of Preparation 99 (120 mg, 0.42 mmol) and the title compound of Preparation 4 (185 mg, 0.47 mmol) were dissolved in 4 ml dioxane in a Schlenk vessel. A solution of caesium carbonate (2M, 0.64 ml, 1.27 mmol) was added and the reaction mixture was submitted to three vacuum-argon cycles. [1,1'-bis(diphenyl-phosphino)ferrocene]dichloropalladium(II), complex with dichloromethane (35 mg, 0.04 mmol) was then added and the mixture was further submitted to three vacuum-argon cycles. The reaction vessel was sealed and the contents were stirred at 100 ºC for 6 h. The mixture was cooled to room temperature, filtered through Celite, the filtrate evaporated and the residue dissolved in ethyl acetate. The organics were washed sequentially with water and brine, dried over sodium sulphate, filtered and evaporated under reduced pressure. The residue was purified using the SP1 Purification System (ethyl acetate-hexane gradient, 0:100 rising to 40:60) to give 55 mg (0.12 mmol, 27%) of the title compound as a yellow semi-solid. Purity 100%.

Partial ¹H NMR (400 MHz, CHLOROFORM-d) 75:25 mixture of two rotamers.

Major rotamer δ ppm 4.59 (s, 2 H), 3.99 (s, 2H).

Minor rotamer δ ppm 4.85 (s, 2 H), 4.00 (2, 2H).

UPLC/MS (3 min) retention time 1.85 min.

LRMS: m/z 474 (M+1).

### PREPARATION 101

### 1,7-Dimethylpyrrolo[1,2-a]pyrazine

2,3-Dimethylpyrazine (4.1 g, 37.7 mmol) and 1-chloropropan-2-one (3.1 ml, 38.9 mmol) were dissolved in 8 ml acetonitrile and were stirred at at 120ºC for 12 h under microwave irradiation. The reaction was allowed to cool and was partitioned between ethyl acetate and water. The aqueous phase was basified with sodium bicarbonate and was extracted three times with ethyl acetate. The combined organics were dried over magnesium sulphate and evaporated under reduced pressure. The resulting residue was purified using the Isolera Purification System (2-propanol-hexane gradient, 0:100 rising to 20:80) to give 0.96 g (6.53 mmol, 17%) of the title compound as a pale orange oil. Purity 98%.

¹H NMR (300 MHz, CHLOROFORM-*d*) δ ppm 7.66 (d, *J*=4.94 Hz, 1 H), 7.41 (d, *J*=4.94 Hz, 1 H), 7.25 (s, 1 H), 6.62 (s, 1 H), 2.67 (s, 3 H), 2.40 (s, 3 H).

UPLC/MS (3 min) retention time 0.48 min.

LRMS: m/z 147 (M+1).

### PREPARATION 102

### Ethyl (1,7-dimethylpyrrolo[1,2-a]pyrazin-6-yl)(oxo)acetate

Aluminium trichloride (2.6 g, 19.5 mmol) was dissolved in 10 ml chlorobenzene under nitrogen atmosphere and the mixture was cooled in an ice-bath. Ethyl 2-chloro-2-oxoacetate (2.2 ml, 19.7 mmol) was added drop-wise and the mixture was stirred at 0º C for 10 min. A solution of the title compound of Preparation 101 (0.96 g, 6.53 mmol) dissolved in 5 ml chlorobenzene was added drop-wise and the resulting mixture was stirred at room temperature overnight. The mixture was washed with cold aqueous ammonia and the aqueous phase was extracted three times with ethyl acetate. The combined organics were washed sequentially with water, brine, dried over anhydrous magnesium sulphate and evaporated under reduced pressure. The resulting residue was purified using the Isolera Purification System (ether-hexane gradient, 50:50 rising to 100:0) to give 0.36 g (1.45 mmol, 23%) of the title compound as colourless oil. Purity 100%.

¹H NMR (300 MHz, CHLOROFORM-*d*) δ ppm 9.41 (d, *J*=5.49 Hz, 1 H), 7.88 (d, *J*=4.94 Hz, 1 H), 6.63 (s, 1 H), 4.46 (q, *J*=7.14 Hz, 2 H), 2.74 (s, 3 H), 2.46 (s, 3 H), 1.44 (t, *J*=7.14 Hz, 3 H).

UPLC/MS (3 min) retention time 1.56 min.

LRMS: m/z 247 (M+1).

### PREPARATION 103

### Ethyl (1,7-dimethylpyrrolo[1,2-a]pyrazin-6-yl)acetate

The title compound of Preparation 102 (0.36 g, 1.45 mmol) was dissolved in 5 ml trifluoroacetic acid. Triethylsilane (14 ml, 88 mmol) was added and the mixture was stirred at 80 ºC for 5 h. The mixture was allowed to cool and was poured into a solution of ammonia in water. The aqueous was extracted twice with chloroform, and the combined organics were dried over anhydrous magnesium sulphate and evaporated under reduced pressure. The resulting residue was purified using the Isolera Purification System (2-propanol-hexane gradient, 0:100 rising to 10:90) to give 0.22 g (0.95 mmol, 65%) of the title compound as yellow oil. Purity 99%.

¹H NMR (300 MHz, CHLOROFORM-*d*) δ ppm 7.63 (d, *J*=5.22 Hz, 1 H), 7.45 (d, *J*=4.94 Hz, 1 H), 6.63 (s, 1 H), 4.15 (q, *J*=7.14 Hz, 2 H), 3.86 (s, 2 H), 2.63 (s, 3 H), 2.35 (s, 3 H), 1.25 (t, *J*=7.14 Hz, 3 H).

UPLC/MS (3 min) retention time 0.78 min.

LRMS: m/z 233 (M+1).

### PREPARATION 104

### Ethyl (8-bromo-1,7-dimethylpyrrolo[1,2-a]pyrazin-6-yl)acetate

The title compound of Preparation 103 (34 mg, 0.13 mmol) was dissolved in 1.5 ml dimethylformamide and the mixture was cooled in an ice-bath. *N*-Bromosuccinimide (24 mg, 0.13 mmol) was added portion-wise and the mixture was stirred at room temperature for 40 main. The mixture was partitioned between ethyl acetate and water. And the organics were washed with 4% w/v sodium bicarbonate solution, dried over anhydrous magnesium sulphate and evaporated under reduced pressure to give 36 mg (0.12 mmol, 85%) of the title compound as a beige solid. Purity 98%.

¹H NMR (300 MHz, CHLOROFORM-*d*) δ ppm 7.61 (d, *J*=4.94 Hz, 1 H), 7.41 (d, *J*=4.94 Hz, 1 H), 4.15 (q, *J*=7.14 Hz, 2 H), 3.86 (s, 2 H), 2.95 (s, 3 H), 2.28 (s, 3 H), 1.24 (t, *J*=7.14 Hz, 4 H).

UPLC/MS (3 min) retention time 1.08 min.

LRMS: m/z 311, 313 (M+1).

### PREPARATION 105

### Ethyl {8-[2-{[(cyclopropylcarbonyl)(ethyl)amino]methyl}-4-(trifluoromethyl)phenyl]-1,7-dimethylpyrrolo[1,2-a]pyrazin-6-yl}acetate

A mixture of the title compound of Preparation 104 (130 mg, 0.42 mmol), the title compound of Preparation 4 (0.31 g, 0.78 mmol), potassium phosphate (0.27 g, 1.26 mmol), 2-dicyclohexylphosphino-2',6'-dimethoxybiphenyl (S-Phos) (19 mg, 0.04 mmol) were suspended in 4.5 ml toluene in a Schlenk tube and the mixture was submitted to three vacuum-argon cyles. [Tris(dibenzylideneacetone)]dipalladium(0) (25 mg, 0.03 mmol) was added and the resulting mixture was submitted to three further vacuum-argon cyclesand was stirred at 100 ºC overnight. The mixture was allowed to cool and was filtered through Celite. The Celite was eluted with ethyl acetate and the combined filtrate was evaporated under reduced pressure. The resulting residue was partially purified using the Isolera Purification System (ethyl acetate-hexane gradient, 0:100 rising to 100:0) to give 43 mg of the crude title compound as a brown solid. Purity 76%.

Partial ¹H NMR (300 MHz, CHLOROFORM-d) 68:32 mixture of two rotamers.

Major rotamer δ ppm 4.36 (s, 2 H), 3.92 (s, 2 H).

Minor rotamer δ ppm 4.28 (s, 2 H), 3.94 (s, 2 H).

UPLC/MS (3 min) retention time 1.63 min.

LRMS: m/z 502 (M+1).

### PREPARATION 106

### 3-[(4-Methylphenyl)sulfonyl]pyrrolo[1,2-c]pyrimidine

*p*-Toluenesulfonylmethyl isocyanide (11.3 g, 58 mmol) and 1,8-diazabicycloundec-7-ene (DBU) (8.7 ml, 0.06 mol) were dissolved in 120 ml tetrahydrofuran under nitrogen atmosphere. A solution of 1*H*-pyrrole-2-carbaldehyde (5 g, 0.05 mol) in 200 ml tetrahydrofuran was added and the mixture was stirred at room temperature for 2 h. The mixture was neutralised with glacial acetic acid and evaporated under reduced pressure. The residue triturated with dichloromethane and the solid was collected by filtration, washed with dichloromethane and dried in vacuo to give 10.9 g (40 mmol, 76%) of the title compound as a white solid. Purity 100%.

¹H NMR (300 MHz, CHLOROFORM-*d*) δ ppm 8.73 (s, 1 H), 8.25 (s, 1 H), 7.94 (d, *J*=7.97 Hz, 2 H), 7.50 (d, *J*=2.75 Hz, 1 H), 7.32 (d, *J*=7.97 Hz, 2 H), 7.03 (t, *J*=3.43 Hz, 1 H), 6.83 (d, *J*=3.02 Hz, 1 H), 2.41 (s, 3 H).

UPLC/MS (3 min) retention time 1.56 min.

LRMS: m/z 273 (M+1).

### PREPARATION 107

### Pyrrolo[1,2-c]pyrimidine

Sodium-mercury amalgam (5% sodium content, 3.89 g, 8.26 mmol) and sodium hydrogenphosphate (3.91 g, 27.5 mmol) were suspended in 60 ml anhydrous methanol under argon atmosphere. A solutions of the title compound in Preparation 106 (1.5 g, 5.51 mmol) in 100 ml anhydrous tetrahydrofuran was added drop-wise and the mixture was stirred at room temperature for 2 h. Further sodium-mercury amalgam (5%, 5 g, 10.9 mmol) was added and the mixture stirred for a further 1 h. Additional sodium-mercury amalgam (5%, 5 g, 10.9 mmol) was added and the mixture stirred for at room temperature overnight. The mixture was diluted with water and was extracted three times with ether. The combined organics were dried over anhydrous magnesium sulphate and evaporated under reduced pressure. The resulting residue was purified using the Isolera Purification System (ether-hexane gradient, 0:100 rising to 30:70) to give 0.23 g (1.93 mmol, 35%) of the title compound as a colourless solid that darkens over time. Purity 100%.

¹H NMR (300 MHz, CHLOROFORM-*d*) δ ppm 8.80 (s, 1 H), 7.39 (d, *J*=3.57 Hz, 1 H), 7.38 (s, 1 H), 7.24 (d, *J*=6.59 Hz, 1 H), 6.89 (t, *J*=3.30 Hz, 1 H), 6.45 (d, *J*=3.57 Hz, 1 H).

UPLC/MS (3 min) retention time 0.78 min.

LRMS: m/z 119 (M+1).

### PREPARATION 108

### 7-Bromopyrrolo[1,2-c]pyrimidine

The title compound of Preparation 107 (139 mg, 1.18 mmol) was treated with N-bromosuccinimide (200 mg, 1.12 mmol) according to the method of Preparation 84 to give 197 mg of the crude title compound as a pale yellow solid. Purity 42%.

UPLC/MS (3 min) retention time 1.40 min.

LRMS: m/z 197, 199 (M+1).

### PREPARATION 109

### Ethyl (7-bromopyrrolo[1,2-c]pyrimidin-5-yl)(oxo)acetate

The crude of Preparation 108 (197 mg) was treated with ethyl 2-chloro-2-oxoacetate (0.35 ml, 3.13 mmol) and aluminium trichloride (0.41 g, 3.04 mmol) according to the method of Preparation 102 followed by partial purification using the Isolera Purification System (ether-hexane, 0:100 rising to 75:25) to give 60 mg of the crude title compound as a yellow solid. Purity 78%.

UPLC/MS (3 min) retention time 1.51 min.

LRMS: m/z 297, 299 (M+1).

### PREPARATION 110

### Ethyl (7-bromopyrrolo[1,2-c]pyrimidin-5-yl)acetate

The crude title compound of Preparation 109 (60 mg) was treated with triethylsilane (0.75 ml, 4.71 mmol) and trifluoroacetic acid (0.64 ml) according to the method of Preparation 103 followed by purification using the Isolera Purification System (ether-hexane, 50:50 rising to 85:15) to give 13 mg of ethyl pyrrolo[1,2-c]pyrimidin-5-ylacetate as a colourless oil. This oil was treated with *N*-bromosuccinimide (11 mg, 0.06 mmol) according to the method of Preparation 84 to give 20 mg (0.071 mmol, 6% over three steps) of the title compound as a dark solid. Purity 95%.

UPLC/MS (3 min) retention time 1.57 min.

LRMS: m/z 283, 285 (M+1).

### PREPARATION 111

### Ethyl (7-[2-{[(cyclopropylcarbonyl)(ethyl)amino]methyl}-4-(trifluoromethyl)phenyl]pyrrolo[1,2-c]pyrimidin-5-yl)acetate

The title compound of Preparation 110 (20 mg, 0.071 mmol) was treated with the title compound of Preparation 4 (31 mg, 0.08 mmol), caesium carbonate (2M aqueous solution, 0.11 ml, 0.22 mmol), [1,1'-bis(diphenylphosphino)ferrocene]dichloro-palladium(II), complex with dichloromethane (6 mg, 0.01 mmol) according to the method of Preparation 23 followed by purification using the Isolera Purification System (ether-hexane, 50:50 rising to 100:0) to give 19 mg (0.040 mmol, 58%) of the title compound as a pale yellow solid. Purity 100%.

Partial ¹H NMR (300 MHz, CHLOROFORM-d) 77:23 mixture of two rotamers.

Major rotamer δ ppm 4.50 (s, 2 H), 3.77 (s, 2 H).

Minor rotamer δ ppm 4.55 (s, 2 H), 3.80 (s, 2 H).

UPLC/MS (3 min) retention time 1.92 min.

LRMS: m/z 474 (M+1).

### PREPARATION 112

### 2-Chloro-4-prop-1-yn-1-ylpyrimidine

2,4-Dichloropyrimidine (0.50 g, 3.36 mmol), tributyl(1-propynyl)tin (0.92 ml, 3.02 mmol) and copper(I) iodide (0.08 g, 0.4 mmol) were suspended in 8 ml toluene in a Schlenk tube and the mixture submitted to three vacuum-argon cycles. Tetrakis(triphenylphosphine)palladium (0.27 g, 0.23 mmol) was added, the mixture was submitted to three further vacuum-argon cycles and was stirred at 60 ºC for 1 h. The reaction was allowed to cool and was filtered through Celite, eluting with dichloromethane. The combined filtrate was evaporated under reduced pressure. The resulting residue was purified using the Isolera Purification System (ether-hexane gradient, 0:100 rising to 30:70) to give 0.31 g (2.03 mmol, 68%) of the title compound as a white solid. Purity 100%.

¹H NMR (300 MHz, CHLOROFORM-*d*) δ ppm 8.55 (d, *J*=4.94 Hz, 1 H), 7.24 (d, *J*=4.94 Hz, 1 H), 2.14 (s, 3 H).

UPLC/MS (3 min) retention time 1.13 min.

LRMS: m/z 153,155 (M+1).

### PREPARATION 113

### 1-Chloropyrrolo[1,2-c]pyrimidine

The title compound of Preparation 112 (1.02 g, 6.69 mmol) was treated with copper (I) bromide (0.96 g, 6.69 mmol), dimethylacetamide (84 ml) and triethylamine (12 ml) according to the method of Preparation 83 followed by purification using the Isolera Purification System (ether-hexane gradient, 0:100 rising to 85:15) to give 0.26 g (1.70 mmol, 25%) of the title compound as a pale yellow oil. Purity 91%.

¹H NMR (300 MHz, CHLOROFORM-*d*) δ ppm 7.55 (s, 1 H), 7.29 - 7.35 (m, 1 H), 7.20 - 7.28 (m, 1 H), 6.94 (dd, *J*=6.87, 1.10 Hz, 1 H), 6.60 (d, J=3.85 Hz, 1 H).

UPLC/MS (3 min) retention time 1.42 min.

LRMS: m/z 153,155 (M+1).

### PREPARATION 114

### 1-Methylpyrrolo[1,2-c]pyrimidine

The title compound of Preparation 113 (0.26 g, 1.67 mmol) was treated with 2,4,6-trimethyl-1,3,5,2,4,6-trioxatriborinane (0.26 ml, 1.84 mmol), potassium carbonate (0.69 g, 5.01 mmol) and [1,1'-bis(diphenylphosphino)ferrocene]dichloropalladium(II) complex with dichloromethane (1:1) (0.07 g, 0.08 mmol) according to the method of Preparation 58 followed by purification using the Isolera Purification System (ether-hexane gradient, 0:100 rising to 65:35) to give 0.15 g (1.13 mmol, 67%) of the title compound as a colourless solid. Purity 100%.

¹H NMR (300 MHz, CHLOROFORM-*d*) δ ppm 7.40 (d, *J*=6.59 Hz, 1 H), 7.26 (s, 1 H), 7.20 (d, *J*=6.59 Hz, 1 H), 6.90 (dd, *J*=3.85, 3.02 Hz, 1 H), 6.49 (dd, *J*=3.85, 1.10 Hz, 1 H), 2.73 (s, 3 H).

UPLC/MS (3 min) retention time 0.56 min.

LRMS: m/z 133 (M+1).

### PREPARATION 115

The title compound of Preparation 114 (0.17 g, 1.25 mmol) was treated with *N-*bromosuccinimide (0.21 g, 1.19 mmol) according to the method of Preparation 84 followed by purification using the Isolera Purification System (ether-hexane gradient, 0:100 rising to 70:30) to give 93 mg of the crude title compound as a dark solid. Purity 50%.

HPLC/MS (15 min) retention time 5.87 min.

LRMS: m/z 211, 213 (M+1).

### PREPARATION116

### Ethyl (7-bromo-1-methylpyrrolo[1,2-c]pyrimidin-5-yl)(oxo)acetate

The crude title compound of Preparation 115 (93 mg) was treated with ethyl 2-chloro-2-oxoacetate (0.35 ml, 3.13 mmol) and aluminium trichloride (0.41 g, 3.08 mmol) according to the method of Preparation 102 followed by purification using the Isolera Purification System (ether-hexane, 0:100 rising to 100:0) to give 45 mg (0.145 mmol, 12% over two steps) of the title compound as a yellow solid. Purity 100%.

¹H NMR (300 MHz, CHLOROFORM-*d*) δ ppm 8.33 (d, *J*=6.32 Hz, 1 H), 7.87 (d, *J*=6.04 Hz, 1 H), 7.68 (s, 1 H), 4.43 (q, *J*=7.32 Hz, 2 H), 3.29 (s, 3 H), 1.44 (t, *J*=7.14 Hz, 3 H).

HPLC/MS (15 min) retention time 7.07 min.

LRMS: m/z 311, 313 (M+1).

### PREPARATION 117

### Ethyl {7-[2-{[(cyclopropylcarbonyl)(ethyl)amino]methyl}-4-(trifluoromethyl)phenyl]-1-methylpyrrolo[1,2-c]pyrimidin-5-yl}(oxo)acetate

The title compound of Preparation 116 (45 mg, 0.14 mmol) was treated with the title compound of Preparation 4 (115 mg, 0.29 mmol), potassium phosphate (92 mg, 0.43 mmol), 2-dicyclohexylphosphino-2',6'-dimethoxybiphenyl (S-Phos) (6 mg, 0.01 mmol) and [tris(dibenzylideneacetone)]dipalladium(0) (9 mg, 0.01 mmol) according to the method of Preparation 47 followed by partial purification using the Isolera Purification System (ether-hexane gradient, 0:100 rising to 100:0) to give 54 mg of the crude title compound as a brown solid. Purity 51%.

HPLC/MS (15 min) retention time 8.52 min.

LRMS: m/z 502 (M+1).

### PREPARATION 118

### Ethyl {7-[2-][(cyclopropylcarbonyl)(ethyl)amino]methyl}-4-(trifluoromethyl)phenyl]-1-methylpyrrolo[1,2-c]pyrimidin-5-yl}acetate

The crude of Preparation 117 (54 mg) was treated with trimethylsilane (0.30 ml, 1.88 mmol) and trifluoroacetic acid (0.5 ml) according to the method of Preparation 103 followed by purification using the Isolera Purification System (ether-hexane, 50:50 rising to 100:0) to give 5 mg (0.010 mmol, 7% over two steps) of the title compound as a white solid. Purity 100%.

Partial ¹H NMR (300 MHz, CHLOROFORM-*d*) δ ppm 4.60 (d, *J*=16.21 Hz, 1 H), 3.99 (d, *J*=16.21 Hz, 1 H), 3.76 (s, 2 H).

HPLC/MS (15 min) retention time 8.53 min.

LRMS: m/z 488 (M+1).

### EXAMPLES

### EXAMPLE 1

### {3-[2-{[(Cyclopropylcarbonyl)(ethyl)amino]methyl}-4-(trifluoromethyl)phenyl]-1H-pyrrolo[3,2-b]pyridin-1-yl}acetic acid

The title compound of Preparation 16 (230 mg, 0.81 mmol) and the title compound of Preparation 4 (320 mg, 0.81 mmol) were treated with aqueous caesium carbonate solution (2M, 0.81 mL, 1.62 mmol) and [1,1'-bis(diphenylphosphino)-ferrocene]dichloropalladium(II), complex with dichloromethane (66 mg, 0.08 mmol) according to the method of Preparation 23. The resulting residue was partitioned between ethyl acetate and 2N sodium hydroxide and the aqueous layer was washed twice with ethyl acetate. The aqueous phase was adjusted to pH 5 with 0.5 N hydrochloric acid and was extracted three times with ethyl acetate. The combined organics were washed with brine, dried over anhydrous magnesium sulphate, filtered and evaporated under reduced pressure. The resulting residue was purified using the Isolera Purification System (ethanol-dichloromethane gradient, 0:100 rising to 80:20) to give 51 mg (0.11 mmol, 14%) of the title compound as a light brown solid. Purity 95%. Partial ¹H NMR (300 MHz, CHLOROFORM-d) 55:45 mixture of two rotamers.

Major rotamer δ ppm 7.67 (1 H, d, *J*=8.0 Hz), 7.32 (1 H, br. s.).

Minor rotamer δ ppm 7.73 (1 H, d, *J*=7.7 Hz), 7.43 (1 H, br. s.).

HPLC/MS (30 min) retention time 12.07 min.

LRMS: m/z 446 (M+1).

### EXAMPLE 2

### [3-(2-{[(Cyclopropylcarbonyl)(ethyl)amino]methyl}-4-fluorophenyl)-1H-pyrrolo[3,2-b]pyridin-1-yl]acetic acid

The title compound of Preparations 16 (80 mg, 0.28 mmol) and the title compound of Preparation 7 (98 mg, 0.28 mmol) were treated with 2M caesium carbonate solution (0.28 mL, 0.56 mmol) and [1,1'-bis(diphenylphosphino)ferrocene]-dichloropalladium(II), complex with dichloromethane (0.23, 0.03 mmol) according to the method of Preparation 23. The resulting residue was partitioned between ethyl acetate and 2N sodium hydroxide and the aqueous layer was washed twice with ethyl acetate. The aqueous phase was adjusted to pH 5 with 0.5 N hydrochloric acid and was extracted three times with ethyl acetate. The combined organics were washed with brine, dried over anhydrous magnesium sulphate, filtered and evaporated under reduced pressure. The resulting residue was purified using the Isolera Purification System (ethanol-dichloromethane gradient, 0:100 rising to 100:0) to give 15 mg (0.038 mmol, 13%) of the title compound as a light brown solid. Purity 95%.

Partial ¹H NMR (300 MHz, CHLOROFORM-d) 50:50 mixture of two rotamers.

Rotamer 1 δ ppm 6.91 (1 H, dd, *J*=10.4, 2.5 Hz), 0.92 (3 H, t, *J*=6.9 Hz). Rotamer 2 δ ppm 6.81 (1 H, dd, *J*=10.4, 2.5 Hz), 1.02 (3 H, t, *J*=6.9 Hz).

HPLC/MS (30 min) retention time 8.98 min.

LRMS: m/z 396 (M+1).

### EXAMPLE 3

### {3-[2-{[(Cyclopropylcarbonyl)(ethyl)amino]methyl}-4-(trifluoromethyl)phenyl]-5-methyl-1H-pyrrolo[3,2-b]pyridin-1-yl}acetic acid

The crude title compound of Preparation 23 (110 mg) was dissolved in 3 ml tetrahydrofuran and 1 ml water. Lithium hydroxide monohydrate (15 mg, 0.36 mmol) was added and the mixture was stirred at room temperature for 1 h. The organic solvent was evaporated under reduced pressure and water was added. The aqueous was adjusted to pH 5 with 1 N hydrochloric acid and was extracted with chloroform. The organics were washed with water, brine, dried over magnesium sulfate, filtered and evaporated under reduced pressure. The resulting residue was purified using the Isolera Purification System (ethanol-methanol-dichloromethane gradient, 0:0:100 rising to 0:5:95 and then rising to 100:0:0) to give 15 mg (0.033 mmol, 10% over two steps) of the title compound as a white solid. Purity 87%.

Partial ¹H NMR (300 MHz, DMSO-d) 55:45 mixture of two rotamers.

Major rotamer δ ppm 5.11 (s, 2 H).

Minor rotamer δ ppm 4.81 (s, 2 H).

UPLC/MS (30 min) retention time 12.05 min.

LRMS: m/z 460 (M+1).

### EXAMPLE 4.

### 2-{3-[2-{[(Cyclopropylcarbonyl)(ethyl)amino]methyl}-4-(trifluoromethyl)phenyl]-1H-pyrrolo[3,2-b]pyridin-1-yl}propanoic acid

The crude title compound of Preparation 25 (38 mg) was treated with lithium hydroxide monohydrate (14 mg, 0.33 mmol) according to the method of Example 3 to give 35 mg (0.076 mmol, 48% over two steps) of the title compound as a solid. Purity 93%.

Partial ¹H NMR (300 MHz, CHLOROFORM-d) 55:45 mixture of two rotamers.

Major rotamer δ ppm 1.01 (3 H, t, *J*=7.0 Hz).

Minor rotamer δ ppm 0.87 (3 H, t, *J*=7.1 Hz).

HPLC/MS (30 min) retention time 12.22 min.

LRMS: m/z 460 (M+1).

### EXAMPLE 5

### {5-Cyclopropyl-3-[2-{[(cyclopropylcarbonyl)(ethyl)amino]methyl}-4-(trifluoromethyl)phenyl]-1H-pyrrolo[3,2-b]pyridin-1-yl}acetic acid

The title compound of Preparation 29 (100 mg, 0.19 mmol) was treated with lithium hydroxide monohydrate (23 mg, 0.55 mmol) according to the method of Example 3 to give 75 mg (0.15 mmol, 79%) of the title compound as a white solid. Purity 96%.

Partial ¹H NMR (300 MHz, DMSO-d) 54:46 mixture of two rotamers.

Major rotamer δ ppm 7.40 (s, 1 H), 5.00 (s, 2 H).

Minor rotamer δ ppm 7.29 (s, 1 H), 4.73 (s, 2 H).

HPLC/MS (30 min) retention time 13.63 min.

LRMS: m/z 486 (M+1).

### EXAMPLE 6

### 2-{3-[2-{[(Cyclopropylcarbonyl)(ethyl)amino]methyl}-4-(trifluoromethyl)phenyl]-5-methyl-1H-pyrrolo[3,2-b]pyridin-1-yl}propanoic acid

The title compound of Preparation 31 (53 mg, 0.11 mmol) was treated with Lithium hydroxide monohydrate (18 mg, 0.43 mmol) according to the method of Example 3. The resulting residue was purified using the Isolera Purification System (ethanol-dichloromethane gradient, 0:100 rising to 100:0) to give 18 mg (0.038 mmol, 36%) of the title compound as a white solid. Purity 100%.

Partial ¹H NMR (300 MHz, CHLOROFORM-d) 50:50 mixture of two rotamers.

Rotamer 1 δ ppm 7.41 (2, 1 H), 5.02 (s, 2 H)

Rotamer 2 δ ppm 7.31 (s, 1 H), 4.77 (s, 2 H).

HPLC/MS (30 min) retention time 12.65 min.

LRMS: m/z 474 (M+1).

### EXAMPLE 7

### [3-(2-{[(Cyclopropylcarbonyl)(ethyl)amino]methyl}-4-fluorophenyl)-5-(trifluoromethyl)-1H-pyrrolo[3,2-b]pyridin-1-yl]acetic acid

The title compound of Preparation 38 (40 mg, 0.081 mmol) was treated with lithium hydroxide monohydrate (10 mg, 0.23 mmol) according to the method of Example 3 to give 32 mg (0.069 mmol, 85%) of the title compound as a white solid. Purity 98%.

Partial ¹H NMR NMR (400 MHz, DMSO-*d*₆) 50:50 mixture of two rotamers. Rotamer 1 δ ppm 6.92 (1 H, dd, *J*=10.4, 2.4 Hz), 0.90 (3 H, t, *J*=7.0 Hz). Rotamer 2 δ ppm 6.84 (1 H, dd, *J*=10.6, 2.3 Hz), 1.01 (3 H, t, *J*=7.0 Hz).

HPLC/MS (30 min) retention time 16.98 min.

LRMS: m/z 464 (M+1).

### EXAMPLE 8

### {3-[2-{[[(Benzylamino)carbonyl](ethyl)amino]methyl}-4-(trifluoromethyl)phenyl]-5-methyl-1H-pyrrolo[3,2-b]pyridin-1-yl}acetic acid

The title compound of Preparation 22 (75 mg, 0.24 mmol) was treated with the title compound of Preparation 9 (178 mg, 0.38 mmol), aqueous caesium carbonate solution (2M, 0.4 ml, 0.80 mmol) and [1,1'-bis(diphenylphosphino)ferrocene]dichloro-palladium(II) complex with dichloromethane (16 mg, 0.02 mmol) according to the method of Preparation 23 followed by reverse-phase chromatography using the Isolera Purification System (methanol-water, 0:100 rising to 100:0) to give 13 mg (0.025 mmol, 10%) of the title compound as a white solid. Purity 87%.

Partial ¹H NMR (300 MHz, METHANOL-*d*₄) δ ppm 4.64 (s, 2 H), 4.37 (s, 2 H), 2.53 (s, 3 H), 1.03 (t, *J*=7.04 Hz, 3 H).

UPLC/MS (5 min) retention time 2.53 min.

LRMS: m/z 525 (M+1).

### EXAMPLE 9

### {3-[2-{[Benzyl(cyclopropylcarbonyl)amino]methyl}-4-(trifluoromethyl)phenyl]-5-methyl-1H-pyrrolo[3,2-b]pyridin-1-yl}acetic acid

The title compound of Preparation 22 (70 mg, 0.23 mmol) was treated with the crude title compound of Preparation 15 (165 mg), aqueous caesium carbonate solution (2M, 0.34 ml, 0.68 mmol) and [1,1'-bis(diphenylphosphino)ferrocene]dichloro-palladium(II), complex with dichloromethane (14 mg, 0.02 mmol) according to the method of Preparation 23 followed by purification using the Isolera Purification System (ethanol-dichloromethane, 0:100 rising to 100:0) to give 11 mg (0.021 mmol, 9%) of the title compound as a grey solid. Purity 94%.

Partial ¹H NMR (300 MHz, CHLOROFORM-*d*) δ ppm 5.80 - 5.94 (1 H, m), 5.65 - 5.80 (1 H, m), 5.56 (1 H, d, *J*=17.0 Hz), 5.04 (1 H, d, *J*=17.6 Hz), 4.63 (1 H, d, *J=*17.6 Hz), 4.07 (1 H, d, *J=*16.4 Hz).

UPLC/MS (5 min) retention time 2.56 min.

LRMS: m/z 522 (M+1).

### EXAMPLES 10

### {3-[2-{[(Cyclopropylcarbonyl)(ethyl)amino]methyl}-4-(trifluoromethyl)phenyl]-1H-pyrrolo[3,2-c]pyridin-1-yl}acetic acid

The title compound of Preparation 40 (100 mg, 0.35 mmol) was treated with the title compound of Preparation 4 (140 mg, 0.35 mmol), aqueous caesium carbonate solution (2M, 0.53 ml, 1.06 mmol) and [1,1'-bis(diphenylphosphino)ferrocene]dichloro-palladium (II) complex with dichloromethane (31 mg, 0.04 mmol) according to the method of Preparation 23 followed by purification using the Isolera Purification System (ethanol-dichloromethane, 0:100 rising to 100:0) to give 109 mg (0.24 mmol, 70%) of the title compound as a grey solid. Purity 98%.

Partial ¹H NMR (300 MHz, DMSO-d) 60:40 mixture of two rotamers.

Major rotamer δ ppm 8.75 (s, 1 H), 4.67 (s, 2 H).

Minor rotamer δ ppm 8.79 (s, 1 H), 4.91 (s, 2 H).

HPLC/MS (30 min) retention time 11.10 min.

LRMS: m/z 446 (M+1).

### EXAMPLE 11

### {3-[2-{[(Cyclopropylcarbonyl)(ethyl)amino]methyl}-4-(trifluoromethyl)phenyl]-2,4-dimethyl-1H-pyrrolo[3,2-c]pyridin-1-yl}acetic acid

The crude title compound of Preparation 48 (60 mg) was dissolved in 1 ml tetrahydrofuran and 1 ml water. Lithium hydroxide monohydrate (11 mg, 0.26 mmol) was added and the mixture was agitated at room temperature for 2.5 h. The organic solvent was evaporated under reduced pressure and the aqueous solution was acidified to pH 6.5 with acetic acid. The mixture was purified directly by Preparative HPLC-MS to give 9 mg (0.019 mmol. 9% over two steps) of the title compound as a white solid. Purity 97%.

Partial ¹H NMR (600 MHz, DMSO-*d*₆) 70:30 mixture of two rotamers.

Major rotamer δ ppm 7.68 (1 H, d, *J*=8.2 Hz), 7.47 (1 H, d, *J*=7.6 Hz), 0.90 (3 H, t, *J*=6.7 Hz).

Minor rotamer δ ppm 7.76 (1 H, d, *J*=7.6 Hz), 7.53 (1 H, d, *J*=7.6 Hz), 0.78 (3 H, t, *J*=6.7 Hz).

UPLC/MS (5 min) retention time 2.22 min.

LRMS: m/z 474 (M+1).

### EXAMPLE 12

### {1-[2-{[(Cyclopropylcarbonyl)(ethyl)amino]methyl}-4-(trifluoromethyl)phenyl]-1H-pyrrolo[2,3-b]pyridin-3-yl}acetic acid

The title compound of Preparation 51 (71 mg, 0.14 mmol) was dissolved in 1.2 ml of chloroform. Trifluoroacetic acid (1.0 ml, 13 mmol) was added and the mixture was stirred at room temperature for 2.5 h. the mixture was evaporated under reduced pressure and the residue was re-suspended in 4% w/v sodium bicarbonate solution. The pH was adjusted to 5 and the aqueous was extracted three times with ethyl acetate. The combined organics were washed with brine, dried over anhydrous magnesium sulphate, filtered and evaporated under reduced pressure to give 61 mg (0.13 mmol, 92%) of the title compound as a white solid. Purity 95%.

Partial ¹H NMR (300 MHz, CHLOROFORM-d) mixture of two rotamers. Rotamer 1 δ ppm 4.51 (1 H, s).

Rotamer 2 δ ppm 4.58 (1 H, br. s).

HPLC/MS (30 min) retention time 15.26 min.

LRMS: m/z 446 (M+1).

### EXAMPLE 13

### [1-(2-{[(Cyclopropylcarbonyl)(ethyl)amino]methyl}-4-fluorophenyl)-1H-pyrrolo[2,3-b]pyridin-3-yl]acetic acid

The title compound of Preparation 54 (45 mg, 0.10 mmol)) dissolved in 1 ml chloroform. Trifluoroacetic acid (0.79 mL, 10.3 mmol) was added and the mixture was stirred for 2.5 h. The mixture was evaporated under reduced pressure and the residue resuspended in 2 N sodium hydroxide. The aqueous was washed twice with ethyl acetate and was adjusted to pH 5 with 0.5 N hydrochloric acid. The aqueous phase was extracted three times with ehyl acetate, the combined organics were washed with brine, dried over anhydrous magnesium sulphate, filtered and evaporated under reduced pressure to give 30 mg (0.076 mmol, 76%) of the title compound as a white solid. Purity 95%.

Partial ¹H NMR (300 MHz, CHLOROFORM-d) 65:35 mixture of two rotamers.

Major rotamer δ ppm 7.98 (1 H, d, *J*=8.0 Hz).

Minor rotamer δ ppm 8.04 (1 H, d, *J*=8.0 Hz).

HPLC/MS (30 min) retention time 13.27 min.

LRMS: m/z 396 (M+1).

### EXAMPLE 14

### {1-[2-{[(Cyclopropylcarbonyl)(ethyl)amino]methyl}-4-(trifluoromethyl)phenyl]-6-methyl-1H-pyrrolo[2,3-b]pyridin-3-yl}acetic acid

The title compound of Preparation 61 (0.49 g, 0.95 mmol) was treated with trifluoroacetic acid according to the method of Example 12. The reaction mixture was evaporated to dryness and the residue was purified using the Isolera Purification System (methanol-dichloromethane gradient, 0:100 rising to 5:95) to give 0.26 g (0.57 mmol, 59%) of the title compound as a light brown solid. Purity 99%.

Partial ¹H NMR (300 MHz, CHLOROFORM-d) 57:43 mixture of two rotamers.

Major rotamer δ ppm 3.81 (s, 2 H), 2.52 (s, 3 H).

Minor rotamer δ ppm 3.83 (s, 2 H), 2.55 (s, 3 H).

HPLC/MS (30 min) retention time 16.37 min.

LRMS: m/z 460 (M+1).

### EXAMPLE 15

### {6-Cyclopropyl-1-[2-{[(cyclopropylcarbonyl)(ethyl)amino]methyl}-4-(trifluoromethyl)phenyl]-1H-pyrrolo[2,3-b]pyridin-3-yl}acetic acid

The title compound of Preparation 65 (42 mg, 0.077 mmol) was treated with trifluoroacetic acid according to the method of Example 12. The reaction mixture was evaporated to dryness and the residue was purified using the Isolera Purification System (methanol-dichloromethane gradient, 0:100 rising to 5:95) to give 16 mg (0.033 mmol, 43%) of the title compound as a light brown solid. Purity 97%.

Partial ¹H NMR (300 MHz, CHLOROFORM-d) 65:35 mixture of two rotamers.

Major rotamer δ ppm 7.82 (1 H, d, *J*=7.4 Hz), 4.56 (s., 2 H).

Minor rotamer δ ppm 7.88 (1 H, d, *J*=8.0 Hz), 4.62 (br. s., 2 H).

HPLC/MS (30 min) retention time 18.03 min.

LRMS: m/z 486 (M+1).

### EXAMPLE 16

### [1-(2-{[(Cyclopropylcarbonyl)(ethyl)amino]methyl}-4-fluorophenyl)-6-methyl-1H-pyrrolo[2,3-b]pyridin-3-yl]acetic acid

The title compound of Preparation 68 (54 mg, 0.12 mmol) was treated with trifluoroacetic acid according to the method of Example 12 to give 34 mg (0.083 mmol, 70%) of the title compound as a white solid. Purity 97%.

Partial ¹H NMR (300 MHz, CHLOROFORM-d) 60:40 mixture of two rotamers.

Major rotamer δ ppm 7.85 (1 H, d, *J*=8.2 Hz), 3.39 (q, *J*=7.0 Hz, 2 H), 2.52 (s, 3 H).

Minor rotamer δ ppm 7.90 (1 H, d, *J*=7.6 Hz), 3.49 (q, *J*=7.0 Hz, 2 H), 2.54 (s, 3 H).

UPLC/MS (5 min) retention time 2.55 min.

LRMS: m/z 410 (M+1).

### EXAMPLE17

### [1-(2-{[(Cyclopropylcarbonyl)(ethyl)amino]methyl}phenyl)-6-methyl-1H-pyrrolo[2,3-b]pyridin-3-yl]acetic acid

The crude title compound of Preparation 71 (68 mg) was treated with trifluoroacetic acid according to the method of Example 12 to give 36 mg (0.092 mmol, 26% over three steps) of the title compound as a white solid. Purity 97%.

Partial ¹H NMR (300 MHz, CHLOROFORM-d) 50:50 mixture of two rotamers.

Rotamer 1 δ ppm 7.90 (1 H, d, *J*=8.2 Hz), 3.83 (s, 2 H), 2.51 (s, 3 H). Rotamer 2 δ ppm 7.83 (1 H, d, *J*=7.6 Hz), 3.84 (s, 2 H), 2.53 (s, 3 H).

UPLC/MS (5 min) retention time 2.46 min.

LRMS: m/z 392 (M+1).

### EXAMPLE18

### {1-[2-{[(Cyclopropylcarbonyl)(ethyl)amino]methyl}-4-(trifluoromethyl)phenyl]-6-methoxy-1H-pyrrolo[2,3-b]pyridin-3-yl}lacetic acid

The crude title compound of Preparation 74 (183 mg) was suspended in 0.8 ml water. Hydrochloric acid (4M in dioxane, 8 ml, 32 mmol) was added and the resultant solution was stirred at room temperature for 2 h. The mixture was diluted with 25 ml water and the pH was adjusted to 7-8 by careful addition of solid sodium hydrogen carbonate. The resultant suspension was extracted with ethyl acetate and the organic phase was washed with brine, dried over magnesium sulfate and evaporated under reduced pressure to give an oil. The residue was re-evaporated from ether to give 104 mg of the title compound (0.22 mmol, 29% over three steps) as a white foam. Purity 97%.

Partial ¹H NMR (300 MHz, CHLOROFORM-d) 60:40 mixture of two rotamers.

Major rotamer δ ppm 4.57 (s, 2H), 3.79 (s, 2H).

Minor rotamer δ ppm 4.67 (s, 2H), 3.75 (s, 2H).

UPLC/MS (5 min) retention time 3.08 min.

LRMS: m/z 476 (M+1).

### EXAMPLE 19

### 2-(1-(2-((N-ethylcyclopropanecarboxamido)methyl)-4-(trifluoromethyl)phenyl)-6-(trifluoromethyl)-1H-pyrrolo[2,3-b]pyridin-3-yl)acetic acid

The title compound of Preparation 80 (55 mg, 0.097 mmol) suspended in water. Hydrochloric acid (4M in dioxane, 2 ml, 8 mmol) was added and the mixture was stirred at room temperature for 3 h. Further hydrochloric acid (4M in dioxanem 2 ml, 8 mmol) was added and the mixture was stirred for a further 3 h. The mixture was then diluted with 15 ml water and the pH was adjusted to 7-8 by careful addition of solid sodium hydrogen carbonate. The resultant suspension was extracted with ethyl acetate and the organic extract was washed with brine, dried over magnesium sulphate, filtered and evaporated under reduced pressure to give 45 mg (0.087 mmol, 91%) of the title compound as a white solid. Purity 100%.

Partial ¹H NMR (300 MHz, DMSO-d) 50:50 mixture of two rotamers.

Rotamer 1 δ ppm 4.64 (s, 2 H).

Rotamer 2 δ ppm 4.33 (s, 2 H).

UPLC/MS (3 min) retention time 1.97 mm.

LRMS: m/z 514 (M+1).

### EXAMPLE 20

### {8-[2-{[(Cyclopropylcarbonyl)(ethyl)amino]methyl}-4-(trifluoromethyl)phenyl]pyrrolo[1,2-a]pyrimidin-6-yl}acetic acid

The title compound of Preparation 85 (57 mg, 0.12 mmol) was treated with lithium hydroxide monohydrate (21 mg, 0.5 mmol) according to the method of Example 3 to give 30 mg (0.067 mmol, 54%) of the title compound as a yellow solid. Purity 99%.

Partial ¹H NMR (300 MHz, DMSO-d) 54:46 mixture of two rotamers.

Major rotamer δ ppm 7.36 (s, 1 H), 4.70 (s, 2 H).

Minor rotamer δ ppm 7.47 (s, 1 H), 4.96 (s, 2 H).

HPLC/MS (30 min) retention time 15.89 min.

LRMS: m/z 446 (M+1).

### EXAMPLE 21

### [8-(2-{[(Cyclopropylcarbonyl)(ethyl)amino]methyl}-4-fluorophenyl)pyrrolo[1,2-a]pyrimidin-6-yl]acetic acid

The title compound of Preparation 86 (60 mg, 0.15 mmol) was treated with Lithium hydroxide monohydrate (25 mg, 0.6 mmol) according to the method of Example 3 to give 33 mg (0.084 mmol, 57%) of the title compound as a yellow solid. Purity 99%.

Partial ¹H NMR (300 MHz, DMSO-d) 50:50 mixture of two rotamers.

Rotamer 1 δ ppm 4.12 (s, 2 H).

Rotamer 2 δ ppm 3.95 (s, 2 H).

HPLC/MS (30 min) retention time 13.96 min.

LRMS: m/z 396 (M+1).

### EXAMPLE 22

### [8-[2-{[(Cyclopropylcarbonyl)(ethyl)amino]methyl}-4-(trifluoromethyl)phenyl]-2-(trifluoromethyl)pyrrolo[1,2-a]pyrimidin-6-yl]acetic acid

The title compound of Preparation 90 (65 mg, 0.12 mmol) was treated with lithium hydroxide monohydrate (21 mg, 0.05 mmol) according to the method of Example 3 to give 42 mg (0.082 mmol, 66%) of the title compound as a yellow solid. Purity 94%.

Partial ¹H NMR (300 MHz, DMSO-d) 44:56 mixture of two rotamers.

Major rotamer δ ppm 4.61 (s, 2 H), 0.99 (3 H, t, *J*=6.7 Hz).

Minor rotamer δ ppm 4.87 (s, 2 H), 0.89 (3 H, t, *J=7.0* Hz).

UPLC/MS (5 min) retention time 3.21 min.

LRMS: m/z 514 (M+1).

### EXAMPLE 23

### {8-[2-{[(Cyclopropylcarbonyl)(ethyl)amino]methyl}-4-(trifluoromethyl)phenyl]-2-methylpyrrolo[1,2-a]pyrimidin-6-yl}acetic acid

The title compound of Preparation 94 (60 mg, 0.13 mmol) was treated with lithium hydroxide monohydrate (16 mg, 0.5 mmol) according to the method of Example 3 to give 30 mg (0.065 mmol, 48%) of the title compound as a yellow solid. Purity 92%.

Partial ¹H NMR (300 MHz, CHLOROFORM-d) 50:50 mixture of two rotamers.

Rotamer 1 δ ppm 7.39 (s, 1 H), 4.95 (s, 2 H).

Rotamer 2 δ ppm 7.30 (s, 1 H), 4.67 (s, 2 H).

UPLC/MS (5 min) retention time 3.03 min.

LRMS: m/z 460 (M+1).

### EXAMPLE 24

### {3-[2-{[(Cyclopropylcarbonyl)(ethyl)amino]methyl}-4-(trifluoromethyl)phenyl]imidazo[1,5-a]pyridin-1-yl}acetic acid

The title compound of Preparation 100 (55 mg, 0.12 mmol) was dissolved in 1.5 ml tetrahydrofuran and 1.5 ml water. Lithium hydroxide monohydrate (20 mg, 0.48 mmol) was added and the mixture was agitated at room temperature for 1 h. The organic solvent was evaporated under reduced pressure and the aqueous solution was acidified to pH 4 with acetic acid. The mixture was agitated for 90 min and was extracted with ethyl acetate. The organics were washed sequentially with water and brine, dried over sodium sulphate, filtered and evaporated to give 40 mg (0.090 mmol, 77%) of the title compound as a beige solid. Purity 91%.

Partial ¹H NMR (300 MHz, CHLOROFORM-d) 83:17 mixture of two rotamers.

Major rotamer δ ppm 4.57 (s, 2 H).

Minor rotamer δ ppm 4.80 (s, 2 H).

HPLC/MS (30 min) retention time 14.51 min.

LRMS: m/z 446 (M+1).

### EXAMPLE 25

### {8-[2-{[(Cyclopropylcarbonyl)(ethyl)amino]methyl}-4-(trifluoromethyl)phenyl]-1,7-dimethylpyrrolo[1,2-a]pyrazin-6-yl}acetic acid

The crude title compound of Preparation 105 (42 mg) was treated with Lithium hydroxide monohydrate (14 mg, 0.33 mmol) according to the method of Example 3 to give 33 mg of the crude title compound as a yellow solid. The crude product was repurified by Preparative HPLC to give 8 mg (0.017 mmol, 4% over two steps) of the title compound. Purity 96%.

Partial ¹H NMR (300 MHz, CHLOROFORM-*d*) 75:25 mixture of rotamers:
Major Rotamer δ ppm 2.24 (s, 3 H), 2.09 (s, 3 H).
Minor Rotamer δ ppm 2.16 (s, 3 H), 2.07 (s, 3 H).

HPLC/MS (30 min) retention time 11.20 min.

LRMS: m/z 474 (M+1).

### EXAMPLE 26

### {7-[2-{[(Cyclopropylcarbonyl)(ethyl)amino]methyl}-4-(trifluoromethyl)phenyl]pyrrolo[1,2-c]pyrimidin-5-yl}acetic acid

The title compound of Preparation 111 (19 mg, 0.040 mmol) was treated with lithium hydroxide monohydrate (7 mg, 0.17 mmol) according to the method of Example 3 to give 14 mg (0.031 mmol, 67%) of the title compound as a beige solid. Purity 94%.

Major Rotamer ¹H NMR (300 MHz, CHLOROFORM-*d*) δ ppm 8.44 (s, 1 H), 7.67 (d, *J*=7.42 Hz, 1 H), 7.61 (s, 1 H), 7.45 - 7.56 (m, 2 H), 7.30 (d, J=6.59 Hz, 1 H), 6.88 (s, 1 H), 4.55 (br. s., 2 H), 3.86 (s, 2 H), 3.32 (q, *J*=6.96 Hz, 2 H), 1.53 - 1.69 (m, 1 H), 1.06 (t, *J*=7.00 Hz, 3 H), 0.81 - 0.92 (m, 2 H), 0.65 - 0.78 (m, 2 H).

HPLC/MS (30 min) retention time 14.97 min.

LRMS: m/z 446 (M+1).

### EXAMPLE 27

### {7-[2-{[(Cyclopropylcarbonyl)(ethyl)amino]methyl}-4-(trifluoromethyl)phenyl]-1-methylpyrrolo[1,2-c]pyrimidin-5-yl}acetic acid

The title compound of Preparation 118 (5 mg, 0.010 mmol) was treated with Lithium hydroxide monohydrate (5 mg, 0.12 mmol) according to the method of Example 3 to give 4.8 mg (0.010 mmol, 98%) of the title compound as a white solid. Purity 95%.

Partial ¹H NMR (300 MHz, CHLOROFORM-d) δ ppm 4.74 (dd, *J*=16.21, 4.12 Hz, 1 H), 3.96 (dd, *J*=15.66, 4.67 Hz, 1 H), 3.82 (br. s., 2 H).

HPLC/MS (30 min) retention time 14.64 min.

LRMS: m/z 460 (M+1).

### PHARMACOLOGICAL ACTIVITY

### CRTh2 radioligand bending assay

The assay is performed using membranes obtained from CHO.K1 cells stably overexpressing the CRTh2 receptor and ³[H]PGD₂ as radioligand. The assay is performed incubating 2-4 µg of membranes per well with the compound to be tested and 5 nM ³[H]PGD₂ in incubation buffer (50 mM HEPES pH 7.0, 10 mM MgCl₂, 1 mM EDTA, 0.2% BSA) for 2 h at room temperature. The reaction is terminated by filtering in GF/C plates pre-treated with 0.3% de polyetilenimine buffer and washing 3 times with wash buffer (50 mM HEPES pH 7.4, 0.5 M NaCl). After washing, plates are dried and scintillation cocktail Optiphase Hisafe II added. The radioactivity retained in the filter is counted using a Microbeta liquid scintillation counter. Compound IC₅₀ are determined using Excel XL-fit for calculations.

### CRTh2 GTPγS antagonism binding assay

The assay is performed using membranes obtained from CHO.K1 cells stably overexpressing the CRTh2 receptor and [³⁵S]-GTPγS as radioligand. Assay is performed pre-incubating 4-8 µg of membranes per well with the compound to be tested for 1 h, followed by incubation with PGD₂ at 50 nM (EC₈₀), 0.1 nM [³⁵S]-GTPγS in incubation buffer (20 mM HEPES, 10 mM MgCl₂, 100 mM NaCl, 10 µM GDP, 10 µg/ml Saponine and 0.2% BSA) for 2 h at room temperature. The reaction is terminated by filtering in GF/C plates pre-treated with 20 mM HEPES, 10 mM MgCl₂, 100 mM NaCl and washing 6 times with wash buffer (20 mM NaH₂PO4, 20 mM Na₂HPO4). After washing, plates are dried and scintillation buffer Optiphase added. The radioactivity retained in the filter is counted using a Microbeta liquid scintillation counter. Compound IC₅₀ are determined using Excel XL-fit for calculations.

### CRTh2 Isolated cell eosinophil cell shape assay

Citrated whole blood is obtained from consenting donors and polymorphonuclear leukocytes obtained by gradient centrifugation on Polymorphprep for 40 min at 500G brake off. PMN fraction is resuspended in calcium free buffer (10 mM HEPES, 10 mM glucose, 0.1 % BSA in calcium free PBS) and centrifuged for 10 min at 400G at room temperature. Lysis of red blood cells is performed by adding 20 ml of 0.2% saline buffer (0.2% NaCl) for 40 s and stopped by adding 20 ml of 1.6% saline buffer (1.6% NaCl). For compound treatment 90 µl of PMNs cells resuspended at a density of 5 x 10⁶ cells/ml in 10 mM HEPES, 10 mM glucose, 0.1 % BSA in calcium containing PBS are incubated for 10 min with 10 µl of compound to be tested followed by incubation with 10µl of 500 nM PGD₂ for exactly 4 min at 37°C. Fixation is performed by addition of 0.2 ml of ice-cold 1:5 diluted Cellfix (BD Biosciences) and immediately analysed on a FACSCalibur (BD Biosciences). Eosinophils are gated on the basis of autofluorescence in the FL2 channels and shape change of 600 cells is assayed by forward scatter and side scatter analysis. Compound IC₅₀ are determined using Excel XL-fit for calculations.

### CRTh2 whole blood eosinophil cell shape assay

Whole blood is obtained from consenting donors mixed with heparin and kept in rotation until use. For compound treatment, 90 µl of blood are mixed with 10 µl of compound to be tested and incubated for 10 min at room temperature followed by addition of 10 µl of 500 nM PGD₂ for exactly 4 min at 37°C. Reaction was stopped by placing the tubes on ice and adding of 0.25 ml of ice-cold 1:5 diluted Cellfix (BD Biosciences). Red blood cells are lysed in two steps by adding 2 ml of lysis solution (150 mM ammonium chloride, 10 mM potassium hydrogencarbonate), incubating for 20 and 10 min respectively and centrifuging at 300G for 5 min at 6ºC. The pellet is resuspended in 0.2 ml of fixative solutions and immediately analyzed on a FACSCalibur (BD Biosciences). Eosinophils are gated on basis of autofluorescence in the FL2 channels and shape change of 600 cells is assayed by forward scatter and side scatter analysis. Compound IC₅₀ are determined using Excel XL-fit for calculations.

| **Example** | **GTP**γ **Binding (nM)** |
|---|---|
| 1 | 15 |
| 2 | 10 |
| 3 | 16 |
| 6 | 18 |
| 7 | 11 |
| 8 | 4 |
| 9 | 14 |
| 12 | 9 |
| 13 | 17 |
| 16 | 5 |
| 17 | 5 |
| 20 | 13 |
| 21 | 20 |
| 22 | 6 |
| 23 | 7 |
| 26 | 32 |

### COMBINATION PRODUCT

The compounds of the invention may also be combined with other active compounds in the treatment of diseases indicated above. For example the compounds of the present invention can be combined with active substances which are known to be useful in the treatment of these diseases.

Examples of such active substances are (a) anticholinergics, such as Aclidinium Bromide or Tiotropium, (b) corticosteroids, or gluococorticoids such as prednisone or methylprednisolone; (c) antihistamines, such as Ebastine, Ranitidine, ABT-239 or JNJ 7777120, (d) beta-2 agonists, such as salmeterol or formoterol, (e) chemokine receptor antagonists, such as Maraviroc or Enfuvirtide, (f) Leukotriene receptor antagonists, (g) JAK inhibitors such as tasooitirrib citrate or INCB018424, (h) Syk inhibitors such as R-112, (i) Phosphosdiesterase IV inhibitors such as GRC-4039, (j) p38 Inhibitors such as ARRY-797; (k) PKC inhibitors such as NVP-AEB071; (l) 5-lipoxygenase activating protein inhibitors, such as Veliflapon; (m) 5-lipoxygenase inhibitors, (n) CYSLTR1 antagonists, such as Montelukast, Pranlukast or Zafirlukast; (o) CYSLTR2 antagonists, such as Pranlukast, Zafirlukast or Tipilukast; (p) BLT1 antagonists, (q) BLT2 antagonists, (r) Thromboxane A2 antagonists such as Ramatroban; (s) DP1 receptor antagonists, such as Laropiprant; (t) DP1 receptor agonists, such as BW-245C; (u) IP receptor agonists, such as RO-1138452; (v) anti-IgE, such as Omalizumab; (w) IL5 antibody, such as Mepolizumab; (x) leukotriene formation inhibitors, (y) bronchodilators, such as pirbuterol, epinephrine, ephedrine, salbutamol or salmeterol; (z) decongestants, such as ephedrine, Levo-methamphetamine, Naphazoline, Oxymetazoline, Phenylephrine, Phenylpropanolamine, Propylhexedrine, Pseudoephedrine, Synephrine or Tetrahydrozoline; (aa) mucolytics such as Acetylcysteine, Ambroxol, Bromhexine, Carbocisteine, Domiodol, Eprazinone, Erdosteine, Letosteine, Neltenexine, Sobrerol, Stepronin or Tiopronin; (bb) antitussives, such as dectromethorphan; (cc) analgesics such as aspirin, paracetamol, rofecoxid, celecoxib, morphine, codeine, oxycodone, hydrocodone, dihydromorphine or flupirtine and (dd) Expectorants such Antimony pentasulfide, Guaiacolsulfonate, Guaifenesin, Potassium iodide or Tyloxapol.

Accordingly, another embodiment of the invention is a combination product comprising (i) at least a compound of formula (I) as defined previously, and (ii) one or more active ingredients as described above, for simultaneous, separate or sequential use in the treatment of the human or animal body.

The combinations of the invention may be used in the treatment of disorders which are susceptible to amelioration by CRTh2 receptor antagonists. Thus, the present application encompasses methods of treatment of these disorders, as well as the use of the combinations of the invention in the manufacture of a medicament for the treatment of these disorders.

Preferred examples of such disorders are asthma, chronic obstructive pulmonary disease (COPD), allergic rhinitis, atopic dermatitis, psoriasis, emphysema, eosinophilic bronchitis, being more preferably asthma, chronic obstructive pulmonary disease (COPD), allergic rhinitis and atopic dermatitis.

The amount of each active which is required to achieve a therapeutic effect will, of course, vary with the particular active, the route of administration, the subject under treatment, and the particular disease or disorder being treated.

The active ingredients may be administered from 1 to 6 times a day, sufficient to exhibit the desired activity. Preferably, the active ingredients are administered once or twice a day, most preferably once a day.

When combinations of actives are used, it is contemplated that all active agents would be administered at the same time, or very close in time. Alternatively, one or two actives could be taken in the morning and the other (s) later in the day. Or in another scenario, one or two actives could be taken twice daily and the other (s) once daily, either at the same time as one of the twice-a-day dosing occurred, or separately. Preferably at least two, and more preferably all, of the actives would be taken together at the same time. Preferably, at least two, and more preferably all actives would be administered as an admixture.

Examples of suitable β2-agonists that can be combined with the compounds of formula (I) are terbutaline sulphate, eformoterol fumarate, formoterol fumarate, bambuterol, ibuterol, isoprenaline hydrochloride, dopexamine, metaprotenerol, tulobuterol, procaterol hydrochloride, sibenadet hydrochloride, mabuterol hydrochloride, albuterol sulphate, salbutamol sulphate, salmefamol, salmeterol xinafoate, carmoterol hydrochloride, (R)-albuterol hydrochloride, Levalbuterol hydrochloride; Levosalbutamol hydrochloride; (-)-Salbutamol hydrochloride, formoterol, (R,R)-Formoterol tartrate; Arformoterol tartrate, sulfonterol, Bedoradrine sulphate, Indacaterol, Trantinterol hydrochloride, Milveterol hydrochloride, Olodaterol, fenoterol hydrobromide, rimoterol hydrobromide, riproterol hydrochloride, Vilanterol broxaterol, pirbuterol hydrochloride, bitolterol mesylate, clenbuterol hydrochloride, AZD-3199, GSK-159802; GSK-597901, GSK-678007, GSK-961081; 4-[2-[3-(1H-Benzimidazol-1-yl)-1,1-dimethylpropylamino]-1-hydroxyethyl]-2-(4-methoxybenzylamino)phenol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-domethoxyphenyl)-2-methyl-2-propylamino]ethanol, 1-[2H-5-hydroxy-3-oxo-4H-1,4-benzoxazin-8-yl]-2-[3-(4-n-butyloxyhenyl)-2-methyl-2-propylamino]ethanol, KUL-1248, HOKU-81, SM-110444, RP-58802B, LAS 100977 and compounds described in PCT patent applications Nos. WO 2007/124898, WO 2006/122788A1, WO 2008/046598, WO 2008095720, WO 2009/068177 and WO 2010/072354.

Examples of suitable corticosteroids and glucocorticoids that can be combined with the compounds of formula (I) are prednisolone, methylprednisolone, dexamethasone, dexamethasone cipecilate, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone furoate, rimexolone, prednisolone farnesylate, ciclesonide, butixocort propionate, RS-85095, CGP-13774, GW-250495, deltacortisone, NO-Prednisolone, etiprednol dicloacetate, QAE-397, 7beta-OH-EPIA, RPR-106541, deprodone propionate, fluticasone propionate, fluticasone furoate, halobetasol propionate, loteprednol etabonate, betamethasone butyrate propionate, flunisolide, prednisone, dexamethasone sodium phosphate, triamcinolone, betamethasone 17-valerate, betamethasone, betamethasone dipropionate, 21-Chloro-11beta-hydroxy-17alpha-[2-(methylsulfanyl)acetoxy]-4-pregnene-3,20-dione, Desisobutyrylciclesonide, hydrocortisone acetate, hydrocortisone sodium succinate, prednisolone sodium phosphate and hydrocortisone probutate, Prednisolone sodium metasulfobenzoate and clobetasol propionate.

Examples of suitable anticholinergics that can be combined with the compounds of formula (I) are tiotropium salts, oxitropium salts, flutropium salts, ipratropium salts, glycopyrronium salts, trospium salts, zamifenacin, revatropate, espatropate, darotropium bromide, CI-923, NPC-14695, BEA-2108, 3-[2-Hydroxy-2,2-bis(2-thienyl)acetoxy]-1-(3-phenoxypropyl)-1-azoniabicyclo[2.2.2]octane salts (in particular aclidinium salts, more preferably aclidinium bromide), 1-(2-Phenylethyl)-3-(9H-xanthen-9-ylcarbonyloxy)-1-azoniabicyclo[2.2.2]octane salts, 2-oxo-1,2,3,4-tetrahydroquinazoline-3-carboxylic acid endo-8-methyl-8-azabicyclo[3.2.1]oct-3-yl ester salts (DAU-5884), 3-(4-Benzylpiperazin-1-yl)-1-cyclobutyl-1-hydroxy-1-phenylpropan-2-one (NPC-14695), N-[1-(6-Aminopyridin-2-ylmethyl)piperidin-4-yl]-2(R)-[3,3-difluoro-1 (R)-cyclopentyl]-2-hydroxy-2-phenylacetamide (J-1 04135), 2(R)-Cyclopentyl-2-hydroxy-N-[1-[4(S)-methylhexyl]piperidin-4-yl]-2-phenylacetamide (J-106366), 2(R)-Cyclopentyl-2-hydroxy-N-[1-(4-methyl-3-pentenyl)-4-piperidinyl]-2-phenylacetamide (J-104129), 1-[4-(2-Aminoethyl)piperidin-1-yl]-2(R)-[3,3-difluorocyclopent-1(R)-yl]-2-hydroxy-2-phenylethan-1-one (Banyu-280634), N-[N-[2-[N-[1-(Cyclohexylmethyl)piperidin-3(R)-ylmethyl]carbamoyl]ethyl]carbamoylmethyl]-3,3,3-triphenylpropionamide (Banyu CPTP), 2(R)-Cyclopentyl-2-hydroxy-2-phenylacetic acid 4-(3-azabicyclo[3.1.0]hex-3-yl)-2-butynyl ester (Ranbaxy 364057), 3(R)-[4,4-Bis(4-fluorophenyl)-2-oxoimidazolidin-1-yl]-1-methyl-1-[2-oxo-2-(3-thienyl)ethyl]pyrrolidinium iodide, N-[1-(3-Hydroxybenzyl)-1-methylpiperidinium-3(S)-yl]-N-[N-[4-(isopropoxycarbonyl)phenyl]carbamoyl]-L-tyrasinamide trifluoroacetate, UCB-101333, Merck's OrM3, 7-endo-(2-hydroxy-2,2-diphenylacetoxy)-9,9-dimethyl-3-oxa-9-azoniatricyclo[3.3.1.0(2,4)]nonane salts, 3(R)-[4,4-Bis(4-fluorophenyl)-2-oxoimidazolidin-1-yl]-1-methyl-1-(2-phenylethyl)pyrrolidinium iodide, trans-4-[2-[Hydroxy-2,2-(dithien-2-yl)acetoxy]-1-methyl-1-(2-phenoxyethyl)piperidinium bromide from Novartis (412682), 7-(2,2-diphenylpropionyloxy)-7,9,9-trimethyl-3-oxa-9-azoniatricyclo[3.3.1.0*2,4*]nonane salts, 7-hydroxy-7,9,9-trimethyl-3-oxa-9-azoniatricyclo[3.3.1.0*2,4*]nonane 9-methyl-9H-fluorene-9-carboxylic acid ester salts, all of them optionally in the form of their racemates, their enantiomers, their diastereomers and mixtures thereof, and optionally in the form of their pharmacologically-compatible acid addition salts. Among the salts chlorides, bromides, iodides and methanesulphonates are preferred.

Examples of suitable anti-histamines that can be combined with the compounds of formula (I) are Methapyrilene, Mequitazine, Azelastine hydrochloride, Ranitinide, Acrivastine, Emedastine difumarate, Emedastine fumarate, Loratadine, Cyproheptadine hydrochloride, Diphenhydramine hydrochloride, Doxepin hydrochloride, Promethazine hydrochloride, Levocabastine hydrochloride, Desloratadine, Cinnarizine, Setastine hydrochloride, Mizolastine, Ebastine, Cetirizine hydrochloride, Epinastine hydrochloride, Olopatadine hydrochloride, Bepotastine besilate, Triprolidine hydrochloride, Rupatadine fumarate, Fexofenadine hydrochloride, Levocetirizine dihydrochloride, Ketotifen, Azatadine maleate, Dimethindene maleate, Clemastine fumarate, Alcaftadine, Bilastine, ABT-239, JNJ7777120, Vapitadine hydrochloride, AZD-1744, GSK-1004723D, GSK-835726, SUN-1334H.

Examples of suitable PDE4 inhibitors that can be combined with the compounds of formula (I) are benafentrine dimaleate, etazolate, denbufylline, rolipram, cipamfylline, zardaverine, arofylline, filaminast, tipelukast, tofimilast, pidamilast, tolafentrine, mesopram, drotaverine hydrochloride, lirimilast, roflumilast, cilomilast, oglemilast, apremilast, tetomilast, filaminast, (R)-(+)-4-[2-(3-Cyclopentyloxy-4-methoxyphenyl)-2-phenylethyl]pyridine (CDP-840), N-(3,5-Dichloro-4-pyridinyl)-2-[1-(4-fluorobenzyl)-5-hydroxy-1H-indol-3-yl]-2-oxoacetamide (GSK-842470), 9-(2-Fluorobenzyl)-N6-methyl-2-(trifluoromethyl)adenine (NCS-613), N-(3,5-Dichloro-4-pyridinyl)-8-methoxyquinoline-5-carboxamide (D-4418), 3-[3-(Cyclopentyloxy)-4-methoxybenzyl]-6-(ethylamino)-8-isopropyl-3H-purine hydrochloride (V-11294A), 6-[3-(N,N-Dimethylcarbamoyl)phenylsulfonyl]-4-(3-methoxyphenylamino)-8-methylquinoline-3-carboxamide hydrochloride (GSK-256066), 4-[6,7-Diethoxy-2,3-bis(hydroxymethyl)naphthalen-1-yl]-1-(2-methoxyethyl)pyridin-2(1H)-one (T-440), (-)-trans-2-[3'-[3-(N-Cyclopropylcarbamoyl)-4-oxo-1,4-dihydro-1,8-naphthyridin-1-yl]-3-fluorobiphenyl-4-yl]cyclopropanecarboxylic acid, MK-0873, CDC-801, UK-500001, BLX-914, 2-carbomethoxy-4-cyano-4-(3-cyclopropylmethoxy-4-difluroromethoxyphenyl)cyclohexan1-one, *cis* [4-cyano-4-(3-cyclopropylmethoxy-4-difluoromethoxyphenyl)cyclohexan-1-ol, 5(S)-[3-(Cyclopentyloxy)-4-methoxyphenyl]-3(S)-(3-methylbenzyl)piperidin-2-one (IPL-455903), ONO-6126 (Eur Respir J 2003, 22(Suppl. 45): Abst 2557) and the salts claimed in the PCT patent applications number WO 03/097613, WO 2004/058729, WO 2005/049581, WO 2005/123693, WO 2005/123692, and WO 2010/069504.

Examples of suitable Syk kinase inhibitors that can be combined with the compounds of formula (I) are fosfamatinib (from Rigel), R-348 (from Rigel), R-343 (from Rigel), R-112 (from Rigel), piceatannol, 2-(2-Aminoethylamino)-4-[3-(trifluoromethyl)phenylamino] pyrimidine-5-carboxamide, R-091 (from Rigel), 6-[5-Fluoro-2-(3,4,5-trimethoxyphenylamino)pyrimidin-4-ylamino]-2,2-dimethyl-3,4-dihydro-2H-pyrido[3,2-b][1,4]oxazin-3-one benzenesulfonate (R-406 from Rigel), 1-(2,4,6-Trihydroxyphenyl)-2-(4-methoxyphenyl)ethan-1-one, N-[4-[6-(Cyclobutylamino)-9H-purin-2-ylamino]phenyl]-N-methylacetamide (QAB-205 from Novartis), 2-[7-(3,4-Dimethoxyphenyl)imidazo[1,2-c]pyrimidin-5-ylamino]pyridine-3-carboxamide dihydrochloride (BAY-61-3606 from Bayer) and AVE-0950 (from Sanofi-Aventis). Particularly preferred combination products according to the invention comprise a compound of formula (I) and a therapeutically effective amount of one or more additional therapeutic agents selected from the group consisting of mometasone furoate, ciclesonide, budesonide, fluticasone propionate, fluticasone furoate, betamethasone valerate, clobetasol propionate, tiotropium salts, glycopyrronium salts, 3-[2-Hydroxy-2,2-bis(2-thienyl)acetoxy]-1-(3-phenoxypropyl)-1-azoniabicydo[2.2.2]octane salts (in particular adidinium salts, preferably aclidinium bromide), 1-(2-Phenylethyl)-3-(9H-xanthen-9-ylcarbonyloxy)-1-azoniabicyclo[2.2.2]octane salts, formoterol, salmeterol, indacaterol, carmoterol, LAS 100977, compounds described in PCT patent applications Nos. WO 2008/077639, WO 2009/021696, WO 2009/153043, and WO 2010/083975 (in particular amino(iso)nicotinic acid derivatives selected from the group consisting of 2-(3'-ethoxy-3-(trifluoromethoxy)biphenyl-4-ylamino)nicotinic acid, 2-(3,5-difluoro-3'-methoxybiphenyl-4-ylamino)nicotinic acid and 2-(3,5-difluoro-2-methylbiphenyl-4-ylamino)nicotinic acid; and azabiphenylaminobenzoic acid derivatives selected from the group consisting of 5-cyclopropyl-2-(2-(2,6-difluorophenyl)pyrimidin-5-ylamino)benzoic acid, 5-cydopropyl-2-((2-(2-(trifluoromethyl)phenyl)pyrimidin-5-yl)amino)benzoic acid and 5-methyl-2-((6-(2,3-difluorophenyl)pyridin-3-yl)amino)benzoic acid), methapyrilene, cetirizine, loratadine, ebastine, desloratadine, fexofenadine, azelastine, levocabastine, olopatadine, Montelukast, picremolimus, tacrolimus, mupiricin, retapamulin, clotrimazole, ketoconazole and terbinafine.

Preferred combination products according to the invention comprises a compound of formula (I) and a therapeutically effective amount of one or more additional therapeutic agents selected from the group consisting of mometasone furoate, ciclesonide, budesonide, fluticasone propionate, fluticasone furoate, tiotropium salts, glycopyrronium salts, 3-[2-Hydroxy-2,2-bis(2-thienyl)acetoxy]-1-(3-phenoxypropyl)-1-azoniabicydo[2.2.2]octane salts (in particular aclidinium salts, preferably aclidinium bromide), 1-(2-Phenylethyl)-3-(9H-xanthen-9-ylcarbonyloxy)-1-azoniabicyclo[2.2.2]octane salts, rolipram, roflumilast, cilomilast and the compounds claimed in the PCT patent applications number WO 03/097613, WO 2004/058729, WO 2005/049581, WO 2005/123693, WO 2005/123692, and WO 2010/069504.

Thus, in one aspect of the invention, the combination product comprises a compound of formula (I) and a corticosteroid. Particularly preferred corticosteroids are those selected from the group consisting of mometasone furoate, ciclesonide, budesonide, fluticasone furoate, fluticasone propionate, betamethasone valerate and clobetasol propionate.

In another aspect of the invention, the combination product comprises a compound of formula (I) and an anticholinergic agent. Particulary preferred anticholinergic agents are those selected from the group consisting of tiotropium salts, glycopirronium salts, 3-[2-Hydroxy-2,2-bis(2-thienyl)acetoxy]-1-(3-phenoxypropyl)-1-azoniabicyclo[2.2.2]octane salts and 1-(2-Phenylethyl)-3-(9H-xanthen-9-ylcarbonyloxy)-1-azoniabicyclo[2.2.2]-octane salts. The combination product may further comprise a corticosteroid selected from the group consisting of mometasone furoate, cidesonide, budesonide, fluticasone furoate and fluticasone propionate.

In a still other aspect of the invention, the combination product comprises a compound of formula (I) and a suitable β2-agonist. Particularly preferred β2-agonists are those selected from the group consisting of formoterol, salmeterol, indacaterol, carmoterol, LAS100977 and compound described in PCT patent applications Nos. WO 2007/124898, WO 2006/122788A1, WO 2008/046598, WO 2008095720, WO 2009/068177 and WO 2010/072354. The composition may further comprise a corticosteroid selected from the group consisting of mometasone furoate, ciclesonide, budesonide, fluticasone furoate, and fluticasone propionate. In addition to the compound of formula (I) and to the β2-agonist, the combination product may further comprise an anticholinergic agent selected from the group consisting of tiotropium salts, glycopyrronium salts, 3-[2-Hydroxy-2,2-bis(2-thienyl)acetoxy]-1-(3-phenoxypropyl)-1-azoniabicyclo[2.2.2] octane salts and 1-(2-Phenylethyl)-3-(9H-xanthen-9-ylcarbonyloxy)-1-azoniabicyclo[2.2.2] octane salts.

In a still other aspect of the invention, the combination product comprises a compound of formula (I) and a PDE4 inhibitors. Particularly preferred PDE4 inhibitors are those selected from the group consisting of rolipram, roflumilast, cilomilast and the compounds claimed in the PCT patent applications number WO 03/097613, WO 2004/058729, WO 2005/049581, WO 2005/123693, WO 2005/123692, and WO 2010/069504. The combination product may further comprise a corticosteroid selected from the group consisting of mometasone furoate, cidesonide, budesonide, fluticasone furoate and fluticasone propionate. In addition to the compound of the invention and to the PDE4 inhibitor, the combination product may further comprise an anticholinergic agent selected from the group consisting of tiotropium salts, glycopirronium salts, 3-[2-Hydroxy-2,2-bis(2-thienyl)acetoxy]-1-(3-phenoxypropyl)-1-azoniabicyclo[2.2.2] octane salts and 1-(2-Phenylethyl)-3-(9H-xanthen-9-ylcarbonyloxy)-1-azoniabicyclo[2.2.2] octane salts.

The active compounds in the combination product may be administered together in the same pharmaceutical composition or in different compositions intended for separate, simultaneous, concomitant or sequential administration by the same or a different route.

It is contemplated that all active agents would be administered at the same time, or very close in time. Alternatively, one or two actives could be administered in the morning and the other (s) later in the day. Or in another scenario, one or two actives could be administered twice daily and the other (s) once daily, either at the same time as one of the twice-a-day dosing occurred, or separately. Preferably at least two, and more preferably all, of the actives would be administered together at the same time. Preferably, at least two, and more preferably all actives would be administered as an admixture.

### PHARMACEUTICAL COMPOSITIONS

Pharmaceutical compositions according to the present invention comprise the compounds of the invention in association with a pharmaceutically acceptable diluent or carrier.

As used herein, the term pharmaceutical composition refers to a mixture of one or more of the compounds described herein, or physiologically/pharmaceutically acceptable salts, solvates, N-oxides, stereoisomers, deuterated derivatives thereof or prodrugs thereof, with other chemical components, such as physiologically/pharmaceutically acceptable carriers and excipients. The purpose of a pharmaceutical composition is to facilitate administration of a compound to an organism.

As used herein, a physiologically/pharmaceutically acceptable diluent or carrier refers to a carrier or diluent that does not cause significant irritation to an organism and does not abrogate the biological activity and properties of the administered compound.

A pharmaceutically acceptable excipient refers to an inert substance added to a pharmaceutical composition to further facilitate administration of a compound.

The invention further provides pharmaceutical compositions comprising the compounds of the invention in association with a pharmaceutically acceptable diluent or carrier together with one or more other therapeutic agents such as the previously described for use in the treatment of a pathological condition or disease susceptible to amelioration by CRTh2 receptor antagonists.

The invention is also directed to pharmaceutical compositions of the invention for use in the treatment of a pathological disease or disorder susceptible to amelioration by CRTh2 receptor antagonists, in particular wherein the pathological disease or disorder is as described above.

The invention also provides a method of treatment of a pathological condition or disease susceptible to amelioration by CRTh2 receptor antagonists in particular wherein the pathological condition or disease described above, comprising administering a therapeutically effective amount of a pharmaceutical composition of the invention.

The present invention also provides pharmaceutical compositions which comprise, as an active ingredient, at least a compound of formula (I) or a pharmaceutically acceptable salt, solvate, N-oxide or deuterated derivative thereof in association with a Pharmaceutical acceptable excipient such as a carrier or diluent. The active ingredient may comprise 0.001% to 99% by weight, preferably 0.01% to 90% by weight, of the composition depending upon the nature of the formulation and whether further dilution is to be made prior to application. Preferably the compositions are made up in a form suitable for oral, inhalation, topical, nasal, rectal, percutaneous or injectable administration.

Pharmaceutical compositions suitable for the delivery of compounds of the invention and methods for their preparation will be readily apparent to those skilled in the art. Such compositions and methods for their preparation can be found, for example, in Remington: The Science and Practice of Pharmacy, 21st Edition, Lippincott Williams & Wilkins, Philadelphia, Pa., 2001.

The pharmaceutically acceptable excipients which are admixed with the active compound or salts of such compound, to form the compositions of this invention are well-known per se and the actual excipients used depend inter alia on the intended method of administering the compositions. Examples, without limitation, of excipients include calcium carbonate, calcium phosphate, various sugars and types of starch, cellulose derivatives, gelatine, vegetable oils and polyethylene glycols.

Additional suitable carriers for formulations of the compounds of the present invention can be found in Remington: The Science and Practice of Pharmacy, 21st Edition, Lippincott Williams & Wilkins, Philadelphia, Pa., 2001.

### i) Oral Administration

The compounds of the invention may be administered orally (peroral administration; per os (latin)). Oral administration involve swallowing, so that the compound is absorbed from the gut and delivered to the liver via the portal circulation (hepatic first pass metabolism) and finally enters the gastrointestinal (GI) tract.

Compositions for oral administration may take the form of tablets, retard tablets, sublingual tablets, capsules, inhalation aerosols, inhalation solutions, dry powder inhalation, or liquid preparations, such as mixtures, solutions, elixirs, syrups or suspensions, all containing the compound of the invention; such preparations may be made by methods well-known in the art. The active ingredient may also be presented as a bolus, electuary or paste.

Where the composition is in the form of a tablet, any pharmaceutical carrier routinely used for preparing solid formulations may be used. Examples of such carriers include magnesium stearate, talc, gelatine, acacia, stearic acid, starch, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent.

Moulded tablets may be made by moulding in a suitable machine a mixture of the powdered compound moistened with an inert liquid diluent. The tablets may optionally be coated or scored and may be formulated so as to provide slow or controlled release of the active ingredient therein.

For tablet dosage forms, depending on dose, the drug may make up from 1 wt% to 80 wt% of the dosage form, more typically from 5 wt% to 60 wt% of the dosage form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl- substituted hydroxypropyl cellulose, starch, pregelatinized starch and sodium alginate. Generally, the disintegrant will comprise from 1 wt% to 25 wt%, preferably from 5 wt% to 20 wt% of the dosage form.

Binders are generally used to impart cohesive qualities to a tablet formulation. Suitable binders include microcrystalline cellulose, gelatine, sugars, polyethylene glycol, natural and synthetic gums, polyvinylpyrrolidone, pregelatinized starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate. Tablets may also optionally include surface active agents, such as sodium lauryl sulfate and polysorbate 80, and glidants such as silicon dioxide and talc. When present, surface active agents are typically in amounts of from 0.2 wt% to 5 wt% of the tablet, and glidants typically from 0.2 wt% to 1 wt% of the tablet.

Tablets also generally contain lubricants such as magnesium stearate, calcium stearate, zinc stearate, sodium stearyl fumarate, and mixtures of magnesium stearate with sodium lauryl sulphate. Lubricants generally are present in amounts from 0.25 wt% to 10 wt%, preferably from 0.5 wt% to 3 wt% of the tablet. Other conventional ingredients include anti-oxidants, colorants, flavoring agents, preservatives and taste-masking agents.

Exemplary tablets contain up to about 80 wt% drug, from about 10 wt% to about 90 wt% binder, from about 0 wt% to about 85 wt% diluent, from about 2 wt% to about 10 wt% disintegrant, and from about 0.25 wt% to about 10 wt% lubricant. Tablet blends may be compressed directly or by roller to form tablets. Tablet blends or portions of blends may alternatively be wet-, dry-, or melt-granulated, melt congealed, or extruded before tabletting. The final formulation may include one or more layers and may be coated or uncoated; or encapsulated.

The formulation of tablets is discussed in detail in "Pharmaceutical Dosage Forms: Tablets, Vol. 1 ", by H. Lieberman and L. Lachman, Marcel Dekker, N.Y.,, 1980.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.

Solid formulations for oral administration may be formulated to be immediate and/or modified release. Modified release formulations include delayed-, sustained-, pulsed-, controlled-, targeted and programmed release.

Suitable modified release formulations are described in U.S. Patent No. 6,106,864. Details of other suitable release technologies such as high energy dispersions and osmotic and coated particles can be found in Verma et al, Pharmaceutical Technology On-line, 25(2), 1-14 (2001). The use of chewing gum to achieve controlled release is described in WO 00/35298. The dislosures of these references are incorporated herein by reference in their entireties.

Liquid formulations include suspensions, solutions, syrups and elixirs. Such formulations may be used as fillers in soft or hard capsules and typically include a carrier, for example, water, ethanol, polyethylene glycol, propylene glycol, methylcellulose, or a suitable oil, and one or more emulsifying agents and/or suspending agents. The solutions may be aqueous solutions of a soluble salt or other derivative of the active compound in association with, for example, sucrose to form a syrup. The suspensions may comprise an insoluble active compound of the invention or a pharmaceutically acceptable salt thereof in association with water, together with a suspending agent or flavouring agent. Liquid formulations may also be prepared by the reconstitution of a solid, for example, from a sachet.

### ii) Oral mucosal administration

The compounds of the invention can also be administered via the oral mucosal administration. Within the oral mucosal cavity, delivery of drugs is classified into three categories: (a) sublingual delivery, which is systemic delivery of drugs through the mucosal membranes lining the floor of the mouth, (b) buccal delivery, which is drug administration through the mucosal membranes lining the cheeks (buccal mucosa), and (c) local delivery, which is drug delivery into the oral cavity.

Pharmaceutical products to be administered via the oral mucosal can be designed using mucoadhesive, quick dissolve tablets and solid lozenge formulations, which are formulated with one or more mucoadhesive (bioadhesive) polymers (such as hydroxy propyl cellulose, polyvinyl pyrrolidone, sodium carboxymethyl cellulose, hydroxy propyl methyl cellulose, hydroxy ethyl cellulose, polyvinyl alcohol, polyisobutylene or polyisoprene); and oral mucosal permeation enhancers (such as butanol, butyric acid, propranolol, sodium lauryl sulphate and others)

### iii) Inhaled administration

The compounds of the invention can also be administered by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurized container, pump, spray, atomizer (preferably an atomizer using electrohydrodynamics to produce a fine mist), or nebulizer, with or without the use of a suitable propellant, such as 1 ,1 ,1 ,2-tetrafluoroethane or 1 ,1 ,1 ,2,3,3,3-heptafluoropropane. For intranasal use, the powder may include a bioadhesive agent, for example, chitosan or cyclodextrin.

Dry powder compositions for topical delivery to the lung by inhalation may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred. Each capsule or cartridge may generally contain between 0.001-500 mg of active ingredient or the equivalent amount of a pharmaceutically acceptable salt thereof. Alternatively, the active ingredient (s) may be presented without excipients. Packaging of the formulation may be suitable for unit dose or multi-dose delivery. In the case of multi- dose delivery, the formulation can be pre-metered or metered in use. Dry powder inhalers are thus classified into three groups: (a) single dose, (b) multiple unit dose and (c) multi dose devices.

For inhalers of the first type, single doses have been weighed by the manufacturer into small containers, which are mostly hard gelatine capsules. A capsule has to be taken from a separate box or container and inserted into a receptacle area of the inhaler. Next, the capsule has to be opened or perforated with pins or cutting blades in order to allow part of the inspiratory air stream to pass through the capsule for powder entrainment or to discharge the powder from the capsule through these perforations by means of centrifugal force during inhalation. After inhalation, the emptied capsule has to be removed from the inhaler again. Mostly, disassembling of the inhaler is necessary for inserting and removing the capsule, which is an operation that can be difficult and burdensome for some patients.

Other drawbacks related to the use of hard gelatine capsules for inhalation powders are (a) poor protection against moisture uptake from the ambient air, (b) problems with opening or perforation after the capsules have been exposed previously to extreme relative humidity, which causes fragmentation or indenture, and (c) possible inhalation of capsule fragments. Moreover, for a number of capsule inhalers, incomplete expulsion has been reported (e. g. Nielsen et al, 1997).

Some capsule inhalers have a magazine from which individual capsules can be transferred to a receiving chamber, in which perforation and emptying takes place, as described in WO 92/03175. Other capsule inhalers have revolving magazines with capsule chambers that can be brought in line with the air conduit for dose discharge (e. g. WO91/02558 and GB 2242134). They comprise the type of multiple unit dose inhalers together with blister inhalers, which have a limited number of unit doses in supply on a disk or on a strip.

Blister inhalers provide better moisture protection of the medicament than capsule inhalers. Access to the powder is obtained by perforating the cover as well as the blister foil, or by peeling off the cover foil. When a blister strip is used instead of a disk, the number of doses can be increased, but it is inconvenient for the patient to replace an empty strip. Therefore, such devices are often disposable with the incorporated dose system, including the technique used to transport the strip and open the blister pockets.

Multi-dose inhalers do not contain pre-measured quantities of the powder formulation. They consist of a relatively large container and a dose measuring principle that has to be operated by the patient. The container bears multiple doses that are isolated individually from the bulk of powder by volumetric displacement. Various dose measuring principles exist, including rotatable membranes (Ex. EP0069715) or disks (Ex. GB 2041763; EP 0424790; DE 4239402 and EP 0674533), rotatable cylinders (Ex. EP 0166294; GB 2165159 and WO 92/09322) and rotatable frustums (Ex. WO 92/00771), all having cavities which have to be filled with powder from the container. Other multi dose devices have measuring slides (Ex. US 5201308 and WO 97/00703) or measuring plungers with a local or circumferential recess to displace a certain volume of powder from the container to a delivery chamber or an air conduit (Ex. EP 0505321, WO 92/04068 and WO 92/04928), or measuring slides such as the Genuair® (formerly known as Novolizer SD2FL), which is described the following patent applications Nos: WO97/000 WO03/000325 and WO2006/008027.

Reproducible dose measuring is one of the major concerns for multi dose inhaler devices.

The powder formulation has to exhibit good and stable flow properties, because filling of the dose measuring cups or cavities is mostly under the influence of the force of g ravity.

For reloaded single dose and multiple unit dose inhalers, the dose measuring accuracy and reproducibility can be guaranteed by the manufacturer. Multi dose inhalers on the other hand, can contain a much higher number of doses, whereas the number of handlings to prime a dose is generally lower.

Because the inspiratory air stream in multi-dose devices is often straight across the dose measuring cavity, and because the massive and rigid dose measuring systems of multi dose inhalers can not be agitated by this inspiratory air stream, the powder mass is simply entrained from the cavity and little de-agglomeration is obtained during discharge.

Consequently, separate disintegration means are necessary. However in practice, they are not always part of the inhaler design. Because of the high number of doses in multi-dose devices, powder adhesion onto the inner walls of the air conduits and the de-agglomeration means must be minimized and/or regular cleaning of these parts must be possible, without affecting the residual doses in the device. Some multi dose inhalers have disposable drug containers that can be replaced after the prescribed number of doses has been taken (Ex. WO 97/000703). For such semi-permanent multi dose inhalers with disposable drug containers, the requirements to prevent drug accumulation are even more strict.

Apart from applications through dry powder inhalers the compositions of the invention can be administered in aerosols which operate via propellant gases or by means of so-called atomisers, via which solutions of pharmacologically-active substances can be sprayed under high pressure so that a mist of inhalable particles results. The advantage of these atomisers is that the use of propellant gases can be completely dispensed with. Such atomiser is the Respimat® which is described, for example, in PCT Patent Applications Nos. W0 91/14468 and WO 97/12687, reference here is being made to the contents thereof.

Spray compositions for topical delivery to the lung by inhalation may for example be formulated as aqueous solutions or suspensions or as aerosols delivered from pressurised packs, such as a metered dose inhaler, with the use of a suitable liquefied propellant. Aerosol compositions suitable for inhalation can be either a suspension or a solution and generally contain the active ingredient (s) and a suitable propellant such as a fluorocarbon or hydrogen-containing chlorofluorocarbon or mixtures thereof, particularly hydrofluoroalkanes, e. g. dichlorodifluoromethane, trichlorofluoromethane, dichlorotetra-fluoroethane, especially 1,1,1, 2-tetrafluoroethane, 1,1,1,2, 3,3, 3-heptafluoro-n-propane or a mixture thereof. Carbon dioxide or other suitable gas may also be used as propellant.

The aerosol composition may be excipient free or may optionally contain additional formulation excipients well known in the art such as surfactants (eg oleic acid or lecithin) and cosolvens (eg ethanol). Pressurised formulations will generally be retained in a canister (eg an aluminium canister) closed with a valve (eg a metering valve) and fitted into an actuator provided with a mouthpiece.

Medicaments for administration by inhalation desirably have a controlled particle size. The optimum particle size for inhalation into the bronchial system is usually 1-10 µm, preferably 2-5 µm. Particles having a size above 20 µm are generally too large when inhaled to reach the small airways. To achieve these particle sizes the particles of the active ingredient as produced may be size reduced by conventional means eg by micronisation. The desired fraction may be separated out by air classification or sieving. Preferably, the particles will be crystalline.

Achieving high dose reproducibility with micronised powders is difficult because of their poor flowability and extreme agglomeration tendency. To improve the efficiency of dry powder compositions, the particles should be large while in the inhaler, but small when discharged into the respiratory tract. Thus, an excipient such as lactose or glucose is generally employed. The particle size of the excipient will usually be much greater than the inhaled medicament within the present invention. When the excipient is lactose it will typically be present as milled lactose, preferably crystalline alpha lactose monohydrate.

Pressurized aerosol compositions will generally be filled into canisters fitted with a valve, especially a metering valve. Canisters may optionally be coated with a plastics material e. g. a fluorocarbon polymer as described in W096/32150. Canisters will be fitted into an actuator adapted for buccal delivery.

### iv) Nasal mucosal administration,

The compounds of the invention may also be administered via the nasal mucosal.

Typical compositions for nasal mucosa administration are typically applied by a metering, atomizing spray pump and are in the form of a solution or suspension in an inert vehicle such as water optionally in combination with conventional excipients such as buffers, anti-microbials, tonicity modifying agents and viscosity modifying agents.

### v) Parenteral Administration

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water.

The preparation of parenteral formulations under sterile conditions, for example, by lyophilization, may readily be accomplished using standard pharmaceutical techniques well known to those skilled in the art. The solubility of compounds of the invention used in the preparation of parenteral solutions may be increased by the use of appropriate formulation techniques, such as the incorporation of solubility-enhancing agents.

Formulations for parenteral administration may be formulated to be immediate and/or modified release. Modified release formulations include delayed-, sustained-, pulsed-, controlled-, targeted and programmed release. Thus compounds of the invention may be formulated as a solid, semi-solid, or thixotropic liquid for administration as an implanted depot providing modified release of the active compound. Examples of such formulations include drug-coated stents and PGLA microspheres.

### vi) Topical Administration

The compounds of the invention may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibers, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated; see, for example, J Pharm Sci, 88 (10), 955-958 by Finnin and Morgan (October 1999). Other means of topical administration include delivery by electroporation, iontophoresis, phonophoresis, sonophoresis and microneedle or needle-free injection.

Formulations for topical administration may be formulated to be immediate and/or modified release. Modified release formulations include delayed-, sustained-, pulsed-, controlled-, targeted and programmed release.

### vii) Rectal/Intravaginal Administration

Compounds of the invention may be administered rectally or vaginally, for example, in the form of a suppository, pessary, or enema. Cocoa butter is a traditional suppository base, but various alternatives may be used as appropriate. Formulations for rectal/vaginal administration may be formulated to be immediate and/or modified release. Modified release formulations include delayed-, sustained-, pulsed-, controlled-, targeted and programmed release.

### viii) Ocular Administration

Compounds of the invention may also be administered directly to the eye or ear, typically in the form of drops of a micronized suspension or solution in isotonic, pH-adjusted, sterile saline. Other formulations suitable for ocular and aural administration include ointments, biodegradable {e.g. absorbable gel sponges, collagen) and nonbiodegradable (e.g. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, golan gum, may be incorporated together with a preservative, such as benzalkonium chloride. Such formulations may also be delivered by iontophoresis.

Formulations for ocular/aural administration may be formulated to be immediate and/or modified release. Modified release formulations include delayed-, sustained-, pulsed-, controlled-, targeted, or programmed release.

### ix) Other Technologies

Compounds of the invention may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.

The amount of the active compound administered will be dependent on the subject being treated, the severity of the disorder or condition, the rate of administration, the disposition of the compound and the discretion of the prescribing physician. However, an effective dosage is typically in the range of 0.01-3000 mg, more preferably 0.5-1000 mg of active ingredient or the equivalent amount of a Pharmaceutical acceptable salt thereof per day. Daily dosage may be administered in one or more treatments, preferably from 1 to 4 treatments, per day.

The pharmaceutical formulations may conveniently be presented in unit dosage form and may be prepared by any of the methods well known in the art of pharmacy.

Preferably the composition is in unit dosage form, for example a tablet, capsule or metered aerosol dose, so that the patient may administer a single dose.

The following preparations forms are cited as formulation examples:

### Formulation Example 1 (Oral suspension)

| **Ingredient** | **Amount** |
|---|---|
| Active Compound | 3 mg |
| Citric acid | 0,5 g |
| Sodium chloride | 2,0 g |
| Methyl paraben | 0,1 g |
| Granulated sugar | 25 g |
| Sorbitol (70% solution) | 11 g |
| Veegum K | 1,0 g |
| Flavoring | 0,02 g |
| Dye | 0,5 g |
| Distilled water | q.s. to 100 mL |

### Formulation Example 2 (Hard gelatine capsule for oral administration)

| **Ingredient** | **Amount** |
|---|---|
| Active Compound | 1 mg |
| Lactose | 150 mg |
| Magnesium stearate | 3 mg |

### Formulation Example 3 (Gelatin cartridge for inhalation)

| **Ingredient** | **Amount** |
|---|---|
| Active Compound (micronized) | 0,2 mg |
| Lactose | 25 mg |

### Formulation Example 4 (Formulation for inhalation with a DPI)

| **Ingredient** | **Amount** |
|---|---|
| Active Compound (micronized) | 15 mg |
| Lactose | 3000 mg |

### Formulation Example 5 (Formulation for a MDI)

| **Ingredient** | **Amount** |
|---|---|
| Active Compound (micronized) | 10 g |
| 1,1,1,2,3,3,3-heptafluoro-n-propane | q.s. to 200 ml |

Modifications, which do not affect, alter, change or modify the essential aspects of the compounds, combinations or pharmaceutical compositions described, are included within the scope of the present invention.

## Claims

1. A compound of formula (I), or a pharmaceutically acceptable salt, solvate, N-oxide,
stereoisomer or deuterated derivative thereof: 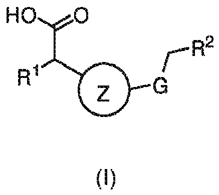 wherein:
• Z represents a 9-membered bicyclic heteroaryl group comprising 2 nitrogen atoms and optionally comprising further heteroatoms selected from N, S and O; wherein said heteroaryl group is optionally substituted by one to four substituents selected from the group consisting of a halogen atom, a linear or branched C₁₋₄ alkyl group, a C₃₋₇ cycloalkyl group, a linear or branched C₁₋₄ haloalkyl group, a -CN group, a -OH group, a -OR^{a} group, a -CO₂H group, a -CO₂R^{a} group, a -SR^{a} group, and a -SO₂R^{a} group;
• R¹ is selected from the group consisting of a hydrogen atom and a linear or branched C₁₋₄ alkyl group;
• G is selected from the group consisting of a phenyl group and a 5 to 6-membered N-containing heteroaryl group, wherein the phenyl and heteroaryl groups are optionally substituted by one or more substituents selected from the group consisting of a halogen atom, a cyano group, a nitro group, a -CHF₂ group, a -CF₃ group, a linear or branched C₁₋₄ alkyl group, a C₃₋₇ cycloalkyl group, a -OH group, a -OR^{a} group, a -SR^{a} group and a -SO₂R^{a} group;
• R² is selected from the group consisting of a -N(R³)COR⁴ group, a -N(R³)SO₂R⁴ group and a heteroaryl group selected from the group consisting of a triazolyl group, an isoxazolyl group, a pyrazolyl group and an oxazolyl group, wherein the heteroaryl group is optionally substituted by one or more substituents selected from the group consisting of a halogen atom, a -CF₃ group, a linear or branched C₁₋₄ alkyl group, a C₃₋₇ cycloalkyl group, a benzyl group, a phenyl group, a hydroxy group and a carboxy group;
• R³ is selected from the group consisting of a linear or branched C₁₋₄ alkyl group; a C₃₋₇ cycloalkyl group, a linear or branched C₁₋₄ haloalkyl group and a benzyl group;
• R⁴ is selected from the group consisting of a linear or branched C₁₋₄ alkyl group, a -(CH₂)ₙ-C₃₋₇ cycloalkyl group, a -CF₃ group, a linear or branched C₁₋₄ alkoxy group, a -C₁₋₄ alkyl-C₁₋₄ alkoxy group, a -(CH₂)ₙNR^{b}R^{c} group, a 4- to 6-membered heterocyclyl group containing at least one heteroatom selected from N, S and O, a -(CH₂)ₙ-phenyl group, a -(CH₂)ₙ-(5- to 6-membered heteroaryl) group containing at least one heteroatom selected from N, S and O, wherein the phenyl, heteroaryl and heterocyclyl groups are optionally substituted by one or more substituents selected from the group consisting of a halogen atom, a linear or branched C₁₋₄ alkyl group, an acyl group and a linear or branched C₁₋₄ alkoxy group;
• R^{a} is selected from the group consisting of a linear or branched C₁₋₄ alkyl group, a linear or branched C₁₋₄ haloakyl group and a C₃₋₇ cycloalkyl group;
• R^{b} and R^{c} are independently selected from the group consisting of a hydrogen atom, a linear or branched C₁₋₄ alkyl group, a C₃₋₇ cycloalkyl group, a phenyl group, a 5- to 6-membered heteroaryl containing at least one heteroatom selected from N, S and O, a -C₁₋₄ alkyl-phenyl group and -C₁₋₄ alkyl-5- to 6-membered heteroaryl group; or R^{b} and R^{c} together with the nitrogen atom to which they are attached form a 3- to 7-membered N-containing heterocyclyl group which is optionally substituted by one or more substitutents selected by the group consisting of a halogen atom, a linear or branched C₁₋₄ alkyl group and a linear or branched C₁₋₄ alkoxy group; and
• n is an integer selected from 0 to 2.

2. A compound according to claim 1, wherein Z is selected from the group consisting of a 4-azaindolyl group, a 5-azaindolyl group, a 7-azaindolyl group, a pyrrolo[1,2-a]pyrimidinyl group, an imidazo[1,5-a]pyridyl group, a pyrrolo[1,2-a]pyrazinyl group, a pyrrolo[1,2-c]pyrimidinyl group and a pyrrolo[1,2-b]pyridazinyl group, wherein said groups are optionally substituted by one to four substituents selected from the group consisting of a halogen atom, a linear or branched C₁₋₄ alkyl group, a C₃₋₇ cycloalkyl group, a linear or branched C₁₋₄ haloalkyl group, a -CN group, a -OH group, a -OR^{a} group, a -CO₂H group, a -CO₂R^{a} group, a -SR^{a} group, and a -SO₂R^{a} group.

3. A compound according to claim 2, wherein Z represents a group of formula A, B, C, D, E, F, G or H: 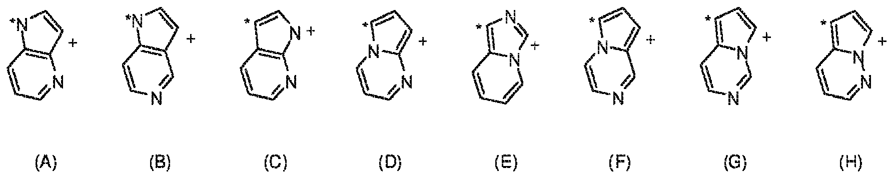 wherein the asterisk symbol indicates the bond that is linked to the -CHR¹- moiety of Formula (I) and the plus symbol indicates the bond that is linked to the G group of Formula (I), and wherein said groups of formula A to H are optionally substituted by one to four substitutents selected from the group consisting of a fluorine atom, a chlorine atom, a C₁₋₄ alkyl group, a methoxy group, a -CN group, a cyclopropyl group, a -CF₃ group and a -CHF₂ group.

4. A compound according to claim 3 wherein Z is a group of formula A, C or D, which is optionally substituted by one or two substituents selected from the group consisting of a methyl group, an ethyl group, a cyclopropyl group, a -CF₃ group, a-CHF₂ group and a methoxy group.

5. A compound according to any preceding claim, wherein R¹ is selected from the group consisting of a hydrogen atom and methyl group.

6. A compound according to any one of claims 1 to 4, wherein G is selected from the group consisting of a phenyl group, a pyridyl group, a pyrimidinyl group, a pyridazinyl group and a pyrazolyl group, wherein said ring groups are optionally substituted by one or two substituents selected from the group consisting of a halogen atom, a methyl group, a -SO₂Me group, a -CHF₂ group and a -CF₃ group.

7. A compound according to claim 5, wherein G represents a phenyl group optionally substituted by one substitutent selected from the group consisting of a fluorine atom and a -CF₃ group.

8. A compound according to any preceding claim, wherein R² is selected from the group consisting of a -N(R³)COR⁴ group and a heteroaryl group selected from the group consisting of a triazolyl group and an isoxazolyl group, wherein the heteroaryl group is optionally substituted by one or more substituents selected from the group consisting of a methyl group and a cyclopropyl group.

9. A compound according to claim 8, wherein R³ is selected from the group consisting of an ethyl group and a benzyl group.

10. A compound according to claim 8, wherein R⁴ is selected from the group consisting of a linear or branched C₁₋₄ alkyl group, a -(CH₂)ₙ-C₃₋₇ cycloalkyl group, a linear or branched C₁₋₄ alkoxy group, a -C₁₋₂ alkyl-C₁₋₂ alkoxy group, a benzyl group, a - (CH₂)ₙNR^{b}R^{c} group, a 4- to 6-membered heterocyclyl group containing at least one heteroatom selected from N, S and O and a -(CH₂)ₙ-(5- to 6-membered N-containing heteroaryl) group, wherein the heterocyclyl and heteroaryl groups are optionally substituted by one or more substituents selected from the group consisting of a halogen atom, an acyl group and a methyl group.

11. A compound according to any preceding claim, wherein
• Z represents a group of formula A, C or D, which is optionally substituted by one or two substituents selected from the group consisting of a methyl group, an ethyl group, a cyclopropyl group, a -CF₃ group, a -CHF₂ group and a methoxy group;
• R¹ is selected from the group consisting of a hydrogen atom and methyl group;
• G is selected from the group consisting of a phenyl group, a pyridyl group, a pyrimidinyl group, a pyridazinyl group and a pyrazolyl group, wherein said ring groups are optionally substituted by one or two substituents selected from the group consisting of a halogen atom, a methyl group, a -CHF₂ group and a -CF₃ group; preferably G represents a phenyl group optionally substituted by one substitutent selected from the group consisting of a fluorine atom and a -CF₃ group;
• R² is selected from the group consisting of a -N(R³)COR⁴ group and a heteroaryl group selected from the group consisting of a triazolyl group and an isoxazolyl group, wherein the heteroaryl group is optionally substituted by one or more substituents selected from the group consisting of a methyl group and a cyclopropyl group;
• R³ is selected from the group consisting of an ethyl group and a benzyl group;
• R⁴ is selected from the group consisting of a linear or branched C₁₋₄ alkyl group, a "(CH₂)ₙ-C₃₋₇ cycloalkyl group, a linear or branched C₁₋₄ alkoxy group, a -(CH₂)ₙNR^{b}R^{c} group and a -(CH₂)ₙ-(5- to 6-membered N-containing heteroaryl) group, which is optionally substituted by one or more substituents selected from the group consisting of a halogen atom and a methyl group, wherein R^{b} and R^{c} are preferably independently selected from a hydrogen atom, a linear or branched C₁₋₄ alkyl group, a phenyl and a benzyl group; and
• n is 0 or 1.

12. A compound according to claim 1, wherein
• Z represents a group of formula A, B, C, D, E, F or G, which is optionally substituted by one or two substituents selected from the group consisting of a methyl group, an ethyl group, a cyclopropyl group, a -CF₃ group, a -CHF₂ group and a methoxy group;
• R¹ is selected from the group consisting of a hydrogen atom and methyl group;
• G represents a phenyl group optionally substituted by one substitutent selected from the group consisting of a fluorine atom and a -CF₃ group;
• R² is a -N(R³)COR⁴ group;
• R³ is selected from the group consisting of a linear or branched C₁₋₄ alkyl group, preferably an ethyl group, and a benzyl group; and
• R⁴ is selected from the group consisting of a C₃₋₇ cycloalkyl group, preferably a cyclopropyl group, and a -(CH₂)ₙNR^{b}R^{c} group, wherein R^{b} represents a hydrogen atom and R^{c} represents a benzyl moiety.

13. A compound according to claim 1 which is one of:
{3-[2-{[(Cyclopropylcarbonyl)(ethyl)amino]methyl}-4-{trifluoromethyl)phenyl]-1H-pyrrolo[3,2-b]pyridin-1-yl)acetic acid;
[3-(2-{[(Cyclopropylcarbonyl)(ethyl)amino]methyl}-4-fluorophenyl)-1H-pyrrolo[3,2-b]pyridin-1-yl]acetic acid;
{3-[2-{[(Cyclopropylcarbonyl)(ethyl)amino]methyl}-4-(trifluoromethyl)phenyl]-5-methyl-1H-pyrrolo[3,2-b]pyridin-1-yl}acetic acid;
2-{3-[2-{[(Cyclopropylcarbonyl)(ethyl)amino]methyl}-4-(trifluoromethyl)phenyl]-1H-pyrrolo[3,2-b]pyridin-1-yl}propanoic acid;
{5-Cyclopropyl-3-[2-{[(cyclopropylcarbonyl)(ethyl)amino]methyl}-4-(trifluoromethyl)phenyl]-1H-pyrrolo[3,2-b]pyridin-1-yl}acetic acid;
2-{3-[2-{[(Cyclopropylcarbonyl)(ethyl)amino]methyl}-4-(trifluoromethyl)phenyl]-5-methyl-1H-pyrrolo[3,2-b]pyridin-1-yl}propanoic acid;
[3-(2-{[(Cyclopropylcarbonyl)(ethyl)amino]methyl}-4-fluorophenyl)-5-(trifluoromethyl)-1H-pyrrolo[3,2-b]pyridin-1-yl]acetic acid;
{3-[2-{[[(Benzylamino)carbonyl](ethyl)amino]methyl}-4-(trifluoromethyl)phenyl]-5-methyl-1H-pyrrolo[3,2-b]pyridin-1-yl}acetic acid;
{3-[2-{[Benzyl(cyclopropylcarbonyl)amino]methyl}-4-(trifluoromethyl)phenyl]-5-methyl-1H-pyrrolo[3,2-b]pyridin-1-yl}acetic acid;
{3-[2-{[(Cyclopropylcarbonyl)(ethyl)amino]methyl}-4-(trifluoromethyl)phenyl]-1H-pyrrolo[3,2-c]pyridin-1-yl}acetic acid;
{3-[2-{[(Cyclopropylcarbonyl)(ethyl)amino]methyl}-4-(trifluoromethyl)phenyl]-2,4-dimethyl-1H-pyrrolo[3,2-c]pyridin-1-yl}acetic acid;
{1-[2-{[(Cyclopropylcarbonyl)(ethyl)amino]methyl}-4-(trifluoromethyl)phenyl]-1H-pyrrolo[2,3-b]pyridin-3-yl}acetic acid;
[1-(2-([(Cyclopropylcarbonyl)(ethyl)amino]methyl)-4-fluorophenyl)-1H-pyrrolo[2,3-b]pyridin-3-yl]acetic acid;
{1-[2-{[(Cyclopropylcarbonyl)(ethyl)amino]methyl)-4-(trifluoromethyl)phenyl]-6-methyl-1H-pyrrolo[2,3-b]pyridin-3-yl}acetic acid;
{6-Cyclopropyl-1-[2-{[(cyclopropylcarbonyl)(ethyl)amino]methyl)-4-(trifluoromethyl)phenyl]-1H-pyrrolo[2,3-b]pyridin-3-yl}acetic acid;
[1-(2-{[(Cyclopropylcarbonyl)(ethyl)amino]methyl}-4-fluorophenyl)-6-methyl-1H-pyrrolo[2,3-b]pyridin-3-yl]acetic acid;
[1-(2-{[(Cyclopropylcarbonyl)(ethyl)amino]methyl}phenyl)-6-methyl-1H-pyrrolo[2,3-b]pyridin-3-yl]acetic acid;
{1-[2-{[(Cyclopropylcarbonyl)(ethyl)amino]methyl}-4-(trifluoromethyl)phenyl]-6-methoxy-1H-pyrrolo[2,3-b]pyridin-3-yl}acetic acid;
2-(1-(2-((N-Ethylcyclopropanecarboxamido)methyl)-4-(trifluoromethyl)phenyl)-6-(trifluoromethyl)-1H-pyrrolo[2,3-b]pyridin-3-yl)acetic acid;
{8-[2-{[(Cyclopropylcarbonyl)(ethyl)amino]methyl}-4-(trifluoromethyl)phenyl]pyrrolo[1,2-a]pyrimidin-6-yl}acetic acid;
[8-(2-{[(Cyclopropylcarbonyl)(ethyl)amino]methyl}-4-fluorophenyl)pyrrolo[1,2-a]pyrimidin-6-yl]acetic acid;
[8-[2-{[(Cyclopropylcarbonyl)(ethyl)amino]methyl}-4-(trifluoromethyl)phenyl]-2-(trifluoromethyl)pyrrolo[1,2-a]pyrimidin-6-yl]acetic acid;
{8-[2-{[(Cyclopropylcarbonyl)(ethyl)amino]methyl}-4-(trifluoromethyl)phenyl]-2-methylpyrrolo[1,2-a]pyrimidin-6-yl}acetic acid;
{3-[2-{[(Cyclopropylcarbonyl)(ethyl)amino]methyl}-4-{trifluoromethyl)phenyl]imidazo[1,5-a]pyridin-1-yl}acetic acid;
{8-[2-{[(Cyclopropylcarbonyl)(ethyl)amino]methyl}-4-(trifluoromethyl)phenyl]-1,7-dimethylpyrrolo[1,2-a]pyrazin-6-yl}acetic acid;
{7-[2-{[(Cyclopropylcarbonyl)(ethyl)amino]methyl}-4-(trifluoromethyl)phenyl]pyrrolo[1,2-c]pyrimidin-5-yl}acetic acid; or
{7-[2-{[(Cyclopropylcarbonyl)(ethyl)amino]methyl}-4-(trifluoromethyl)phenyl]-1-methylpyrrolo[1,2-c]pyrimidin-5-yl}acetic acid.

14. A compound according to any one of claims 1 to 13 for use in the treatment of the human or animal body by therapy.

15. A compound according to any one of claims 1 to 13 for use in the treatment of a pathological condition or disease mediated by prostaglandin D₂, which condition or disease is preferably selected from asthma, chronic obstructive pulmonary disease (COPD), rhinitis, emphysema, allergic airway syndrome, allergic rhinobronchitis, psoriasis, dermatitis, allergic conjunctivitis, atopic dermatitis, pulmonary hypertension, interstitial lung fibrosis, allergy, psoriasis, adult respiratory distress syndrome, myocardial infarction, aneurysm, stroke, cancer, wound healing, endotoxic shock, pain, inflammatory conditions, eosinophilic esophagitis, eosinophil-associated gastrointestinal disorders (EGID), idiopathic, otitis, airway constriction, mucus secretion, nasal congestion, increased microvascular permeability and recruitment of eosinophils, urticaria, sinusitis, angioedema, anaphylaxia, chronic cough, Churg Strauss syndrome, arthritis, Crohn's disease, ulcerative colitis, irritable bowel disease and inflammatory bowel disease, more preferably selected from asthma, chronic obstructive pulmonary disease (COPD), allergic rhinitis and atopic dermatitis.

16. Use of a compound as defined in any one of claims 1 to 13 for the manufacture of a medicament for the treatment of a pathological condition or disease as defined in claim 15.

17. A pharmaceutical composition comprising a compound as defined in any one of claims 1 to 13 in association with a pharmaceutically acceptable diluent or carrier.

18. A method for treating a subject afflicted with a pathological condition or disease as defined in claim 15, which comprises administering to said subject an effective amount of a compound as defined in any one of claims 1 to 13.

19. A combination product comprising (i) a compound according to any one of claims 1 to 13; and (ii) another compound selected from
a. Anticholinergics, such as adidinium bromide or tiotropium,
b. Corticosteroids, or gluococorticoids such as prednisone or methylprednisolone,
c. Antihistamines, such as ebastine, ranitidine, ABT-239 or JNJ 7777120;
d. Beta-2 agonists, such as salmeterol or formoterol,
e. Chemokine receptor antagonists, such as maraviroc or enfuvirtide,
f. Leukotriene receptor antagonists,
g. JAK inhibitors such as tasocitinib citrate or INCB018424,
h. Syk inhibitors such as R-112,
i. Phosphosdiesterase IV inhibitors such as roflumilast or revamilast,
j. Dual beta-2 adrenoceptor agonist/M3 receptor antagonist (MABA compounds) such as GSK-961081,
k. p38 Inhibitors such as ARRY-797,
l. PKC inhibitors such as NVP-AEB071,
m. 5-lipoxygenase activating protein inhibitors, such as veliflapon,
n. 5-lipoxygenase inhibitors,
o. CYSLTR1 antagonists, such as montelukast, pranlukast or zafirlukast;
p. CYSLTR2 antagonists, such as pranlukast, zafirlukast or tipilukast;
q. BLT1 antagonists,
r. BLT2 antagonists,
s. Thromboxane A2 antagonists such as ramatroban,
t. DP1 receptor antagonists, such as laropiprant,
u. DP1 receptor agonists, such as BW-245C,
v. IP receptor agonists, such as RO-1138452,
w. Anti-IgE, such as omalizumab,
x. IL5 antibody, such as mepolizumab,
y. Leukotriene formation inhibitors,
z. Bronchodilators, such as pirbuterol, epinephrine, ephedrine or salbutamol;
aa. Decongestants, such as ephedrine, levo-methamphetamine, naphazoline, oxymetazoline, phenylephrine, phenylpropanolamine, propylhexedrine, pseudoephedrine, synephrine or tetrahydrozoline;
bb. Mucolytics such as acetylcysteine, ambroxol, bromhexine, carbocisteine, domiodol, eprazinone, erdosteine, letosteine, neltenexine, sobrerol, stepronin or tiopronin;
cc. Antitussives, such as dextromethorphan,
dd. Analgesics such as aspirin, paracetamol, rofecoxid, celecoxib, morphine, codeine, oxycodone, hydrocodone, dihydromorphine or flupirtine; and
ee. Expectorants such antimony pentasulfide, guaiacolsulfonate, guaifenesin, potassium iodide or tyloxapol.
